# EUROPÄISCHE PATENTSCHRIFT

(11) **EP 1 613 607 B1**
(45) Veröffentlichungstag und Bekanntmachung des Hinweises auf die Patenterteilung: **09.01.2013**
(21) Anmeldenummer: 04725933.8
(22) Anmeldetag: 06.04.2004
(51) Int. Cl.: C07D 239/54, C07D 521/00, C07D 403/12, C07D 401/12, C07D 405/12, C07D 409/12, A01N 43/54

(54) **BENZOLSULFONAMID-DERIVATE ALS HERBIZIDE ODER DESIKKANTE/DEFOLIANTE VERBINDUNGEN**
BENZENESULPHONAMIDE DERIVATIVES AS HERBICIDES OR DESICCANT/DEFOLIANT COMPOUNDS
DERIVES DE BENZOLSULFONAMIDE CONSTITUANT DES COMPOSES HERBICIDES OU DESSICCANTS/DEFOLIANTS

(30) Priorität: 08.04.2003 DE 10316311
(43) Veröffentlichungstag der Anmeldung: 11.01.2006
(73) Patentinhaber: BASF SE, 67056 Ludwigshafen (DE)
(72) Erfinder: HAMPRECHT, Gerhard, 69469 Weinheim (DE); PUHL, Michael, 68623 Lampertheim (DE); REINHARD, Robert, 67065 Ludwigshafen (DE); SEITZ, Werner, 68723 Plankstadt (DE); ZAGAR, Cyrill, 68167 Mannheim (DE); WITSCHEL, Matthias, 67098 Bad Dürkheim (DE); LANDES, Andreas, 67354 Römerberg-Heiligenstein (DE)
(86) Internationale Anmeldenummer: PCT/EP2004/003624
(87) Internationale Veröffentlichungsnummer: WO 2004/089914

(56) Entgegenhaltungen:
- EP-A- 0 361 114
- WO-A-97/00246
- WO-A-97/42176
- US-A- 4 369 058
- US-A- 5 169 430
- DATABASE CHEMABS [Online] CHEMICAL ABSTRACTS SERVICE, COLUMBUS, OHIO, US; YOSHIOKA, YASUHIRO: "Heat-developable photographic films containing specific hydrazine and specific heterocyclic compound" retrieved from STN Database accession no. 2002:959013 XP002289556 & JP 2002 365759 A2 (FUJI PHOTO FILM CO., LTD., JAPAN) 18. Dezember 2002 (2002-12-18)
- DATABASE CAOLD [Online] CHEMICAL ABSTRACTS SERVICE, COLUMBUS, OHIO, US; SHREEKRISHNA G M ET AL: "Anthranilamides as intermediates for 3-substituted quinazoline-2,4-diones " Database accession no. CA62:4028g XP002289557

## Beschreibung

Die vorliegende Erfindung betrifft Benzolsulfonamid-Derivate der Formel I 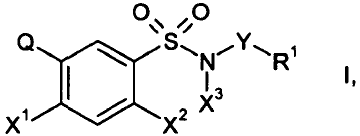 in der die Variablen die folgenden Bedeutungen haben:
- X¹: Wasserstoff oder Halogen;
- X²: Chlor;
- X³: Wasserstoff, Cyano, C₁-C₆-Alkyl, C₁-C₆-Alkoxy-C₁-C₄-alkyl, C₃-C₇-Cycloalkyl, C₃-C₆-Alkenyl, C₃-C₆-Alkinyl oder Phenyl-C₁-C₄-alkyl, wobei der Phenylrest seinerseits partiell oder vollständig halogeniert und/oder durch ein bis drei Reste aus der Gruppe C₁-C₆-Alkyl und C₁-C₆-Alkoxy substituiert sein kann;
- Y: eine Gruppe -C(A)B;
- A: Sauerstoff;
- B: Sauerstoff oder Schwefel;
- R¹: Wasserstoff, Hydroxy, C₁-C₈-Alkyl, C₃-C₇-Cycloalkyl, C₃-C₇-Cycloalkyl-C₁-C₄-alkyl, C₂-C₈-Alkenyl, C₅-C₇-Cycloalkenyl, C₃-C₈-Alkinyl, C₁-C₈-Alkoxy, C₃-C₇-Cycloalkyloxy, C₂-C₈-Alkenyloxy, C₃-C₈-Alkinyloxy, Aryl, Aryloxy, Aryl-C₁-C₄-alkyl;
wobei die 13 letztgenannten Reste ihrerseits partiell oder vollständig halogeniert sein können und/oder ein bis drei Substituenten aus der Gruppe Cyano, NO₂, Hydroxy, C₁-C₆-Alkyl, C₁-C₆-Halogenalkyl, C₃-C₇-Cycloalkyl, C₁-C₆-Alkoxy, C₁-C₆-Halogenalkoxy, C₃-C₇-Cycloalkyloxy, C₂-C₆-Alkenyloxy, C₃-C₆-Alkinyloxy, C₁-C₆-Alkylthio, C₁-C₆-Halogenalkylthio, Amino, C₁-C₆-Alkylamino, Di(C₁-C₆-alkyl)amino, C₁-C₆-Alkylsulfinyl, C₁-C₆-Halogenalkylsulfinyl, C₁-C₆-Alkylsulfonyl, C₁-C₆-Halogenalkylsulfonyl, C₁-C₆-Alkoxysulfonyl, Formyl, C₁-C₆-Alkylcarbonyl, C₁-C₆-Halogenalkylcarbonyl, C₂-C₆-Alkenylcarbonyl, C₃-C₆-Alkinylcarbonyl, Carboxy, C₁-C₆-Alkoxycarbonyl, C₁-C₆-Halogenalkoxy-carbonyl, C₂-C₆-Alkenyloxycarbonyl, C₃-C₆-Alkinyloxycarbonyl, Mercapto-carbonyl, C₁-C₆-Alkylthiocarbonyl, C₁-C₆-Halogenalkylthiocarbonyl, C₂-C₆-Alkenylthiocarbonyl, C₃-C₆-Alkinylthiocarbonyl, Aminocarbonyl, C₁-C₆-Alkylaminocarbonyl, Di(C₁-C₆-alkylamino)carbonyl, C₁-C₆-Halogenalkylaminocarbonyl, Di(C₁-C₆-halogenalkylamino)carbonyl, C₂-C₆-Alkenylaminocarbonyl, Di(C₂-C₆-alkenylamino)carbonyl, C₃-C₆-Alkinylaminocarbonyl, Di(C₃-C₆-alkinylamino)carbonyl, Phenyl, Phenoxy, Phenyl-C₁-C₄-Alkyl und Phenyl-C₁-C₄-alkoxy, tragen können;
vier- bis sechsgliedriges Heterocyclyl, das partiell oder vollständig halogeniert und/oder durch ein bis drei Reste aus der Gruppe C₁-C₆-Alkyl und C₁-C₆-Alkoxy substituiert sein kann; oder
vier- bis sechsgliedriges Heterocycyl-C₁-C₄-alkyl, das partiell oder vollständig halogeniert und/oder durch ein bis drei Reste aus der Gruppe C₁-C₆-Alkyl und C₁-C₆-Alkoxy substituiert sein kann; oder
fünf- bis sechsgliedriges Heteroaryl mit ein bis vier Stickstoffatomen, oder mit ein bis drei Stickstoffatomen und einem Sauerstoff- oder einem Schwefelatom, oder mit einem Sauerstoff oder Schwefelatom; das partiell oder vollständig halogeniert und/oder durch ein bis drei Reste aus der Gruppe C₁-C₆-Alkyl, C₁-C₆-Halogenalkyl, C₁-C₆-Alkoxy, C₁-C₆-Halogenalkoxy, Amino, C₁-C₆-Alkylamino und Di(C₁-C₆alkyl)amino substituiert sein kann, oder
fünf- bis sechsgliedriges Heteroaryl-C₁-C₄-alkyl mit ein bis vier Stickstoffatomen, oder mit ein bis drei Stickstoffatomen und einem Sauerstoff- oder einem Schwefelatom, oder mit einem Sauerstoff oder Schwefelatom; das partiell oder vollständig halogeniert und/oder durch ein bis drei Reste aus der Gruppe C₁-C₆-Alkyl, C₁-C₆-Halogenalkyl, C₁-C₆-Alkoxy, C₁-C₆-Halogenalkoxy, Amino, C₁-C₆-Alkylamino und Di(C₁-C₆alkyl)amino substituiert sein kann;
- R²: Wasserstoff, C₁-C₈-Alkyl, C₂-C₈-Alkenyl, C₃-C₈-Alkinyl, C₃-C₇-Cycloalkyl, wobei die vier letztgenannten Reste teilweise oder vollständig halogeniert sein können; oder
- R¹ und R²: bilden zusammen mit dem N-Atom, an das sie gebunden sind, einen dreibis siebengliedrigen Heterocyclus, welcher seinerseits partiell oder vollständig halogeniert und/oder durch ein bis drei Reste aus der Gruppe C₁-C₆-Alkyl, C₁-C₆-Halogenalkyl und C₁-C₆-Alkoxy substituiert sein kann;
- Q: ein Rest aus der Gruppe Q¹ bis Q³⁹ 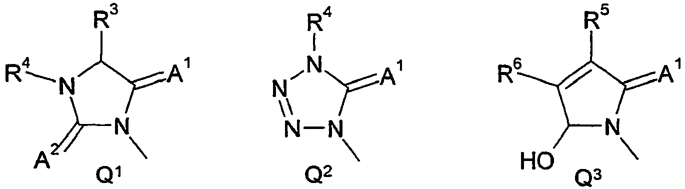 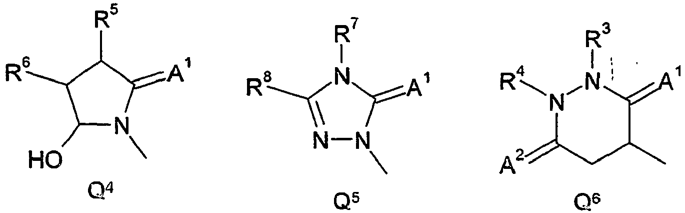 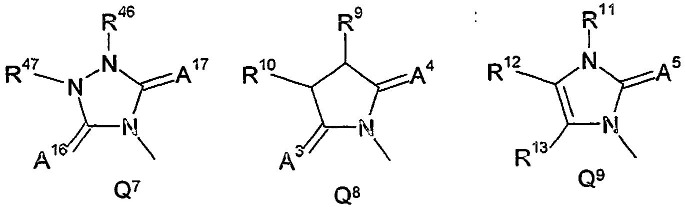 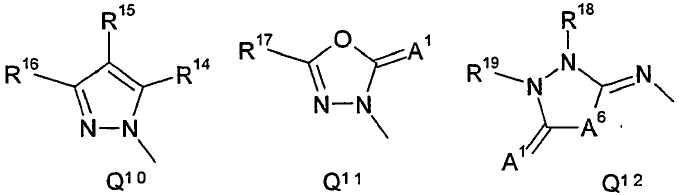 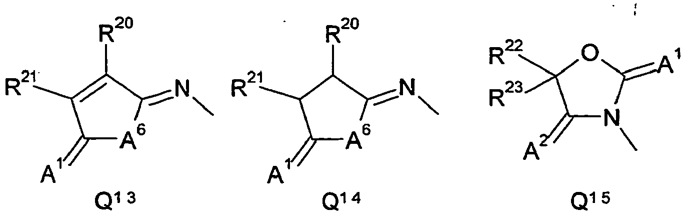 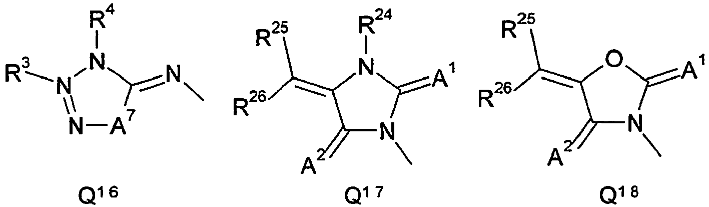 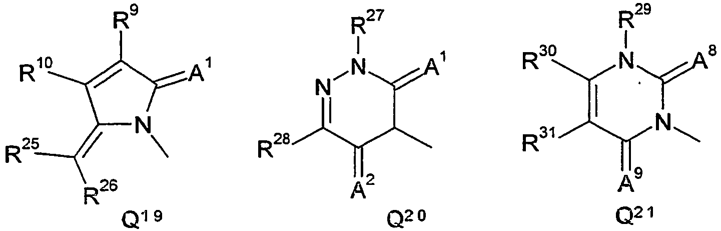 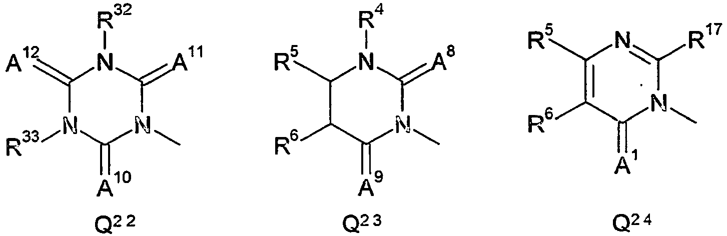 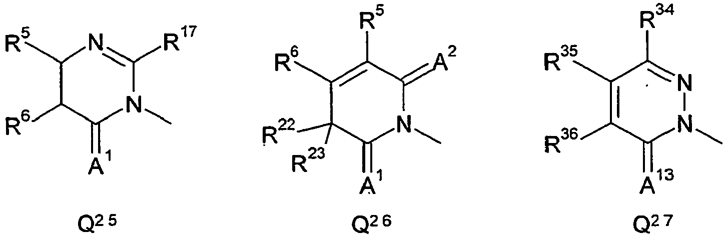 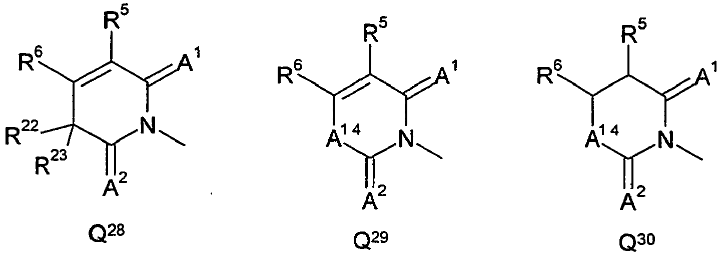 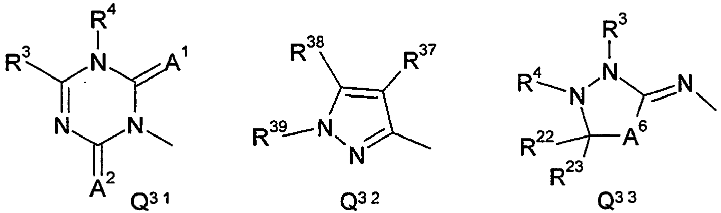 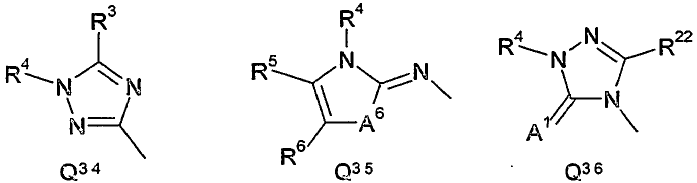 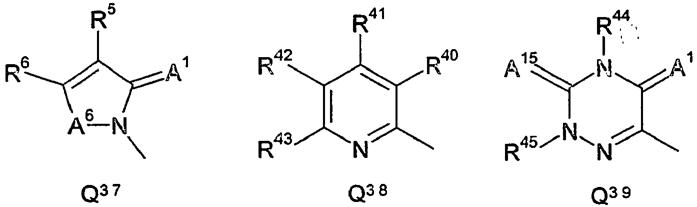
- A¹ bis A¹⁷: Sauerstoff oder Schwefel;
- R³, R⁴, R⁷, R⁸,:
- R¹¹, R¹², R¹⁸, R¹⁹,:
- R²⁷, R²⁹, R³², R³³,:
- R³⁸, R³⁹, R⁴⁴, R⁴⁵,:
- R⁴⁶ und R⁴⁷: Wasserstoff, Cyano, Hydroxy, C₁-C₆-Alkyl, C₁-C₆-Cyanoalkyl, C₁-C₆-Halogenalkyl, C₃-C₇-Cycloalkyl, C₃-C₇-Cycloalkyloxy, C₁-C₆-Alkoxy, C₁-C₆-Halogenalkoxy, C₂-C₆-Alkenyl, C₂-C₆-Halogenalkenyl, C₂-C₆-Alkenyloxy, C₃-C₆-Alkinyl, C₃-C₆-Alkinyloxy, C₁-C₆-Alkylsulfinyl, C₁-C₆-Alkylsulfonyl, Phenyl-C₁-C₆-alkyl, Amino, C₁-C₆-Alkylamino oder Di(C₁-C₆-alkyl)amino; oder
- R³ und R⁴, R¹¹:
- und R¹², R¹⁸ und R¹⁹,:
- oder R⁴⁶ und R⁴⁷: bilden zusammen mit den Atomen, an die sie gebunden sind, einen drei- bis siebengliedrigen Heterocyclus, welcher seinerseits partiell oder vollständig halogeniert und/oder durch ein bis drei Reste aus der Gruppe C₁-C₆-Alkyl und C₁-C₆-Alkoxy substituiert sein kann;
- R⁵, R⁶, R⁹, R¹⁰,:
- R¹⁵, R¹⁶, R²⁰, R²¹,:
- R³⁰, R³¹, R³⁵, R³⁶,:
- R⁴¹, R⁴² und R⁴³: Wasserstoff, Hydroxy, C₁-C₆-Alkyl, C₁-C₆-Halogenalkyl, C₃-C₇-Cycloalkyl, C₃-C₇-Cycloalkyloxy, C₁-C₆-Alkoxy, C₁-C₆-Halogenalkoxy, C₂-C₆-Alkenyl, C₂-C₆-Halogenalkenyl, C₂-C₆-Alkenyloxy, C₃-C₆-Alkinyl, C₃-C₆-Alkinyloxy, C₁-C₆-Alkylthio, C₁-C₆-Alkyisulfinyl, C₁-C₆-Alkylsulfonyl, C₁-C₆-Alkoxysulfonyl, C₁-C₆-Alkylsulfonyloxy, Amino, C₁-C₆-Alkylamino oder Di(C₁-C₆-alkyl)amino; oder
- R⁵ und R⁶, R⁹ und R¹⁰,:
- R¹⁵ und R¹⁶, R²⁰ und:
- R²¹, oder R³⁰ und R³¹: bilden zusammen mit den Atomen, an die sie gebunden sind, einen drei- bis siebengliedrigen Heterocyclus, welcher seinerseits partiell oder vollständig halogeniert und/oder durch ein bis drei Reste aus der Gruppe C₁-C₆-Alkyl und C₁-C₆-Alkoxy substituiert sein kann;
- R¹³, R¹⁴, R²²,:
- R²³, R²⁵ und R²⁶: Wasserstoff, Halogen oder C₁-C₆-Alkyl;
- R¹⁷, R²⁸, R³⁴,:
- R³⁷ oder R⁴⁰: Wasserstoff, Halogen, Hydroxy, C₁-C₆-Alkyl, C₁-C₆-Halogenalkyl, C₃-C₇-Cycloalkyl, C₃-C₇-Cycloalkyloxy, C₁-C₆-Alkoxy, C₁-C₆-Halogenalkoxy C₁-C₆-Alkylthio, C₁-C₆-Halogenalkylthio, C₂-C₆-Alkenyl, C₂-C₆-Halogenalkenyl, C₂-C₆-Alkenyloxy, C₃-C₆-Alkinyl oder C₃-C₆-Alkinyloxy;
- R²⁴: Wasserstoff, C₁-C₆-Alkyl, C₁-C₆-Halogenalkyl, C₂-C₆-Alkenyl, C₃-C₆-Alkinyl, C₁-C₆-Halogenalkoxy, Amino, C₁-C₆-Alkylamino oder Di(C₁-C₆-alkyl)amino;
sowie deren landwirtschaftlich brauchbaren Salze.

Außerdem betrifft die Erfindung Verfahren und Zwischenprodukte zur Herstellung von Verbindungen der Formel I, Mittel, welche diese enthalten, sowie die Verwendung dieser Derivate oder diese enthaltende Mittel zur Bekämpfung unerwünschter Pflanzen.

Des Weiteren betrifft die Erfindung die Verwendung der Verbindungen der Formel I oder diese enthaltende Mittel zur Desikkation und/oder Defoliation von Pflanzen.

Ferner betrifft die Erfindung Verfahren zur Herstellung von herbiziden Mitteln und Mitteln zur Desikkation / Defoliation von Pflanzen unter Verwendung der Verbindungen I, sowie Verfahren zur Bekämpfung von unerwünschtem Pflanzenwuchs oder zur Desikkation / Defoliation von Pflanzen mit den Verbindungen I.

Aus der Literatur, beispielsweise aus WO 96/07323, WO 96/08151, WO 97/42176 und DE 44 37 197 sind substituierte Phenyluracile bekannt. Phenylpyrazole werden in WO 95/32188 beschrieben. Bicyclische Triazolone werden in WO 02/38562 beschrieben. Weiterhin sind aus der Literatur phenylsubstituierte Pyrimidin(thi)one (WO 96/07647), Phenylpyridazone (WO 99/52878) und Triazolderivate (WO 96/18618) bekannt. In WO 93/03019 werden phenylsubstituierte Sulfonamide offenbart.

Die herbiziden oder desikkanten / defolianten Eigenschaften der bisher bekannten Verbindungen bzw. die Verträglichkeiten gegenüber Kulturpflanzen sind jedoch nicht immer voll befriedigend. Es lag daher dieser Erfindung die Aufgabe zugrunde, neue, insbesondere herbizid wirksame, Verbindungen mit verbesserten Eigenschaften zu finden.

Die Aufgabe erstreckt sich auch auf die Bereitstellung neuer desikkant / defoliant wirksamer Verbindungen.

Demgemäß wurden die Benzolsulfonamid-Derivate der Formel I sowie deren herbizide Wirkung gefunden.

Ferner wurden herbizide Mittel gefunden, welche die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Des Weiteren wurde gefunden, dass die Verbindungen I auch zur Desikkation und Defoliation von Pflanzenteilen geeignet sind, wofür Kulturpflanzen wie Baumwolle, Kartoffel, Raps, Sonnenblume, Sojabohne oder Ackerbohnen, insbesondere Baumwolle, in Betracht kommen. Diesbezüglich wurden Mittel zur Desikkation und/oder Defoliation von Pflanzen, Verfahren zur Herstellung dieser Mittel und Verfahren zur Desikkation und/oder Defoliation von Pflanzen mit den Verbindungen I gefunden.

Die Verbindungen der Formel I können je nach Substitutionsmuster ein oder mehrere Chiralitätszentren enthalten und liegen dann als Enantiomeren oder Diastereomerengemische vor. Gegenstand der Erfindung sind sowohl die reinen Enantiomeren oder Diastereomeren als auch deren Gemische.

Die Verbindungen der Formel I können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl, Hydroxy-C₁-C₄-alkyl, C₁-C₄-Alkoxy-C₁-C₄-alkyl, Hydroxy-C₁-C₄-alkoxy-C₁-C₄-alkyl, Phenyl oder Benzyl ersetzt sein können, vorzugsweise Ammonium, Dimethylammonium, Diisopropylammonium, Tetramethylammonium, Tetrabutylammonium, 2(2Hydroxyeth-1-oxy)eth-1-ylammonium, Di(2-hydroxyeth-1-yl)ammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Die für die Substituenten X³, R¹-R⁴⁷ oder als Reste an Phenyl-, Heterocyclyl- oder Heteroaryl-Resten genannten organischen Molekülteile stellen Sammelbegriffe für individuelle Aufzählungen der einzelnen Gruppenmitglieder dar. Sämtliche Kohlenwasserstoffketten, also alle Alkyl-, Alkylen-, Halogenalkyl-, Cyanoalkyl-, Phenylalkyl-, Alkenyl-, Halogenalkenyl-, Alkinyl-, Alkoxy-, Alkylenoxy-, Halogenalkoxy-, Alkylamino-, Dialkylamino- und Alkoxyalkyl-Teile können geradkettig oder verzweigt sein. Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₁-C₄-Alkyl sowie die Alkylteile von C₁-C₆-Alkoxycarbonyl-C₁-C₄-alkyl, C₂-C₆-Alkynyloxycarbonyl-C₁-C₄-alkyl, Aryl-C₁-C₄-alkyl, Cycloalkyl-C₁-C₄-alkyl, Heterocyclyl-C₁-C₄-alkyl und Heteroaryl-C₁-C₄-alkyl: Methyl, Ethyl, n-Propyl, 1-Methylethyl, n-Butyl, 1-Methylpropyl, 2-Methylpropyl und 1,1-Dimethylethyl;
- C₁-C₆-Alkyl sowie die C₁-C₆-Alkylteile von C₁-C₆-Cyanoalkyl, C₁-C₆-Alkylthio, C₁-C₆-Alkylamino, Di(C₁-C₆-alkyl)amino, C₁-C₆-Alkylsulfinyl, C₁-C₆-Alkylsulfonyl, C₁-C₆-Alkylcarbonyl, C₁-C₆-Alkylthiocarbonyl, C₁-C₆-Alkylaminocarbonyl, Di(C₁-C₆-alkyl)aminocarbonyl: C₁-C₄-Alkyl, wie voranstehend genannt, sowie z.B. n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und 1-Ethyl-3-methylpropyl;
- C₁-C₈-Alkyl: C₁-C₆-Alkyl, wie voranstehend genannt, sowie z.B. Heptyl, 2-Methylhexyl, 3-Methylhexyl, 2,2-Dimethylpentyl, 2,3-Dimethylpentyl, 2,4-Dimethylpentyl, 3,3-Dimethylpentyl, 2,2-Dimethyl-3-methylbutyl, Octyl, 2-Methylheptyl, 3-methylheptyl, 4-Methylheptyl, 2,2-Diemthylhexyl, 2,3-Dimethylhexyl, 2,4-Dimethylhexyl, 3,3-Dimethylhexyl, 2,2,3-Trimethylpentyl, 2,3,3-Trimethylpentyl, 2,3,4-Trimethylpentyl und 2,2,3,3-Tetramethylbutyl;
- C₃-C₇-Cycloalkyl sowie die C₃-C₇-Cycloalkyl-Teile von C₃-C₇-Cycloalkyl-C₁-C₄-alkyl und C₃-C₇-Cycloalkyloxy: monocyclischer, gesättigter Kohlenwasserstoff mit 3 bis 7 Ringgliedern, wie Cyclopropyl, Cyclobutyl, Cyclopentyl, Cyclohexyl und Cyclo-heptyl;
- C₅-C₇-Cycloalkenyl: monocyclischer, ungesättigter Kohlenwasserstoff mit 5 bis 7 Ringgliedern, z.B. 1-Cyclopentenyl, 2-Cyclopentenyl, 3-Cyclopentenyl, 2,4-Cyclopentadienyl, 1-Cyclohexenyl, 2-Cyclohexenyl, 3-Cyclohexenyl, 1,3-Cyclohexadienyl, 2,5-Cyclohexadienyl, 1-Cycloheptenyl, 2-Cycloheptenyl, 3-Cycloheptenyl, 4-Cycloheptenyl, 2,6-Cycloheptadienyl, 3,5-Cycloheptadienyl;
- vier- bis sechsgliedriges Heterocyclyl: monocyclischer, gesättigter oder partiell ungesättigter Kohlenwasserstoff mit vier bis sechs Ringgliedern wie voranstehend genannt, welche neben Kohlenstoffatomen ein bis vier Stickstoffatome, ein oder zwei Sauerstoffatome, ein Schwefelatom, ein bis drei Stickstoffatome und ein Sauerstoff- oder ein Schwefelatom, oder ein Sauerstoff und ein Schwefelatom enthalten können, und welche über ein C-Atom oder ein N-Atom verknüpft sein können,
   z.B. 2-Oxetanyl, 3-Oxetanyl, 3-Thiethanyl, 1-Azetidinyl, 2-Azetidinyl, 1-Azetinyl, 2-Azetinyl;
   z.B. 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 1,2,3,4-Tetrazolidin-5-yl;
   z.B. 1-Pyrrolidinyl, 2-Isothiazolidinyl, 2-Isothiazolidinyl, 1-Pyrazolidinyl, 3-Oxazolidinyl, 3-Thiazolidinyl, 1-Imidazolidinyl, 1,2,4-Triazolidin-1-yl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,3,4-Tetrazolidin-5-yl,
   z.B. 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 4,5-Dihydropyrrol-2-yl, 4,5-Dihydropyrrol-3-yl, 2,5-Dihydropyrrol-2-yl, 2,5-Dihydropyrrol-3-yl, 4,5-Dihydroisoxazol-3-yl, 2,5-Dihydroisoxazol-3-yl, 2,3-Dihydroisoxazol-3-yl, 4,5-Dihydroisoxazol-4-yl, 2,5-Dihydroisoxazol-4-yl, 2,3-Dihydroisoxazol-4-yl, 4,5-Dihydroisoxazol-5-yl, 2,5-Dihydroisoxazol-5-yl, 2,3-Dihydroisoxazol-5-yl, 4,5-Dihydroisothiazol-3-yl, 2,5-Dihydroisothiazol-3-yl, 2,3-Dihydroisothiazol-3-yl, 4,5-Dihydroisothinol-4-yl, 2,5-Dihydroisothiazol-4-yl, 2,3-Dihydroisothiazol-4-yl, 4,5-Dihydroisothiazol-5-yl, 2,5-Dihydroisothiazol-5-yl, 2,3-Dihydroisothiazol -5-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-3-yl, 3,4-Dihydropyrazol-4-yl, 3,4-Dihydropyrazol-5-yl, 4,5-Dihydropyrazol-3-yl, 4,5-Dihydropyrazol-4-yl, 4,5-Dihydropyrazol-5-yl, 2,3-Dihydroimidazol-2-yl, 2,3-Dihydroimidazol-3-yl ,2,3-Dihydroimidazol-4-yl, 2,3-Dihydroimidazol-5-yl, 4,5-Dihydroimidazol-2-yl, 4,5-Dihydroimidazol-4-yl, 4,5-Dihydroimidazol-5-yl, 2,5-Dihydroimidazol-2-yl, 2,5-Dihydroimidazol-4-yl, 2,5-Dihydroimidazol-5-yl, 2,3-Dihydrooxazol-3-yl, 2,3-Dihydrooxazol-4.-yl, 2,3-Dihydrooxazol-5-yl, 3,4-Dihydrooxazol-3-yl, 3,4-Dihydrooxazol-4-yl, 3,4-Dihydrooxazol-5-yl, 2,3-Dihydrothiazol-3-yl, 2,3-Dihydrothiazol-4-yl, 2,3-Dihydrothiazol-5-yl, 3,4-Dihydrothiazol-3-yl, 3,4-Dihydrothiazol-4-yl, 3,4-Dihydrothiazol-5-yl, 3,4-Dihydrothiazol-2-yl, 3,4-Dihydrothiazol-3-yl, 3,4-Dihydrothiazol-4-yl,
   z.B. 4,5-Dihydropyrrol-1-yl, 2,5-Dihydropyn-ol-1-yl, 4,5-Dihydroisoxazol-2-yl, 2,3-Dihydroisoxazol-1-yl, 4,5-Dihydroisothiazol-1-yl, 2,3-Dihydroisothiazol-1-yl, 2,3-Dihydropyrazol-1-yl, 4,5-Dihydropyrazol-1-yl, 3,4-Dihydropyrazol-1-yl, 2,3-Dihydroimidazol-1-yl, 4,5-Dihydroimidazol-1-yl, 2,5-Dihydroimidazol-1-yl, 2,3-Dihydrooxazol-2-yl, 3,4-Dihydrooxazol-2-yl, 2,3-Dihydrothiazol-2-yl, 3,4-Dihydrothiazol-2-yl;
   z.B. 2-Piperidinyl, 3-Piperidinyl, 4-Piperidinyl, 1,3-Dioxan-5-yl, 1,3-Dithian-5-yl, 2-Tetrahydropyranyl, 4-Tetrahydropyranyl, 2-Tetrahydrothiopyranyl, 4-Tetrahydrothiopyranyl 3-Hexahydropyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydrotriazin-2-yl, 1,2,4-Hexahydrotriazin-3-yl, Tetrahydro-1,3-oxazin-2-yl, Tetrahydro-1,3-oxazin-6-yl, 2-Morpholinyl, 3-Morpholinyl, ;
   z.B. 1-Piperidinyl, 1-Hexahydropyridazinyl, 1-Hexahydropyrimidinyl, 1-Piperazinyl, 1,3,5-Hexahydrotriazin-1-yl, 1,2,4-Hexahydrotriazin-1-yl, Tetrahydro-1,3-oxazin-1-yl, 1-Morpholinyl;
   z.B. 2H-Pyran-2-yl, 2H-Pyran-3-yl, 2H-Pyran-4-yl, 2H-Pyran-5-yl, 2H-Pyran-6-yl, 2H-Thiopyran-2-yl, 2H-Thiopyran-3-yl, 2H-Thiopyran-4-yl, 2H-Thiopyran-5-yl, 2H-Thiopyran-6-yl, 5,6-Dihydro-4H-1,3-oxazin-2-yl;
- drei- bis siebengliedriges Heterocyclyl: vier- bis sechsgliedriges Heterocyclyl wie voranstehend genannt sowie
   z.B. 2-Oxrianyl, 1-Aziridinyl, 2-Aziridinyl, 2-Thiiranyl;
   z.B. Azepan-2-yl, Azepan-3-yl, Azepan-4-yl, Oxepan-2-yl, Oxepan-3-yl, Oxepan-4-yl, Thiepan-2-yl, Thiepan-3-yl, Thiepan-4-yl, 1,2-Diazepan-3-yl, 1,2-Diazepan-4-yl, 1,2-Diazepan-5-yl;
   z.B. Azepan-1-yl, 1,2-Diazepan-1-yl, 1,4-Oxazepan-4-yl, 1,4-Thiazepan-4-yl;
   z.B. 2,3,6,7-Tetrahydro-1H-azepin-2-yl, 2,3,6,7-Tetrahydro-1H-azepin-3-yl, 2,3,6,7-Tetrahydro-1H-azepin-4-yl, 2,3,4,5-Tetrahydro-1H-azepin-2-yl, 2,3,4,5-Tetrahydro-1H-azepin-3-yl, 2,3,4,5-Tetrahydro-1H-azepin-4-yl, 1H-Azepin-2-yl, 1H-Azepin-3-yl, 1H-Azepin-4-yl, Oxepin-2-yl, Oxepin-3-yl, Oxepin-4-yl, Thiepin-2-yl, Thiepin-3-yl, Thiepin-4-yl, 1,4-Oxazepin-2-yl, 1,4-Oxazepin-3-yl, 1,4-Oxazepin-5-yl, 1,4-Oxazepin-6-yl, 1,4-Oxazepin-7-yl, 1,4-Thiazepin-2-yl, 1,4-Thiazepin-3-yl, 1,4-Thiazepin-5-yl, 1,4-Thiazepin-6-yl, 1,4-Thiazepin-7-yl, 4,5,6,7-Tetrahydro-1H-[1,3]-diazepin-2-yl, 4,5,6,7-Tetrahydro-1H-[1,3]-diazepin-4-yl, 4,5,6,7-Tetrahydro-1H-[1,3]-diazepin-5-yl, 4,5,6,7-Tetrahydro-1H-[1,3]-diazepin-6-yl, 4,5,6,7-Tetrahydro-1H-[1,3]-diazepin-7-yl, 2,3,4,5-Tetrahydro-1H-[1,4]-diazepin-2-yl, 2,3,4,5-Tetrahydro-1H-[1 ,4]-diazepin-3-yl, 2,3,4,5-Tetrahydro-1H-[1,4]-diazepin-5-yl, 2,3,4,5-Tetrahydro-1H-[1 ,4]-diazepin-6-yl, 2,3,4,5-Tetrahydro-1H-[1,4]-diazepin-7-yl, 2,3-Dihydro-1H-[1,2]diazepin-3-yl, 2,3-Dihydro-1H-[1,2]diazepin-4-yl, 2,3-Dihydro-1H-[1,2]diazepin-5-yl, 2,3-Dihydro-1H-[1,2]diazepin-6-yl, 2,3-Dihydro-1H-[1,2]diazepin-7-yl, 4,7-Dihydro-[1,4]-oxazepin-2-yl, 4,7-Dihydro-[1,4]-oxazepin-3-yl, 4,7-Dihydro-[1,4]-oxazepin-5-yl, 4,7-Dihydro-[1,4]-oxazepin-6-yl, 4,7-Dihydro-[1,4]-oxazepin-7-yl, 2,3-Dihydro-[1,3]-thiazepin-2-yl, 2,3-Dihydro-[1,3]-thiazepin-4-yl, 2,3-Dihydro-[1,3]-thiazepin-5-yl, 2,3-Dihydro-[1,3]-thiazepin-6-yl, 2,3-Dihydro-[1,3]-thiazepin-7-yl;
   z.B. Azepin-1-yl, 2,3,6,7-Tetrahydroazepin-1-yl, 2,3,4,5-Tetrahydroazepin-1-yl, 4,5,6,7-Tetrahydro-[1,3]-diazepin-1-yl, 2,3,4,5-Tetrahydro-[1,4]-diazepin-1-yl, 2,3-Dihydro-[1,2]diazepin-1-yl, 4,7-Dihydro-[1,4]-oxazepin-4-yl, 2,3-Dihydro-[1,3]-thiazepin-3-yl;
- C₂-C₄-Alkenyl sowie die Alkenylteile von C₁-C₆-Alkoxycarbonyl-C₂-C₄-alkenyl: Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl;
- C₃-C₆-Alkenyl: z.B. 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
- C₂-C₆-Alkenyl sowie die C₂-C₆-Alkenylteile von C₂-C₆-Alkenyloxy, C₂-C₆-Alkenylcarbonyl, C₂-C₆-Alkenyloxycarbonyl, C₂-C₆-Alkenyloxycarbonyl-C₁-C₄-alkyl, C₂-C₆-Alkenylthiocarbonyl, C₂-C₆-Alkenylaminocarbonyl, Di-(C₂-C₆-alkenyl)aminocarbonyl: C₃-C₆-Alkenyl wie voranstehend genannt, sowie Ethenyl;
- C₂-C₈-Alkenyl sowie die C₂-C₈-Alkenylteile von C₂-C₈-Alkenyloxy: C₂-C₆-Alkenyl wie voranstehend genannt, sowie z.B. 1-Heptenyl, 2-Heptenyl, 3-Heptenyl, 2-Methyl-1-hexenyl, 2-Methyl-2-hexenyl, 2-Methyl-3-hexenyl, 2-Methyl-4-hexenyl, 2-Methyl-5-hexenyl, 3-Methyl-1-hexenyl, 3-Methyl-2-hexenyl, 3-Methyl-3-hexenyl, 3-Methyl-4-hexenyl, 3-Methyl-5-hexenyl, 2,2-Dimethyl-3-pentenyl, 2,2-Dimethyl-4-pentenyl, 2,3-Dimethyl-1-pentenyl, 2,3-Dimethyl-2-pentenyl, 2,3-Dimethyl-3-pentenyl, 2,3-Dimethyl-4-pentenyl, 2,4-Dimethyl-1-pentenyl, 2,4-Dimethyl-2-pentenyl, 3,3-Dimethyl-1-pentenyl, 2,2-Dimethyl-3-methyl-3-butentyl, 1-Octenyl, 2-Octenyl, 3-Octenyl, 4-Octenyl, 2-Methyl-1-heptenyl, 2-Methyl-2-heptenyl, 2-Methyl-3-heptenyl, 2-Methyl-4-heptenyl, 2-Methyl-5-heptenyl, 2-Methyl-6-heptenyl, 3-Methyl-1-heptenyl, 3-Methyl-2-heptenyl, 3-Methyl-3-heptenyl, 3-Methyl-4.-heptenyl, 3-Methyl-5-heptenyl, 3-Methyl-6-heptenyl, 4-Methyl-1-heptenyl, 4-Methyl-2-heptenyl, 4-Methyl-3-heptenyl, 2,2-Dimethyl-3-hexenyl, 2,2-Dimethyl-4-hexenyl, 2,2-Dimethyl-5-hexenyl, 2,3-Dimethyl-1-hexenyl, 2,3-Dimethyl-2-hexenyl, 2,3-Dimethyl-3-hexenyl, 2,3-Dimethyl-4-hexenyl, 2,3-Dimethyl-5-hexenyl, 2,4-Dimethyl-1-hexenyl, 2,4-Dimethyl-2-hexenyl, 2,4-Dimethyl-3-hexenyl, 2,4-Dimethyl-4-hexenyl, 2,4-Dimethyl-5-hexenyl, 3,3-Dimethyl-1-hexenyl, 3,3-Dimethyl-4-hexenyl, 3,3-Dimethyl-5-hexenyl, 2,2,3-Trimethyl-3-pentenyl, 2,2,3-Trimethyl-4-pentenyl, 2,3,3-Trimethyl-1-pentenyl, 2,3,3-Trimethyl-4-pentenyl, 2,3,4-Trimethyl-1-pentenyl und 2,3,4-Trimethyl-2-pentenyl;
- C₃-C₆-Alkinyl sowie die C₃-C₆-Alkinyl-Teile von C₃-C₆-Alkinyloxy, C₃-C₆-Alkinylcarbonyl, C₃-C₆-Alkinyloxycarbonyl, C₃-C₆-Alkinylthiocarbonyl, C₃-C₆-Alkinylaminocarbonyl, Di(C₃-C₆-alkinyl)aminocarbonyl: z.B. 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
- C₃-C₈-Alkinyl sowie die C₃-C₈-Alkinyl-Teile von C₃-C₈-Alkinyloxy: C₃-C₆-Alkinyl wie voranstehend genannt, sowie z.B. 1-Heptinyl, 2-Heptinyl, 3-Heptinyl, 2-Methyl-3-hexinyl, 2-Methyl-4-hexinyl, 2-Methyl-5-hexinyl, 3-Methyl-1-hexinyl, 3-Methyl-4-hexinyl, 3-Methyl-5-hexinyl, 2,2-Dimethyl-3-pentinyl, 2,2-Dimethyl-4-pentinyl, 2,3-Dimethyl-4-pentinyl, 3,3-Dimethyl-1-pentinyl, 1-Octinyl, 2-Octinyl, 3-Octinyl, 4-Octinyl, 2-Methyl-3-heptinyl, 2-Methyl-4-heptinyl, 2-Methyl-5-heptinyl, 2-Methyl-6-heptinyl, 3-Methyl-1-heptinyl, 3-Methyl-4-heptinyl, 3-Methyl-5-heptinyl, 3-Methyl-6-heptinyl, 4-Methyl-1-heptinyl, 4-Methyl-2-heptinyl, 2,2-Dimethyl-3-hexinyl, 2,2-Dimethyl-4-hexinyl, 2,2-Dimethyl-5-hexinyl, 2,3-Dimethyl-4-hexinyl, 2,3-Dimethyl-5-hexinyl, 2,4-Dimethyl-5-hexinyl, 3,3-Dimethyl-1-hexinyl, 3,3-Dimethyl-4-hexinyl, 3,3-Dimethyl-5-hexinyl, 2,233-Trimethyl -3-pentinyl, 2,2,3-Trimethyl4-pentinyl und 2,3,3-Trimethyl-4-pentinyl;
- C₁-C₄-Halogenalkyl: ein C₁-C₄-Alkylrest wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-lodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl;
- C₁-C₆-Halogenalkyl sowie die C₁-C₆-Halogenalkyl-Teile von C₁-C₆-Halogenalkylthio, C₁-C₆-Halogenalkylsulfinyl, C₁-C₆-Halogenalkylsulfonyl, C₁-C₆-Halogenalkylcarbonyl, C₁-C₆-Halogenalkylthiocarbonyl, C₁-C₆-Halogenalkylaminocarbonyl, Di(C₁-C₆-halogenalkyl)aminocarbonyl: C₁-C₄-Halogenalkyl wie voranstehend genannt, sowie z.B. 5-Fluorpentyl, 5-Chlorpentyl, 5-Brompentyl, 5-lodpentyl, Undecafluorpentyl, 6-Fluorhexyl, 6-Chlorhexyl, 6-Bromhexyl, 6-Iodhexyl und Dodecafluorhexyl;
- C₁-C₈-Halogenalkyl: C₁-C₆-Halogenalkyl wie voranstehend genannt, sowie z.B. 7-Fluorheptyl, 7-Chlorheptyl, 7-Bromheptyl, 7-lodheptyl, Perfluorheptyl, 8-Fluoroctyl, 8-Chloroctyl, 8-Bromoctyl, 8-lodoctyl und Perfluoroctyl;
- C₂-C₆-Halogenalkenyl: ein C₂-C₆-Alkenylrest, wie voranstehend genannt, der partiell oder vollständig durch Fluor, Chlor, Brom und/oder Iod substituiert ist, z.B. 2-Chlorvinyl, 2-Chlorallyl, 3-Chlorallyl, 2,3-Dichlorallyl, 3,3-Dichlorallyl, 2,3,3-Trichlorallyl, 2,3-Dichlorbut-2-enyl, 2-Bromvinyl, 2-Bromallyl, 3-Bromallyl, 2,3-Dibromallyl, 3,3-Dibromallyl, 2,3,3-Tribromallyl oder 2,3-Dibrombut-2-enyl;
- C₂-C₈-Halogenalkenyl: ein C₂-C₆-Halogenalkenylrest wie voranstehend genannt, sowie z.B. 2-Chlor-1-heptenyl, 3-Chlor-1-heptenyl, 2,3-Dichlor-1-heptenyl, 3,3-Dichlor-1-heptenyl, 2,3,3-Trichlor-1-heptenyl, 2-Brom-1-heptenyl, 3-Brom-1-heptenyl, 2,3-Dibrom-1-heptenyl, 3,3-Dibrom-1-heptenyl, 2,3,3-Tribrom-1-heptenyl, 2-Chlor-1-octenyl, 3-Chlor-1-octenyl, 2,3-Dichlor-1-octenyl, 3,3-Dichlor-1-octenyl, 2,3,3-Trichlor-1-octenyl, 2-Brom-1-octenyl, 3-Brom-1-octenyl, 2,3-Dibrom-1-octenyl, 3,3-Dibrom-1-octenyl und 2,3,3-Tribrom-1-octenyl;
- C₃-C₆-Halogenalkinyl: ein C₃-C₆-Alkinylrest, wie voranstehend genannt, der partiell oder vollständig durch Fluor, Chlor, Brom und/oder Iod substituiert ist, z.B. 1,1-Difluor-prop-2-in-1-yl, 3-lod-prop-2-in-1-yl, 4-Fluorbut-2-in-1-yl, 4-Chlorbut-2in-1-yl, 1,1-Difluorbut-2-in-1-yl, 4-lodbut-3-in-1-yl, 5-Fluorpent-3-in-1-yl, 5-lodpent-4-in-1-yl, 6-Fluorhex-4-in-1-yl oder 6-lodhex-5-in-1-yl;
- C₁-C₄-Alkoxy sowie die C₁-C₄-Alkoxy-Teile von C₁-C₄-Alkoxy-C₁-C₄-alkyl und Phenyl-C₁-C₄-alkoxy: z.B. Methoxy, Ethoxy, Propoxy, 1-Methylethoxy, Butoxy, 1-Methylpropoxy, 2-Methylpropoxy und 1,1-Dimethylethoxy;
- C₁-C₆-Alkoxy sowie die C₁-C₆-Alkoxyteile von C₁-C₆-Alkoxy-C₁-C₄-alkyl, C₁-C₆-Alkoxycarbonyl, C₁-C₆-Alkoxycarbonyl-C₁-C₄-alkyl, C₁-C₆-Alkoxycarbonyl-C₂-C₄-alkenyl, C₁-C₆. Alkoxysulfonyl: C₁-C₄-Alkoxy wie voranstehend genannt, sowie z.B. Pentoxy, 1-Methylbutoxy, 2-Methylbutoxy, 3-Methoxylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy,1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy;
- C₁-C₈-Alkoxy: C₁-C₆-Alkoxy wie voranstehend genannt, sowie z.B. Heptoxy, 2-Methylhexoxy, 3-Methylhexoxy, 2,2-Dimethylpentoxy, 2,3-Dimethylpentoxy, 2,4-Dimethylpentoxy, 3,3-Dimethylpentoxy, 2,2-Dimethyl-3-methylbutoxy, Octoxy, 2-Methylheptoxy, 3-Methylheptoxy, 4-Methylheptoxy, 2,2-Diemthylhexoxy, 2,3-Dimethylhexoxy, 2,4-Dimethylhexoxy, 3,3-Dimethylhexoxy, 2,2,3-Triimethylpentoxy, 2,3,3-Trimethylpentoxy, 2,3,4-Trimethylpentoxy und 2,2,3,3-Tetramethylbutoxy;
- C₁-C₄-Halogenalkoxy: einen C₁-C₄-Alkoxyrest wie voranstehend genannt, der partiell oder vollständig durch Fluor, Chlor, Brom und/oder Iod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Brommethoxy, 2-lodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy und Nonafluorbutoxy;
- C₁-C₆-Halogenalkoxy sowie die C₁-C₆-Halogenalkoxy-Teile von C₁-C₆-Halogenalkoxycarbonyl: C₁-C₄-Halogenalkoxy wie voranstehend genannt, sowie z.B. 5-Fluorpentoxy, 5-Chlorpentoxy, 5-Brompentoxy, 5-lodpentoxy, Undecafluorpentoxy, 6-Fluorhexoxy, 6-Chlorhexoxy, 6-Bromhexoxy, 6-Iodhexoxy und Dodecafluorhexoxy;
- C₁-C₆-Alkoxy-C₁-C4-alkyl sowie die Alkylreste von C₁-C₆-Alkylthio-C₁-C₄-alkyl:ein C₁-C₄-Alkyl, welches durch C₁-C₆-Alkoxy wie vorstehend genannt, substituiert ist also z.B. Methoxymethyl, Ethoxymethyl, Propoxymethyl, (1-Methylethoxy)methyl, Butoxymethyl, (1-Methylpropoxy)methyl, (2-Methylpropoxy)methyl, (1,1-Dimethylethoxy)methyl, 2-(Methoxy)ethyl, 2-(Ethoxy)ethyl, 2-(Propoxy)ethyl, 2-(1-Methylethoxy)ethyl, 2-(Butoxy)ethyl, 2-(1-Methylpropoxy)ethyl, 2-(2-Methylpropoxy)ethyl, 2-(1,1-Dimethylethoxy)ethyl, 2-(Methoxy)-propyl, 2-(Ethoxy)propyl, 2-(Propoxy)propyl, 2-(1-Methylethoxy)propyl, 2-(Butoxy)propyl, 2-(1-Methylpropoxy)propyl, 2-(2-Methylpropoxy)propyl, 2-(1,1-Dimethylethoxy)propyl, 3-(Methoxy)propyl, 3-(Ethoxy)-propyl, 3-(Propoxy)propyl, 3-(1-Methylethoxy)propyl, 3-(Butoxy)propyl, 3-(1-Methylpropoxy)propyl, 3-(2-Methylpropoxy)propyl, 3-(1,1-Dimethylethoxy)propyl, 2-(Methoxy)-butyl, 2-(Ethoxy)butyl, 2-(Propoxy)butyl, 2-(1-Methylethoxy)butyl, 2-(Butoxy)butyl, 2-(1-Methylpropoxy)butyl, 2-(2-Methylpropoxy)butyl, 2-(1,1-Dimethylethoxy)butyl, 3-(Methoxy)butyl, 3-(Ethoxy)-butyl, 3-(Propoxy)butyl, 3-(1-Methylethoxy)butyl, 3-(Butoxy)-butyl, 3-(1-Methylpropoxy)butyl, 3-(2-Methylpropoxy)butyl, 3-(1,1-Dimethylethoxy)butyl, 4-(Methoxy)butyl, 4-(Ethoxy)butyl, 4-(Propoxy)butyl, 4-(1-Methylethoxy)butyl, 4-(Butoxy)butyl, 4-(1-Methylpropoxy)butyl, 4-(2-Methylpropoxy)butyl und 4-(1,1-Dimethylethoxy)butyl;
- C₁-C₆-Alkoxycarbonyl-C₁-C₄-alkyl: durch C₁-C₆-Alkoxycarbonyl wie vorstehend genannt substituiertes C₁-C₄-Alkyl, also z.B. für Methoxycarbonylmethyl, Ethoxycarbonylmethyl, Propoxycarbonylmethyl, (1-Methylethoxycarbonyl)methyl, Butoxycarbonylmethyl, (1-Methylpropoxycarbonyl)methyl, (2-Methylpropoxycarbonyl)methyl, (1,1-Dimethylethoxycarbonyl)methyl, 2-(Methoxycarbonyl)ethyl, 2-(Ethoxycarbonyl)ethyl, 2-(Propoxycarbonyl)ethyl, 2-(1-Methylethoxycarbonyl)ethyl, 2-(Butoxycarbonyl)ethyl, 2-(1-Methylpropoxycarbonyl)ethyl, 2-(2-Methylpropoxycarbonyl)ethyl, 2-(1,1-Dimethylethoxycarbonyl)ethyl, 2-(Methoxycarbonyl)propyl, 2-(Ethoxycarbonyl)propyl, 2-(Propoxycarbonyl)propyl, 2-(1-Methylethoxycarbonyl)propyl, 2-(Butoxycarbonyl)propyl, 2-(1-Methylpropoxycarbonyl)propyl, 2-(2-Methylpropoxycarbonyl)propyl, 2-(1,1-Dimethylethoxycarbonyl)propyl, 3-(Methoxycarbonyl)- propyl, 3-(Ethoxycarbonyl)propyl, 3-(Propoxycarbonyl)propyl, 3-(1-Methylethoxycarbonyl)propyl, 3-(Butoxycarbonyl)propyl, 3-(1-Methylpropoxycarbonyl)propyl, 3-(2-Methylpropoxycarbonyl)propyl, 3-(1,1-Dimethylethoxycarbonyl)propyl, 2-(Methoxycarbonyl)butyl, 2-(Ethoxycarbonyl)butyl, 2-(Propoxycarbonyl)butyl, 2-(1-Methylethoxycarbonyl)butyl, 2-(Butoxycarbonyl)butyl, 2-(1-Methylpropoxycarbonyl)butyl, 2-(2-Methylpropoxycarbonyl)butyl, 2-(1,1-Dimethylethoxycarbonyl)butyl, 3-(Methoxycarbonyl)butyl, 3-(Ethoxycarbonyl)butyl, 3-(Propoaycarbonyl)butyl, 3-(1-Methylethoxycarbonyl)butyl, 3-(Butoxycarbonyl)butyl, 3-(1-Methylpropoxycarbonyl)butyl, 3-(2-Methylpropoxycarbonyl)butyl, 3-(1,1-Dimethylethoxycarbonyl)butyl, 4-(Methoxycarbonyl)butyl, 4-(Ethoxycarbonyl)butyl, 4-(Propoxycarbonyl)butyl, 4-(1-Methylethoxycarbonyl)butyl, 4-(Butoxycarbonyl)butyl, 4-(1-Methylpropoxy)butoxy, 4-(2-Methylpropoxy)butoxy und 4-(1,1-Dimethylethoxycarbonyl)butyl;
- C₁-C₄-Alkylthio: z.B. Methylthio, Ethylthio, n-Propylthio, 1-Methylethylthio, Butylthio, 1-Methylpropylthio, 2-Methylpropylthio und 1,1-Dimethylethylthio;
- C₁-C₆-Alkylthio: C₁-C₄-Alkylthio wie voranstehend genannt, sowie z.B. Pentylthio, Methylbutylthio, 2-Methylbutylthio, 3-Methylbutylthio, 2,2-Dimethylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio;
- C₁-C₈-Alkylthio: C₁-C₆-Alkylthio wie voranstehend genannt, sowie die Alkylthio-Teile von C₁-C₈-Alkylthio-C₁-C₈-alkyl, sowie z.B. Heptylthio, 2-Methylhexylthio, 3-Methylhexylthio, 2,2-Dimethylpentylthio, 2,3-Dimethylpentylthio, 2,4-Dimethylpentylthio, 3,3-Dimethylpentylthio, 2,2-Dimethyl-3-methylbutylthio, Octylthio, 2-Methylheptylthio, 3-Methylheptylthio, 4-Methylheptylthio, 2,2-Diemthylhexylthio, 2,3-Dimethylhexylthio, 2,4-Dimethylhexylthio, 3,3-Dimethylhexylthio, 2,2,3-Trimethylpentylthio, 2,3,3-Trimethylpentylthio, 2,3,4-Trimethylpentylthio und 2,2,3,3-Tetramethylbutylthio;
- C₁-C₆-Alkylamino:z.B. Methylamino, Ethylamino, Propylamino, 1-Methylethylamino, Butylamino, 1-Methylpropylamino, 2-Methylpropylamino, 1,1-Dimethylethylamino, Pentylamino, 1-Methylbutylamino, 2-Methylbutylamino, 3-Methylbutylamino, 2,2-Dimethylpropylamino, 1-Ethylpropylamino, Hexylamino, 1,1-Dimethylpropylamino, 1,2-Dimethylpropylamino, 1-Methylpentylamino, 2-Methylpentylamino, 3-Methylpentylamino, 4-Methylpentylamino, 1,1-Dimethylbutylamino, 1,2-Dimethylbutylamino, 1,3-Dimethylbutylämino, 2,2-Dimethylbutylamino, 2,3-Dimethylbutylamino, 3,3-Dimethylbutylamino, 1-Ethylbutylamino, 2-Ethylbutylamino, 1,1,2-Trimethylpropylamino, 1,2,2-Trimethylpropylamino, 1-Ethyl-1-methylpropylamino oder 1-Ethyl-2-methylpropylamino;
- Di(C₁-C₄-alkyl)amino: z.B. N,N-Dimethylamino, N,N-Diethylamino, N,N-Dipropylamino, N,N-Di(1-methylethyl)amino, N,N-Dibutylamino, N,N-Di(1-methylpropyl)amino, N,N-Di(2-methylpropyl)amino, N,N-Di(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)-amino, N-Methyl-N-(2-methylpropyl)amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino, N-(1,1-Dimethylethyl)-N-(1-methylethyl)amino, N-Butyl-N-(1-methylpropyl)amino, N-Butyl-N-(2-methylpropyl)amino, N-Butyl-N-(1,1-dimethylethyl)amino, N-(1-Methylpropyl)-N-(2-methylpropyl)amino, N-(1,1-Dimethylethyl)-N-(1-methylpropyl)amino und N-(1,1-Dimethylethyl)-N-(2-methylpropyl)amino;
- Di(C₁-C₆-alkyl)amino: Di(C₁-C₄-alkyl)amino wie voranstehend genannt, sowie die Dialkylamino-Teile von Di(C₁-C₆-alkyl)amino-C₁-C₆-alkyl, Di(C₁-C₆-alkyl)-aminocarbonyl und Di(C₁-C₆-alkyl)aminocarbonyl-C₁-C₆-alkyl: z.B. N,N-Dipentylamino, N,N-Dihexylamino, N-Methyl-N-pentylamino, N-Ethyl-N-pentylamino, N-Methyl-N-hexylamino und N-Ethyl-N-hexylamino;
- Aryl sowie die Aryl-Teile von Aryloxy und Aryl-C₁-C₄-alkyl: ein- bis dreikemiger aromatischer Carbocyclus mit 6 bis 14 Ringgliedern, wie z.B. Phenyl, Naphthyl und Anthracenyl;
- 5-oder 6-gliedriges Heteroaryl sowie die 5-oder 6-gliedrigen Heteroaryl-Teile von 5-oder 6-gliedrigem Heteroaryl-C₁-C₆-alkyl: aromatische 5- oder 6-Ring-Heterocyclen, welche neben Kohlenstoffatomen ein bis vier Stickstoffatome, oder ein bis drei Stickstoffatome und ein Schwefel- oder Sauerstoffatom, oder ein Sauerstoff- oder Schwefelatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl und Tetrazol-2-yl; 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl, 1,2,4-Triazin-5-yl, 1,2,4-Triazin-6-yl und 1,2,4,5-Tetrazinyl;

In einer besonderen Ausführungsform haben die Variablen der Verbindungen der Formel I folgende Bedeutungen, wobei diese für sich allein betrachtet als auch in Kombination miteinander besondere Ausgestaltungen der Verbindungen der Formel I darstellen:

Bevorzugt sind die Benzolsulfonamide der Formel I, in der
- X¹: Wasserstoff, Fluor oder Chlor;
besonders bevorzugt Wasserstoff oder Fluor;
insbesondere bevorzugt Fluor;
bedeutet.

Ebenso bevorzugt sind die Benzolsulfonamid-Derivate der Formel I, in der
- X³: Wasserstoff, Cyano, C₁-C₆-Alkyl oder Phenyl-C₁-C₄-alkyl; besonders bevorzugt Wasserstoff, Cyano, C₁-C₄-Alkyl wie CH₃, und C₂H₅, oder Benzyl;
insbesondere bevorzugt Wasserstoff oder Cyano;
außerordentlich bevorzugt Wasserstoff;
bedeutet.

Ebenso bevorzugt sind die Benzolsulfonamid-Derivate der Formel I, in der
- Y: eine Gruppe C(A)B, wobei A und B für Sauerstoff stehen;
bedeutet.

Ebenso bevorzugt sind die Benzolsulfonamid-Derivate der Formel I, in der
- R¹: Wasserstoff, Hydroxy, C₁-C₈-Alkyl, C₁-C₈-Halogenalkyl, C₃-C₇-Cycloalkyl, C₃-C₇-Cycloalkyl-C₁-C₄-alkyl, C₂-C₈-Alkenyl, C₂-C₈-Halogenalkenyl, C₃-C₈-Alkinyl, C₁-C₈-Alkoxy, C₁-C₆-Alkoxy-C₁-C₄-alkyl, C₁-C₆-Alkoxycarbonyl-C₁-C₄-alkyl, C₁-C₆-Alkoxycarbonyl-C₂-C₄-alkenyl, C₂-C₆-Alkenyloxycarbonyl-C₁-C₄-alkyl, C₃-C₆-Alkinyloxycarbonyl-C₁-C₄-alkyl, C₁-C₆-Alkylthio-C₁-C₄-alkyl, Aryl, Aryl-C₁-C₄-alkyl;
vier- bis sechsgliedriges Heterocyclyl, welches partiell oder vollständig halogeniert sein kann und/oder ein bis drei Reste aus der Gruppe C₁-C₄-Alkyl und C₁-C₄-Alkoxy tragen kann;
vier- bis sechsgliedriges Heterocyclyl-C₁-C₄-alkyl, welches partiell oder vollständig halogeniert sein kann und/oder ein bis drei Reste aus der Gruppe C₁-C₄-Alkyl und C₁-C₄-Alkoxy tragen kann;
fünf- bis sechsgliedriges Heteroaryl mit ein bis vier Stickstoffatomen, oder mit ein bis drei Stickstoffatomen und einem Sauerstoff- oder einem Schwefelatom, oder mit einem Sauerstoff oder Schwefelatom, welches partiell oder vollständig halogeniert und/oder ein bis drei Reste aus der Gruppe C₁-C₄-Alkyl, C₁-C₄-Halogenalkyl, C₁-C₄-Alkoxy, C₁-C₄-Halogenalkoxy, Amino, C₁-C₄-Alkylamino und Di(C₁-C₄-alkyl)amino tragen kann;
fünf- bis sechsgliedriges Heteroaryl-C₁-C₄-alkyl mit ein bis vier Stickstoffatomen, oder mit ein bis drei Stickstoffatomen und einem Sauerstoff- oder einem Schwefelatom, oder mit einem Sauerstoff oder Schwefelatom, welches partiell oder vollständig halogeniert sein kann und/oder ein bis drei Reste aus der Gruppe C₁-C₄-Alkyl und C₁-C₄-Alkoxy tragen kann;
besonders bevorzugt
Wasserstoff, Hydroxy, C₁-C₆-Alkyl, C₁-C₆-Halogenalkyl, C₃-C₇-Cycloalkyl, C₂-C₆-Alkenyl, C₂-C₆-Halogenalkenyl, C₃-C₆-Alkinyl, C₁-C₆-Alkoxy, C₁-C₄-Alkoxy-C₁-C₄-alkyl, C₁-C₄-Alkoxycarbonyl-C₁-C₄-alkyl, C₁-C₄-Alkoxycarbonyl-C₂-C₄-alkenyl, C₂-C₄-Alkenyloxycarbonyl-C₁-C₄-alkyl, C₃-C₆-Alkinyloxycarbonyl-C₁-C₄-alkyl, C₁-C₄-Alkylthio-C₁-C₄-alkyl, Phenyl, Benzyl;
fünf- bis sechsgliedriges Heterocyclyl, welches partiell oder vollständig halogeniert sein kann und/oder ein bis drei Reste aus der Gruppe C₁-C₄-Alkyl und C₁-C₄-Alkoxy tragen kann;
fünf- bis sechsgliedriges Heteroaryl mit ein bis vier Stickstoffatomen, oder mit ein bis drei Stickstoffatomen und einem Sauerstoff- oder einem Schwefelatom, oder mit einem Sauerstoff oder Schwefelatom, welches partiell oder vollständig halogeniert und/oder ein bis drei Reste aus der Gruppe C₁-C₄-Alkyl, C₁-C4-Halogenalkyl, C₁-C₄-Alkoxy, C₁-C₄-Halogenalkoxy, Amino, C₁-C₄-Alkylamino und Di(C₁-C₄-alkyl)amino tragen kann;
insbesondere bevorzugt
C₁-C₄-Alkyl, C₁-C₄-Halogenalkyl, C₅-C₈-Cycloalkyl, C₂-C₆-Alkenyl, C₃-C₆-Alkinyl, C₁-C₄-Alkoxy-C₁-C₄-alkyl, C₁-C₄-Alkylthio-C₁-C₄-alkyl, Phenyl, Benzyl;
fünf- bis sechsgliedriges Heterocyclyl, welches partiell oder vollständig halogeniert sein kann und/oder ein bis drei Reste aus der Gruppe C₁-C₄-Alkyl und C₁-C₄-Alkoxy tragen kann;
fünf- bis sechsgliedriges Heteroaryl mit ein bis vier Stickstoffatomen, oder mit ein bis drei Stickstoffatomen und einem Sauerstoff- oder einem Schwefelatom, oder mit einem Sauerstoff oder Schwefelatom, wobei die zwei letztgenannten Reste partiell oder vollständig halogeniert sein können und/oder ein bis drei Reste aus der Gruppe C₁-C₄-Alkyl und C₁-C₄-Alkoxy tragen können;
außerordentlich bevorzugt
C₁-C₄-Alkyl wie CH₃, C₂H₅, CH(CH₃)₂, -CH₂-CH₂-CH₃, C₁-C₄-Halogenalkyl wie CF₃, C₅-C₆-Cycloalkyl, C₂-C₄-Alkenyl, C₃-C₄-Alkinyl, C₁-C₄-Alkoxy-C₁-C₄-alkyl, C₁-C₄-Alkylthio-C₁-C₄-alkyl, Phenyl, Benzyl, fünf- bis sechsgliedriges Heterocyclyl oder fünf- bis sechsgliedriges Heteroaryl mit ein bis vier Stickstoffatomen, wobei die zwei letztgenannten Reste partiell oder vollständig halogeniert sein können und/oder ein bis drei Reste aus der Gruppe C₁-C₄-Alkyl und C₁-C₄-Alkoxy tragen können;
bedeutet.

Ebenso bevorzugt sind die Benzolsulfonamid-Derivate der Formel I, in der
- R²: Wasserstoff, C₁-C₈-Alkyl oder C₂-C₈-Alkenyl, besonders bevorzugt Wasserstoff, C₁-C₈-Alkyl oder C₂-C₆-Alkenyl, insbesondere bevorzugt Wasserstoff oder C₁-C₄-Alkyl, außerordentlich bevorzugt Wasserstoff, CH₃, C₂H₅ oder CH(CH₃)₂, sehr außerordentlich bevorzugt Wasserstoff oder CH₃;
bedeutet.

Ebenso bevorzugt sind die Benzolsulfonamid-Derivate der Formel I, in der
- R¹ und R²: zusammen mit dem N-Atom, an das sie gebunden sind, einen drei- bis siebengliedrigen Heterocyclus, welcher seinerseits partiell oder vollständig halogeniert sein kann und/oder ein bis drei Reste aus der Gruppe C₁-C₆-Alkyl, C₁-C₆-Halogenalkyl und C₁-C₆-Alkoxy tragen kann;
besonders bevorzugt einen drei- bis siebengliedrigen Heterocyclus, welcher seinerseits partiell oder vollständig halogeniert sein kann und/oder ein bis drei Reste aus der Gruppe C₁-C₄-Alkyl und C₁-C₄-Alkoxy tragen kann;
insbesondere bevorzugt einen fünf- bis siebengliedrigen Heterocyclus, welcher seinerseits partiell oder vollständig halogeniert sein kann und/oder ein bis drei Reste aus der Gruppe C₁-C₄-Alkyl und C₁-C₄- Alkoxy tragen kann;
außerordentlich bevorzugt einen fünf- oder sechsgliedrigen Heterocyclus, welcher seinerseits partiell oder vollständig halogeniert sein kann und/oder ein bis drei Reste aus der Gruppe C₁-C₄-Alkyl und C₁-C₄-Alkoxy tragen kann;
sehr außerordentlich einen Heterocyclus aus der Gruppe Pyrrolidin-1-yl, 2,3-Dihydropyrrol-1-yl, 2,5-Dihydropyrrol-1-yl, Piperidin-1-yl, 1,2,3,4-Tetrahydropyridin-1-yl, 1,2,3,6-Tetrahydropyridin-1-yl, Piperazin-1-yl, Morpholin-4-yl, welcher seinerseits partiell oder vollständig halogeniert sein kann und/oder ein bis drei Reste aus der Gruppe C₁-C₄-Alkyl und C₁-C₄- Alkoxy tragen kann;
bilden.

Ebenso bevorzugt sind die Benzolsulfonamid-Derivate der Formel I, in der
- Y: C(A)B, wobei A und B für Sauerstoff stehen; und
- R¹: die voranstehend genannten Bevorzugungen hat,
bedeutet.

Ebenso bevorzugt sind die Benzolsulfonamid-Derivate der Formel I, in der
- Q: Q¹, Q², Q⁵, Q⁷, Q⁸, Q¹⁰, Q¹², Q¹³, Q¹⁷, Q²⁰, Q²¹, Q²², Q²³, Q²⁴, Q²⁷, Q³¹, Q³², Q³⁴, Q³⁸ oder Q³⁹;
besonders bevorzugt Q¹, Q², Q⁵, Q⁷, Q⁸, Q¹⁰, Q¹², Q¹³, Q¹⁷, Q²⁰, Q²¹, Q²², Q²⁴, Q²⁷, Q³¹, Q³², Q³⁸ oder Q³⁹;
insbesondere bevorzugt Q⁵, Q⁷, Q²¹, Q²², Q²⁷, Q³², Q³⁸ oder Q³⁹;
außerordentlich bevorzugt Q²¹, Q³² oder Q³⁸;
bedeutet.

Ebenso bevorzugt sind die Benzolsulfonamid-Derivate der Formel I, in der
- Q: Q¹, Q², Q³, Q⁴, Q⁶, Q⁷, Q⁸, Q⁹, Q¹⁰, Q¹¹, Q¹², Q¹³, Q¹⁴, Q¹⁵, Q¹⁶, Q¹⁷, Q¹⁸, Q¹⁹, Q²⁰, Q²¹, Q²², Q²³, Q²⁴, Q²⁵, Q²⁶, Q²⁷, Q²⁸, Q²⁹, Q³⁰, Q³¹, Q³², Q³³, Q³⁴, Q³⁵, Q³⁶, Q³⁷, Q³⁸ oder Q³⁹,
besonders bevorzugt Q¹, Q², Q⁷, Q⁸, Q¹⁰, Q¹², Q¹³, Q¹⁷, Q²⁰, Q²¹, Q²², Q²³, Q²⁴, Q²⁷, Q³¹, Q³², Q³⁴, Q³⁸ oder Q³⁹,
insbesondere bevorzugt Q¹, Q², Q⁷, Q⁸, Q¹⁰, Q¹², Q¹³, Q¹⁷, Q²⁰, Q²¹, Q²², Q²⁴, Q²⁷, Q³¹, Q³², Q³⁸ oder Q³⁹,
außerordentlich bevorzugt Q⁷, Q²¹, Q²², Q²⁷, Q³², Q³⁸ oder Q³⁹,
sehr außerordentlich bevorzugt Q²¹, Q³² oder Q³⁸
bedeutet.

Ebenso bevorzugt sind die Benzolsulfonamid-Derivate der Formel I, in der
- Q: Q⁷, Q²¹, Q²², Q²⁷ Q³², Q³⁸ oder Q³⁹;
besonders bevorzugt
Q⁷, wobei Y für C(A)B mit B = Sauerstoff steht;
Q²¹, wobei Y für C(A)B mit B = Sauerstoff oder Schwefel steht,
   bevorzugt Y für C(A)B mit B = Sauerstoff steht;
Q²²; Q²⁷; Q³²; Q³⁸ oder Q³⁹;
insbesondere bevorzugt
Q²¹, wobei Y für C(A)B mit B = Sauerstoff oder Schwefel steht, bevorzugt Y für C(A)B mit B = Sauerstoff;
Q³² oder Q³⁸;
bedeutet.

Ebenso bevorzugt sind die Benzolsulfonamid-Derivate der Formel I, in der
- X¹: Wasserstoff, Fluor oder Chlor;
besonders bevorzugt Wasserstoff oder Fluor;
insbesondere bevorzugt Fluor; und
- Q: Q¹, Q², Q⁵, Q⁷, Q⁹, Q¹⁰, Q¹², Q¹³, Q¹⁷, Q²⁰, Q²¹, Q²², Q²³, Q²⁴, Q²⁷, Q³¹, Q³², Q³⁴, Q³⁸ oder Q³⁹,
besonders bevorzugt Q¹, Q², Q⁵, Q⁷, Q⁸, Q¹⁰, Q¹², Q¹³, Q¹⁷, Q²⁰, Q²¹, Q²², Q²⁴, Q²⁷, Q³¹, Q³², Q³⁸ oder Q³⁹,
insbesondere bevorzugt Q⁵, Q⁷, Q²¹, Q²², Q²⁷, Q³², Q³⁸ oder Q³⁹,
außerordentlich bevorzugt Q²¹, Q³² oder Q³⁸
bedeuten.

Ebenso bevorzugt sind die Benzolsulfonamid-Derivate der Formel I, in der
- Q: Q¹ bis Q³⁹; und
- R³, R⁴, R⁷, R⁸,:
- R¹¹, R¹², R¹⁸, R¹⁹,:
- R²⁷, R²⁹, R³², R³³,:
- R³⁸, R³⁹, R⁴⁴, R⁴⁵,:
- R⁴⁶ und R⁴⁷: Wasserstoff, C₁-C₆-Alkyl, C₁-C₆-Halogenalkyl, C₁-C₆-Alkoxy, C₁-C₆-Halogenalkoxy, C₁-C₆-Alkylsulfonyl oder Amino;
bevorzugt Wasserstoff, C₁-C₄-Alkyl, C₁-C₄-Halogenalkyl, C₁-C₄-Alkoxy, C₁-C₄-Halogenalkoxy, C₁-C₄-Alkylsulfonyl oder Amino;
insbesondere bevorzugt Wasserstoff, CH₃, C₂H₅, CF₃, CHF₂, CH₂CF₃, OCH₃, OCHF₂, OCF₂CHF₂, SO₂CH₃ oder Amino;
- R⁵, R⁶, R⁹, R¹⁰,:
- R¹⁵, R¹⁶, R²⁰, R²¹,:
- R³⁰, R³¹, R³⁵, R³⁶,:
- R⁴¹, R⁴² und R⁴³: Wasserstoff, C₁-C₆-Alkyl, C₁-C₆-Halogenalkyl, C₁-C₆-Alkoxy, C₁-C₆-Halogenalkoxy, C₁-C₆-Alkylsulfonyl oder Amino;
bevorzugt Wasserstoff, C₁-C₄-Alkyl, C₁-C₄-Halogenalkyl, C₁-C₄-Alkoxy, C₁-C₄-Halogenalkoxy, C₁₋C₄-Alkylsulfonyl oder Amino;
insbesondere bevorzugt Wasserstoff, CH₃, C₂H₅, CF₃, CHF₂, OCH₃, OCHF₂, SO₂CH₃ oder Amino;
- R¹³, R¹⁴, R²², R²³,:
- R²⁵ und R²⁶: Wasserstoff, Halogen oder C₁-C₄-Alkyl;
besonders bevorzugt Wasserstoff, Halogen oder CH₃;
insbesondere bevorzugt Wasserstoff, Chlor oder Brom;
R¹⁷, R²⁸, R³⁴, R³⁷ oder R⁴⁰
Wasserstoff, Halogen oder C₁-C₄-Alkyl;
besonders bevorzugt Wasserstoff, Halogen oder CH₃;
insbesondere bevorzugt Wasserstoff, Chlor oder Brom;
bedeuten.

Ebenso bevorzugt sind die Benzolsulfonamid-Derivate der Formel I, in der
- Q: Q⁵, Q⁷, Q²¹, Q²², Q²⁷, Q³², Q³⁸ oder Q³⁹,
besonders bevorzugt Q²¹, Q³² oder Q³⁸;
- A¹, A⁸, A⁹, A¹⁰,:
- A¹¹, A¹², A¹³, A¹⁵,:
- A¹⁶ und A¹⁷: Sauerstoff;
- R⁷, R⁸, R²⁹, R³²,:
- R³³, R³⁸, R³⁹, R⁴⁴,:
- R⁴⁵, R⁴⁶, R⁴⁷: Wasserstoff, C₁-C₆-Alkyl, C₁-C₆-Halogenalkyl, C₁-C₆-Alkoxy, C₁-C₆-Halogenalkoxy, C₁-C₆-Alkylsulfonyl oder Amino;
bevorzugt Wasserstoff, C₁-C₄-Alkyl, C₁-C₄-Halogenalkyl, C₁-C₄-Alkoxy, C₁-C₄-Halogenalkoxy, C₁-C₄-Alkylsulfonyl oder Amino;
insbesondere bevorzugt Wasserstoff, CH₃, C₂H₅, CF₃, CHF₂, CH₂CF₃, OCH₃, OCHF₂, OCF₂CHF₂, SO₂CH₃ oder Amino;
- R³⁰, R³¹, R³⁵, R³⁶,:
- R⁴¹, R⁴², R⁴³: Wasserstoff, C₁-C₆-Alkyl, C₁-C₆-Halogenalkyl, C₁-C₆-Alkoxy, C₁-C₆-Halogenalkoxy, C₁-C₆-Alkylsulfonyl oder Amino;
bevorzugt Wasserstoff, C₁-C₄-Alkyl, C₁-C₄-Halogenalkyl, C₁-C₄-Alkoxy, C₁-C₄-Halogenalkoxy, C₁-C₄-Alkylsulfonyl oder Amino;
insbesondere bevorzugt Wasserstoff, CH₃, C₂H₅, CF₃, CHF₂, OCH₃, OCHF₂, SO₂CH₃ oder Amino; und
- R³⁴, R³⁷ R⁴⁰: Wasserstoff, Halogen oder C₁-C₄-Alkyl;
besonders bevorzugt Wasserstoff, Halogen oder CH₃;
insbesondere bevorzugt Wasserstoff, Chlor oder Brom;
bedeuten.

Ebenso bevorzugt sind die Benzolsulfonamid-Derivate der Formel I, in der
- Q: Q¹, Q⁷, Q⁸, Q¹⁰, Q¹², Q¹³, Q²¹, Q²³, Q²⁴, Q³¹ oder Q³⁴;
- A¹, A², A³ A⁴:
- A⁵, A⁶, A⁷, A⁸,:
- A⁹, A¹⁴, A¹⁶ und A¹⁷: Sauerstoff bedeuten; und
- R³ und R⁴, R⁵ und R⁶,:
- R⁹ und R¹⁰, R¹⁵ und R¹⁶,:
- R¹⁸ und R¹⁹, R²⁰ und R²¹ ,:
- R³⁰ und R³¹ oder R⁴⁶ und R⁴⁷: zusammen mit den Atomen, an die sie gebunden sind, einen drei- bis siebengliedrigen Heterocyclus bilden, welcher seinerseits partiell oder vollständig halogeniert sein kann und/oder ein bis drei Reste aus der Gruppe C₁-C₆-Alkyl und C₁-C₆-Alkoxy tragen kann;
besonders bevorzugt
- Q: Q¹, Q⁷, Q⁸, Q¹⁰, Q¹², Q¹³, Q²¹, Q²⁴ oder Q³¹;
- A¹, A², A³, A⁴,:
- A⁶, A⁸, A⁹, A¹⁶:
- und A¹⁷: Sauerstoff bedeuten; und
- R³ und R⁴, R⁵ und R⁶:
- R⁹ und R¹⁰, R¹⁵ und R¹⁶,:
- R¹⁸ und R¹⁹, R²⁰ und:
- R²¹, R³⁰ und R³¹:
- oder R⁴⁶ und R⁴⁷: zusammen mit den Atomen, an die sie gebunden sind, einen drei- bis siebengliedrigen Heterocyclus bilden, welcher seinerseits partiell oder vollständig halogeniert sein kann und/oder ein bis drei Reste aus der Gruppe C₁-C₆-Alkyl und C₁-C₆-Alkoxy tragen kann;
insbesondere bevorzugt
Q Q⁷ oder Q²¹,
A⁸, A⁹, A¹⁶ und A¹⁷ Sauerstoff;
R²⁹ Wasserstoff, C₁-C₆-Alkyl oder Amino bedeuten; und
R³⁰ und R³¹ oder R⁴⁶ und R⁴⁷ zusammen mit den Atomen, an die sie gebunden sind, einen drei- bis siebengliedrigen Heterocyclus bilden, welcher seinerseits partiell oder vollständig halogeniert sein kann und/oder ein bis drei Reste aus der Gruppe C₁-C₆-Alkyl und C₁-C₆-Alkoxy tragen kann,
bedeuten.

Außerordentlich bevorzugt sind die Verbindungen der Formel 1.2, insbesondere die Verbindungen der Formel I.2.1 bis I.2.689, die sich von den entsprechenden Verbindungen der Formel I.1.1 bis I.1.689 dadurch unterscheiden, daß B für Schwefel steht.

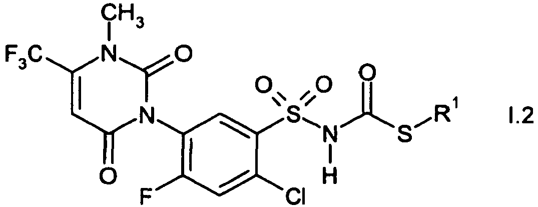

Ebenso außerordentlich bevorzugt sind die Verbindungen der Formel I.3, insbesondere die Verbindungen der Formel I.3.1 bis 1.3.689, die sich von den entsprechenden Verbindungen der Formel I.1.1 bis I.1.689 dadurch unterscheiden, daß R²⁹ für Amino steht.

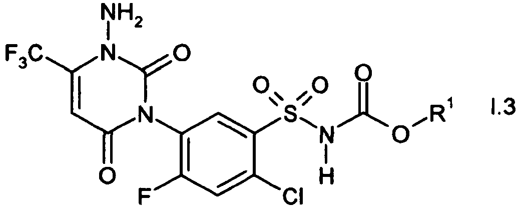

Ebenso außerordentlich bevorzugt sind die Verbindungen der Formel **I.4,** insbesondere die Verbindungen der Formel I.4.1 bis I.4.689, die sich von den entsprechenden Verbindungen der Formel I.1.1 bis I.1.689 dadurch unterscheiden, daß B für Schwefel und R²⁹ für Amino stehen.

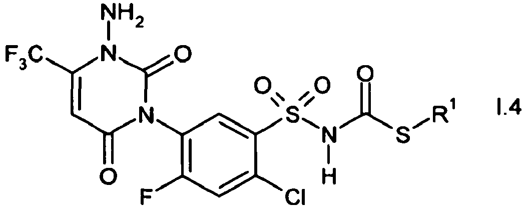

Ebenso außerordentlich bevorzugt sind die Verbindungen der Formel I.5, insbesondere die Verbindungen der Formel I.5.1 bis I.5.689, die sich von den entsprechenden Verbindungen der Formel I.1.1 bis 1.1.689 dadurch unterscheiden, daß X¹ für Wasserstoff steht.

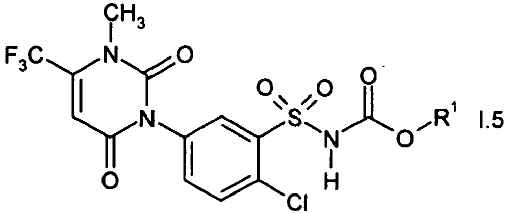

Ebenso außerordentlich bevorzugt sind die Verbindungen der Formel 1.6, insbesondere die Verbindungen der Formel I.6.1 bis I.6.689, die sich von den entsprechenden Verbindungen der Formel I.1.1 bis I.1.689 dadurch unterscheiden, daß X¹ für Wasserstoff und B für Schwefel stehen.

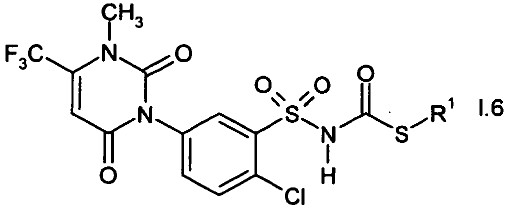

Ebenso außerordentlich bevorzugt sind die Verbindungen der Formel I.7, insbesondere die Verbindungen der Formel I.7.1 bis I.7.689, die sich von den entsprechenden Verbindungen der Formel I.1.1 bis I.1.689 dadurch unterscheiden, daß X¹ für Wasserstoff und R²⁹ für Amino stehen.

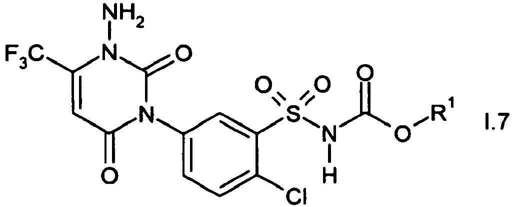

Ebenso außerordentlich bevorzugt sind die Verbindungen der Formel I.8, insbesondere die Verbindungen der Formel I.8.1 bis 1.8.689, die sich von den entsprechenden Verbindungen der Formel I.1.1 bis I.1.689 dadurch unterscheiden, daß X¹ für Wasserstoff, B für Schwefel und R²⁹ für Amino stehen.

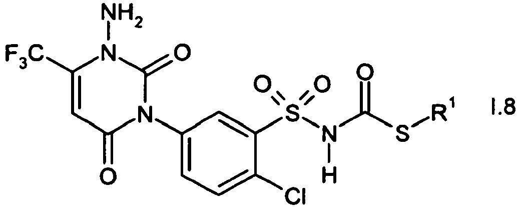

Ebenso außerordentlich bevorzugt sind die Verbindungen der Formel 1.9, insbesondere die Verbindungen der Formel I.9.1 bis I.9.689, die sich von den entsprechenden Verbindungen der Formel 1.1.1 bis I.1.689 dadurch unterscheiden, daß Q für Q⁵ (mit A¹ = Sauerstoff, R⁷ = Difluormethyl und R⁸ = Methyl) steht.

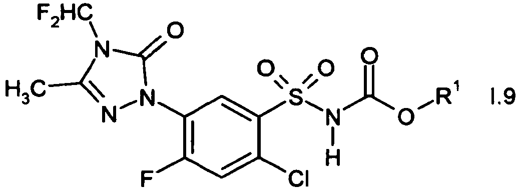

Ebenso außerordentlich bevorzugt sind die Verbindungen der Formel I.10, insbesondere die Verbindungen der Formel I.10.1 bis 1.10.689, die sich von den entsprechenden Verbindungen der Formel I.1.1 bis I.1.689 dadurch unterscheiden, daß B für Schwefel und Q für Q⁵ (mit A¹ = Sauerstoff, R⁷ = Difluormethyl und R⁸ = Methyl) stehen.

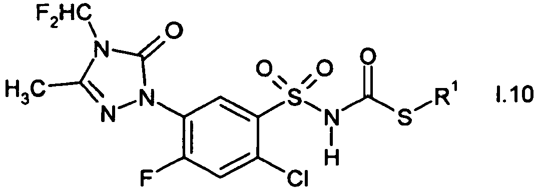

Ebenso außerordentlich bevorzugt sind die Verbindungen der Formel I.11, insbesondere die Verbindungen der Formel I.11.1 bis I.11.689, die sich von den entsprechenden Verbindungen der Formel I.1.1 bis I.1.689 dadurch unterscheiden, daß X¹ für Chlor und Q für Q⁵ (mit A¹ = Sauerstoff, R⁷= Difluormethyl und R⁸ = Methyl) stehen.

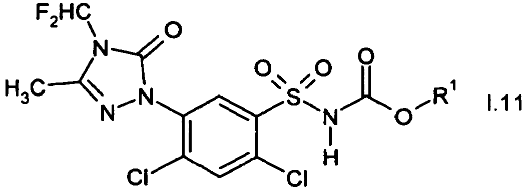

Ebenso außerordentlich bevorzugt sind die Verbindungen der Formel 1.12, insbesondere die Verbindungen der Formel I.12.1 bis 1.12.689, die sich von den entsprechenden Verbindungen der Formel I.1.1 bis I.1.689 dadurch unterscheiden, daß X¹ für Chlor, B für Schwefel und Q für Q⁵ (mit A¹ = Sauerstoff, R⁷ = Difluormethyl und R⁸ = Methyl) stehen.

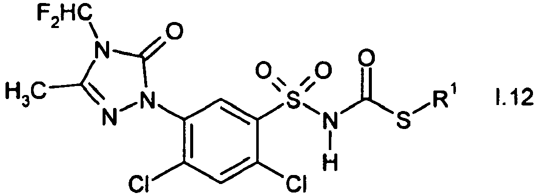

Ebenso außerordentlich bevorzugt sind die Verbindungen der Formel I.13, insbesondere die Verbindungen der Formel I.13.1 bis 1.13.689, die sich von den entsprechenden Verbindungen der Formel I.1.1 bis I.1.689 dadurch unterscheiden, daß Q für Q²² (mit A¹⁰ und A¹¹ = Sauerstoff, A¹² = Schwefel und R³², R³³ = Methyl) steht.

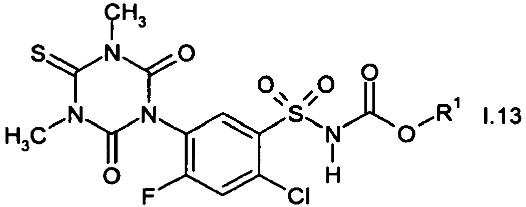

Ebenso außerordentlich bevorzugt sind die Verbindungen der Formel I.14, insbesondere die Verbindungen der Formel I.14.1 bis 1.14.689, die sich von den entsprechenden Verbindungen der Formel I.1.1 bis I.1.689 dadurch unterscheiden, daß B für Schwefel und Q für Q²² (mit A¹⁰ und A¹¹ = Sauerstoff, A¹² = Schwefel und R³², R³³ = Methyl) stehen.

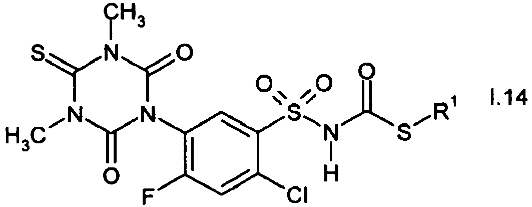

Ebenso außerordentlich bevorzugt sind die Verbindungen der Formel I.15, insbesondere die Verbindungen der Formel 1.15.1 bis 1.15.689, die sich von den entsprechenden Verbindungen der Formel I.1.1 bis I.1.689 dadurch unterscheiden, daß Q für Q²² (mit A¹⁰, A¹¹, A¹² = Sauerstoff und R³², R³³ = Methyl) steht.

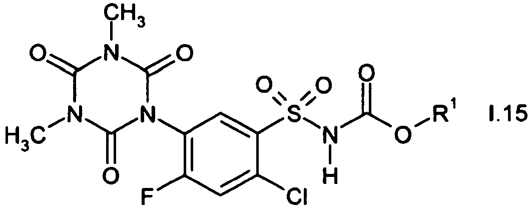

Ebenso außerordentlich bevorzugt sind die Verbindungen der Formel I.16, insbesondere die Verbindungen der Formel I.16.1 bis 1.16.689, die sich von den entsprechenden Verbindungen der Formel I.1.1 bis I.1.689 dadurch unterscheiden, daß B für Schwefel und Q für Q²² (mit A¹⁰, A¹¹, A¹² = Sauerstoff und R³², R³³ = Methyl) stehen.

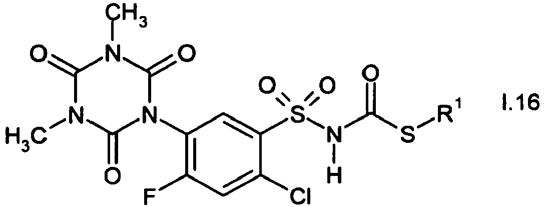

Ebenso außerordentlich bevorzugt sind die Verbindungen der Formel I.17, insbesondere die Verbindungen der Formel 1.17.1 bis 1.17.689, die sich von den entsprechenden Verbindungen der Formel I.1.1 bis I.1.689 dadurch unterscheiden, daß Q für Q²⁷ (mit A¹³ = Sauerstoff, R³⁴, R³⁶ = Wasserstoff, R³⁵ = Trifluormethyl) steht.

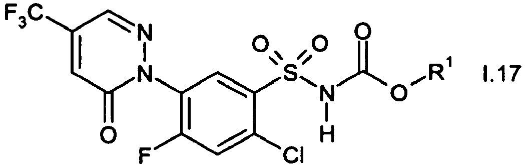

Ebenso außerordentlich bevorzugt sind die Verbindungen der Formel 1.18, insbesondere die Verbindungen der Formel I.18.1 bis I.18.689, die sich von den entsprechenden Verbindungen der Formel I.1.1 bis 1.1.689 dadurch unterscheiden, daß B für Schwefel und Q für Q²⁷ (mit A¹³ = Sauerstoff, R³⁴, R³⁶ = Wasserstoff, R³⁵ = Trifluormethyl) stehen.

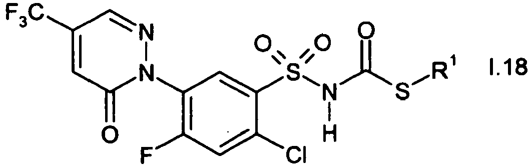

Ebenso außerordentlich bevorzugt sind die Verbindungen der Formel 1.19, insbesondere die Verbindungen der Formel I.19.1 bis 1.19.689, die sich von den entsprechenden Verbindungen der Formel I.1.1 bis I.1.689 dadurch unterscheiden, daß Q für Q²⁷ (mit A¹³ = Sauerstoff, R³⁴ = Wasserstoff, R³⁵ = Trifluormethyl, R³⁶ = Methyl) steht.

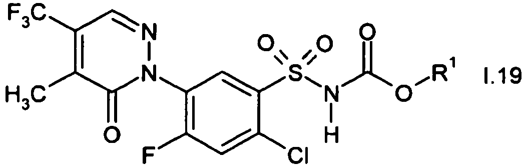

Ebenso außerordentlich bevorzugt sind die Verbindungen der Formel I.20, insbesondere die Verbindungen der Formel I.20.1 bis 1.20.689, die sich von den entsprechenden Verbindungen der Formel I.1.1 bis I.1.689 dadurch unterscheiden, daß B für Schwefel und Q für Q²⁷ (mit A¹³ = Sauerstoff, R³⁴ = Wasserstoff, R³⁵ = Trifluormethyl, R³⁸ = Methyl) stehen.

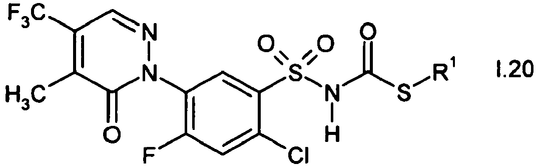

Ebenso außerordentlich bevorzugt sind die Verbindungen der Formel 1.21, insbesondere die Verbindungen der Formel I.21.1 bis 1.21.689, die sich von den entsprechenden Verbindungen der Formel I.1.1 bis I.1.689 dadurch unterscheiden, daß Q für Q²⁷ (mit A¹³ = Sauerstoff, R³⁴ = Wasserstoff, R³⁵ = Methylsulfonyl, R³⁶ = Amino) steht.

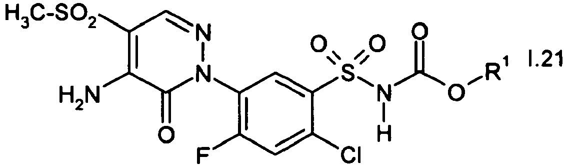

Ebenso außerordentlich bevorzugt sind die Verbindungen der Formel 1.22, insbesondere die Verbindungen der Formel I.22.1 bis 1.22.689, die sich von den entsprechenden Verbindungen der Formel I.1.1 bis I.1.689 dadurch unterscheiden, daß B für Schwefel und Q für Q²⁷ (mit A¹³ = Sauerstoff, R³⁴ = Wasserstoff, R³⁵ = Methylsulfonyl, R³⁶ = Amino) stehen.

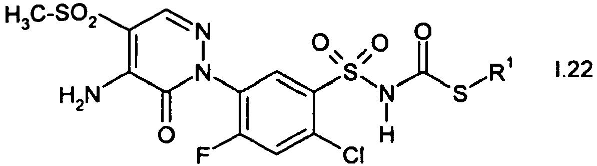

Ebenso außerordentlich bevorzugt sind die Verbindungen der Formel I.23, insbesondere die Verbindungen der Formel I.23.1 bis I.23.689, die sich von den entsprechenden Verbindungen der Formel I.1.1 bis I.1.689 dadurch unterscheiden, daß Q für Q³² (mit R³⁷ = Chlor, R³⁸ = Difluormethoxy, R³⁹ = Methyl) steht.

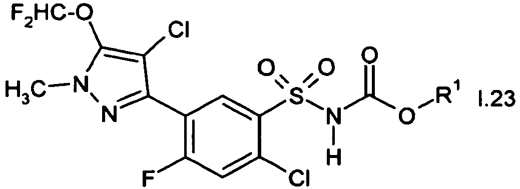

Ebenso außerordentlich bevorzugt sind die Verbindungen der Formel I.24, insbesondere die Verbindungen der Formel I.24.1 bis I.24.689, die sich von den entsprechenden Verbindungen der Formel I.1.1 bis I.1.689 dadurch unterscheiden, daß B für Schwefel und Q für Q³² (mit R³⁷ = Chlor, R³⁸ = Difluormethoxy, R³⁹ = Methyl) stehen.

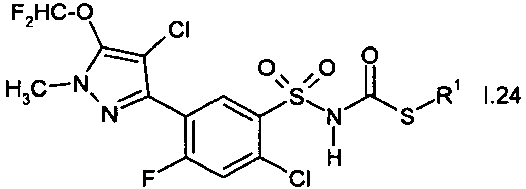

Ebenso außerordentlich bevorzugt sind die Verbindungen der Formel I.25, insbesondere die Verbindungen der Formel I.25.1 bis I.25.689, die sich von den entsprechenden Verbindungen der Formel I.1.1 bis I.1.689 dadurch unterscheiden, daß Q für Q³² (mit R³⁷ = Brom, R³⁸ = Difluormethoxy, R³⁹ = Methyl) steht.

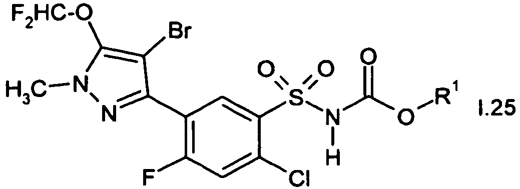

Ebenso außerordentlich bevorzugt sind die Verbindungen der Formel I.26, insbesondere die Verbindungen der Formel I.26.1 bis 1.26.689, die sich von den entsprechenden Verbindungen der Formel I.1.1 bis I.1.689 dadurch unterscheiden, daß B für Schwefel und Q für Q³² (mit R³⁷ = Brom, R³⁸ = Difluormethoxy, R³⁹ = Methyl) stehen.

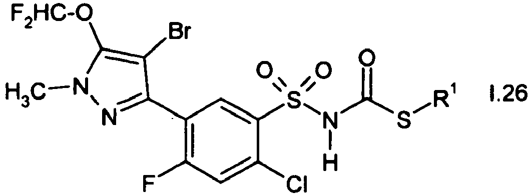

Ebenso außerordentlich bevorzugt sind die Verbindungen der Formel I.27, insbesondere die Verbindungen der Formel I.27.1 bis 1.27.689, die sich von den entsprechenden Verbindungen der Formel I.1.1 bis I.1.689 dadurch unterscheiden, daß X¹ für Chlor und Q für Q³² (mit R³⁷ = Brom, R³⁸ = Difluormethoxy, R³⁹ = Methyl) stehen.

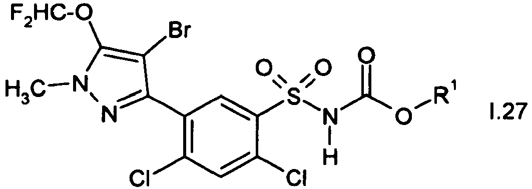

Ebenso außerordentlich bevorzugt sind die Verbindungen der Formel I.28, insbesondere die Verbindungen der Formel I.28.1 bis I.28.689, die sich von den entsprechenden Verbindungen der Formel I.1.1 bis I.1.689 dadurch unterscheiden, daß X¹ für Chlor, B für Schwefel und Q für Q³² (mit R³⁷ = Brom, R³⁸ = Difluormethoxy, R³⁹ = Methyl) stehen.

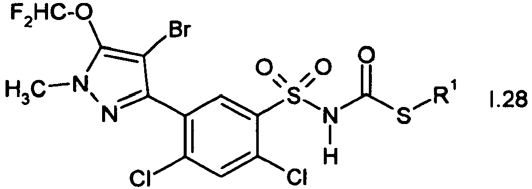

Ebenso außerordentlich bevorzugt sind die Verbindungen der Formel I.29, insbesondere die Verbindungen der Formel I.29.1 bis I.29.689, die sich von den entsprechenden Verbindungen der Formel I.1.1 bis I.1.689 dadurch unterscheiden, daß Q für Q³² (mit R³⁷ = Chlor, R³⁸ = Trifluormethyl, R³⁹ = Methyl) steht.

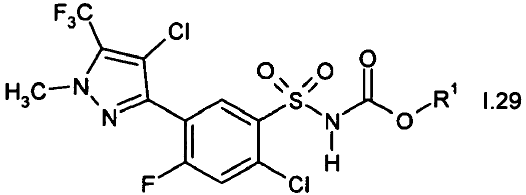

Ebenso außerordentlich bevorzugt sind die Verbindungen der Formel I.30, insbesondere die Verbindungen der Formel I.30.1 bis I.30.689, die sich von den entsprechenden Verbindungen der Formel I.1.1 bis I.1.689 dadurch unterscheiden, daß B für Schwefel und Q für Q³² (mit R³⁷ = Chlor, R³⁸ = Trifluormethyl, R³⁹ = Methyl) stehen.

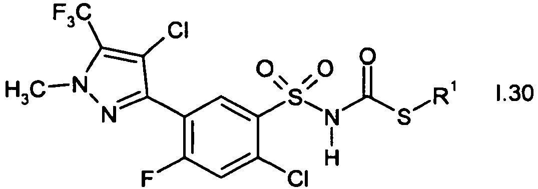

Ebenso außerordentlich bevorzugt sind die Verbindungen der Formel I.31, insbesondere die Verbindungen der Formel I.31.1 bis I.31.689, die sich von den entsprechenden Verbindungen der Formel I.1.1 bis I.1.689 dadurch unterscheiden, daß Q für Q³² (mit R³⁷ = Brom, R³⁸ = Trifluormethyl, R³⁹ = Methyl) steht.

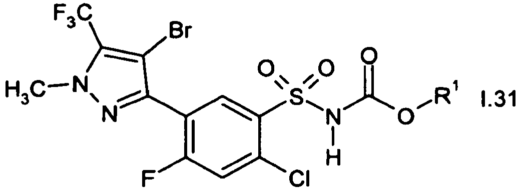

Ebenso außerordentlich bevorzugt sind die Verbindungen der Formel I.32, insbesondere die Verbindungen der Formel I.32.1 bis I.32.689, die sich von den entsprechenden Verbindungen der Formel I.1.1 bis I.1.689 dadurch unterscheiden, daß B für Schwefel und Q für Q³² (mit R³⁷ = Brom, R³⁸ = Trifluormethyl, R³⁹ = Methyl) stehen.

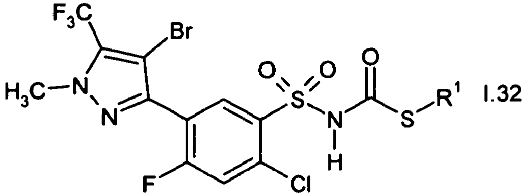

Ebenso außerordentlich bevorzugt sind die Verbindungen der Formel 1.33, insbesondere die Verbindungen der Formel I.33.1 bis I.33.689, die sich von den entsprechenden Verbindungen der Formel I.1.1 bis I.1.689 dadurch unterscheiden, daß X¹ für Chlor und Q für Q³² (mit R³⁷ = Brom, R³⁸ = Trifluormethyl, R³⁹ = Methyl) stehen.

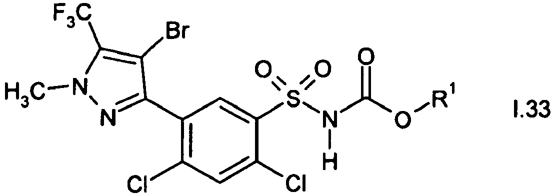

Ebenso außerordentlich bevorzugt sind die Verbindungen der Formel I.34, insbesondere die Verbindungen der Formel I.34.1 bis I.34.689, die sich von den entsprechenden Verbindungen der Formel I.1.1 bis I.1.689 dadurch unterscheiden, daß X¹ für Chlor, B für Schwefel und Q für Q³² (mit R³⁷ = Brom, R³⁸ = Trifluormethyl, R³⁹ = Methyl) stehen.

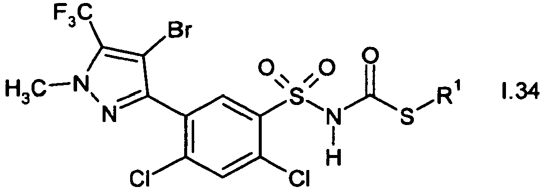

Ebenso außerordentlich bevorzugt sind die Verbindungen der Formel I.35, insbesondere die Verbindungen der Formel I.35.1 bis I.35.689, die sich von den entsprechenden Verbindungen der Formel I.1.1 bis I.1.689 dadurch unterscheiden, daß Q für Q³² (mit R³⁷ = Chlor, R³⁸ = Methylsulfonyl, R³⁹ = Methyl) steht.

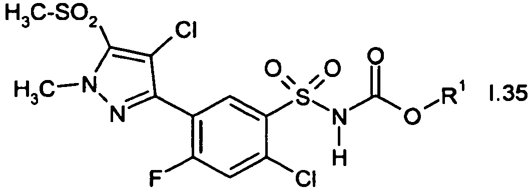

Ebenso außerordentlich bevorzugt sind die Verbindungen der Formel I.36, insbesondere die Verbindungen der Formel I.36.1 bis 1.36.689, die sich von den entsprechenden Verbindungen der Formel I.1.1 bis I.1.689 dadurch unterscheiden, daß B für Schwefel und Q für Q³² (mit R³⁷ = Chlor, R³⁸ = Methylsulfonyl, R³⁹ = Methyl) stehen.

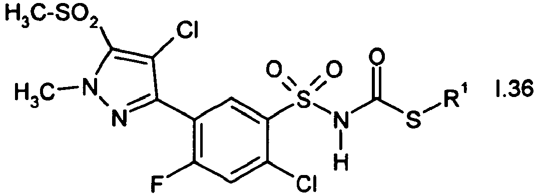

Ebenso außerordentlich bevorzugt sind die Verbindungen der Formel 1.37, insbesondere die Verbindungen der Formel I.37.1 bis 1.37.689, die sich von den entsprechenden Verbindungen der Formel I.1.1 bis I.1.689 dadurch unterscheiden, daß Q für Q³² (mit R³⁷ = Brom, R³⁸ = Methylsulfonyl, R³⁹ = Methyl) steht.

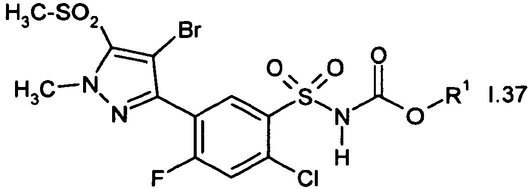

Ebenso außerordentlich bevorzugt sind die Verbindungen der Formel I.38, insbesondere die Verbindungen der Formel I.38.1 bis I.38.689, die sich von den entsprechenden Verbindungen der Formel I.1.1 bis I.1.689 dadurch unterscheiden, daß B für Schwefel und Q für Q³² (mit R³⁷ = Brom, R³⁸ = Methylsulfonyl, R³⁹ = Methyl) stehen.

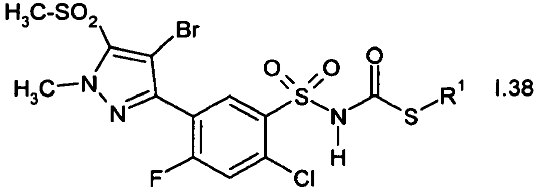

Ebenso außerordentlich bevorzugt sind die Verbindungen der Formel I.39, insbesondere die Verbindungen der Formel I.39.1 bis I.39.689, die sich von den entsprechenden Verbindungen der Formel I.1.1 bis I.1.689 dadurch unterscheiden, daß X¹ für Chlor und Q für Q³² (mit R³⁷ = Brom, R³⁸ = Methylsulfonyl, R³⁹ = Methyl) stehen.

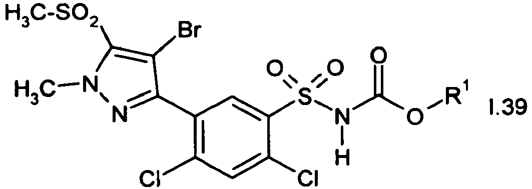

Ebenso außerordentlich bevorzugt sind die Verbindungen der Formel I.40, insbesondere die Verbindungen der Formel I.40.1 bis 1.40.689, die sich von den entsprechenden Verbindungen der Formel 1.1.1 bis I.1.689 dadurch unterscheiden, daß X¹ für Chlor, B für Schwefel und Q für Q³² (mit R³⁷ = Brom, R³⁸ = Methylsulfonyl, R³⁹ = Methyl) stehen.

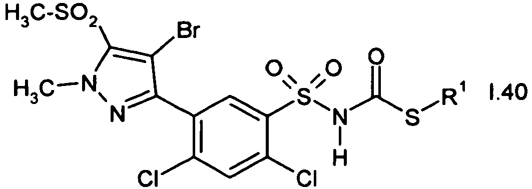

Ebenso außerordentlich bevorzugt sind die Verbindungen der Formel I.41, insbesondere die Verbindungen der Formel I.41.1 bis I.41.689, die sich von den entsprechenden Verbindungen der Formel I.1.1 bis I.1.689 dadurch unterscheiden, daß Q für Q³⁸ (mit R⁴⁰ = Chlor, R⁴¹, R⁴³ = Wasserstoff, R⁴² = Trifluormethyl) steht.

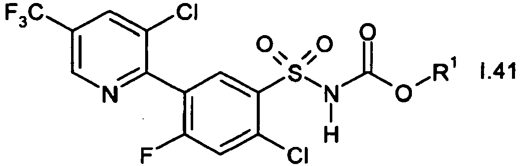

Ebenso außerordentlich bevorzugt sind die Verbindungen der Formel I.42, insbesondere die Verbindungen der Formel I.42.1 bis I.42.689, die sich von den entsprechenden Verbindungen der Formel I.1.1 bis I.1.689 dadurch unterscheiden, daß B für Schwefel und Q für Q³⁸ (mit R⁴⁰ = Chlor, R⁴¹, R⁴³ = Wasserstoff, R⁴² = Trifluormethyl) stehen.

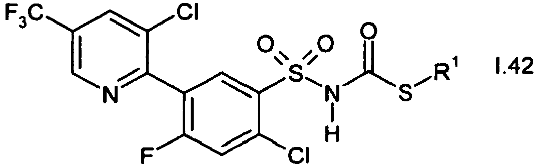

Ebenso außerordentlich bevorzugt sind die Verbindungen der Formel 1.43, insbesondere die Verbindungen der Formel I.43.1 bis 1.43.689, die sich von den entsprechenden Verbindungen der Formel 1.1.1 bis I.1.689 dadurch unterscheiden, daß Q für Q³⁹ (mit A¹ = Sauerstoff, A¹⁵ = Schwefel, R⁴⁴, R⁴⁵ = Methyl) steht.

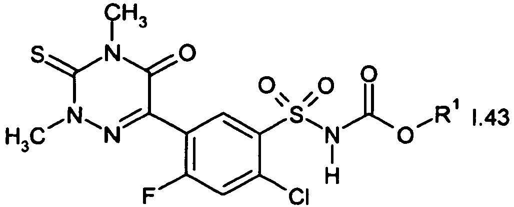

Ebenso außerordentlich bevorzugt sind die Verbindungen der Formel 1.44, insbesondere die Verbindungen der Formel I.44.1 bis 1.44.689, die sich von den entsprechenden Verbindungen der Formel I.1.1 bis 1.1.689 dadurch unterscheiden, daß B für Schwefel und Q für Q³⁹ (mit A¹ = Sauerstoff, A¹⁵ = Schwefel, R⁴⁴, R⁴⁵ = Methyl) stehen.

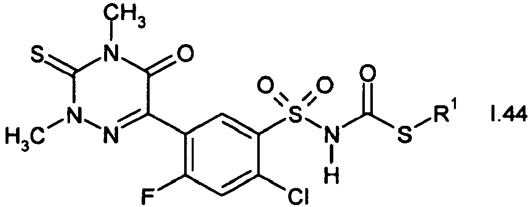

Ebenso außerordentlich bevorzugt sind die Verbindungen der Formel I.45, insbesondere die Verbindungen der Formel I.45.1 bis I.45.689, die sich von den entsprechenden Verbindungen der Formel I.1.1 bis I.1.689 dadurch unterscheiden, daß Q für Q⁷ (mit A¹⁶, A¹⁷ = Sauerstoff und R⁴⁸, R⁴⁷ bilden eine Kette -CH₂-CH₂-O-CH₂-) steht.

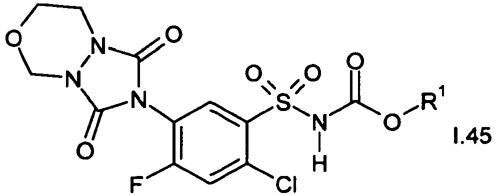

Ebenso außerordentlich bevorzugt sind die Verbindungen der Formel I.46, insbesondere die Verbindungen der Formel I.46.1 bis 1.46.689, die sich von den entsprechenden Verbindungen der Formel I.1.1 bis I.1.689 dadurch unterscheiden, daß B für Schwefel und Q für Q⁷ (mit A¹⁶, A¹⁷ = Sauerstoff und R⁴⁶, R⁴⁷ bilden eine Kette -CH₂-CH₂-O-CH₂-) stehen.

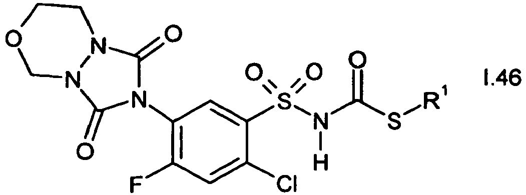

Ebenso außerordentlich bevorzugt sind die Verbindungen der Formel I.47, insbesondere die Verbindungen der Formel I.47.1 bis 1.47.689, die sich von den entsprechenden Verbindungen der Formel I.1.1 bis I.1.689 dadurch unterscheiden, daß Q für Q⁷ (mit A¹⁶ = Schwefel, A¹⁷ = Sauerstoff und R⁴⁶, R⁴⁷ bilden eine Kette -CH₂-CH₂-O-CH₂-) steht.

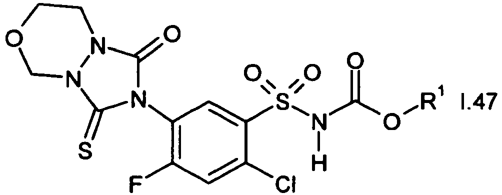

Ebenso außerordentlich bevorzugt sind die Verbindungen der Formel I48, insbesondere die Verbindungen der Formel I.48.1 bis I.48.689, die sich von den entsprechenden Verbindungen der Formel I.1.1 bis I.1.689 dadurch unterscheiden, daß B für Schwefel und Q für Q⁷ (mit A¹⁶ = Schwefel, A¹⁷ = Sauerstoff und R⁴⁶, R⁴⁷ bilden eine Kette -CH₂-CH₂-O-CH₂-) stehen.

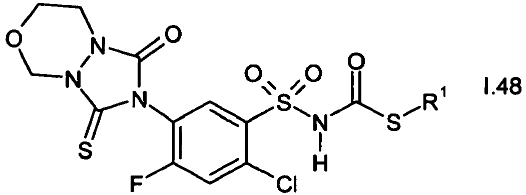

Ebenso außerordentlich bevorzugt sind die Verbindungen der Formel 1.49, insbesondere die Verbindungen der Formel I.49.1 bis I.49.689, die sich von den entsprechenden Verbindungen der Formel I.1.1 bis 1.1.689 dadurch unterscheiden, daß Q für Q⁷ (mit A¹⁶, A¹⁷ = Schwefel und R⁴⁶, R⁴⁷ bilden eine Kette -CH₂-CH₂-O-CH₂-) steht.

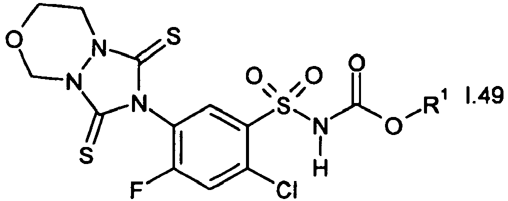

Ebenso außerordentlich bevorzugt sind die Verbindungen der Formel 1.50, insbesondere die Verbindungen der Formel I.50.1 bis I.50.689, die sich von den entsprechenden Verbindungen der Formel I.1.1 bis I.1.689 dadurch unterscheiden, daß B für Schwefel und Q für Q⁷ (mit A¹⁶, A¹⁷ = Schwefel und R⁴⁶, R⁴⁷ bilden eine Kette -CH₂-CH₂-O-CH₂-) stehen.

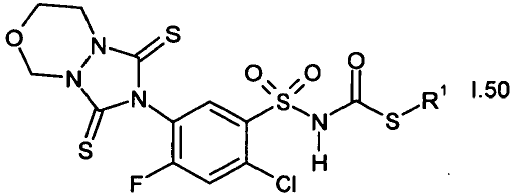

Ebenso außerordentlich bevorzugt sind die Verbindungen der Formel I.51, insbesondere die Verbindungen der Formel I.51.1 bis I.51.689, die sich von den entsprechenden Verbindungen der Formel I.1.1 bis I.1.689 dadurch unterscheiden, daß Q für Q⁷ (mit A¹⁶ = Sauerstoff, A¹⁷ = Schwefel und R⁴⁶, R⁴⁷ bilden eine Kette -CH₂-CH₂-O-CH₂-) steht.

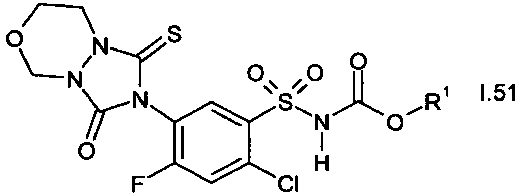

Ebenso außerordentlich bevorzugt sind die Verbindungen der Formel I.52, insbesondere die Verbindungen der Formel I.52.1 bis I.52.689, die sich von den entsprechenden Verbindungen der Formel I.1.1 bis I.1.689 dadurch unterscheiden, daß B für Schwefel und Q für Q⁷ (mit A¹⁶ = Sauerstoff, A¹⁷ = Schwefel und R⁴⁶, R⁴⁷ bilden eine Kette -CH₂-CH₂-O-CH₂-) stehen.

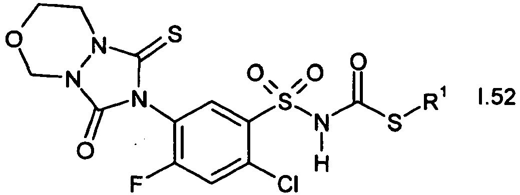

Die Benzolsulfonamid-Derivate der Formel I sind auf verschiedene Art und Weise erhältlich, beispielsweise nach einem der folgenden Verfahren:

### Verfahren A

Entsprechend substituierte Aromaten der Formel VIII werden über eine Chlorsulfonylierung in die entsprechenden Benzolsulfonylchloride der Formel VII überführt, welche dann mit Ammoniak zu den entsprechenden Sulfonamiden der Formel V umgesetzt werden. Die Sulfonamide der Formel V werden dann mit (Thio)phosgen der Formel VI zu den Benzolsulfonyliso(thio)cyanaten der Formel II umgesetzt, welche anschließend mit Aminen der Formel III oder Alkoholen bzw. Thiolen der Formel IV zu den gewünschten Benzolsulfonamid-Derivaten der Formel I, wobei X³ für Wasserstoff steht und die übrigen Reste die unter Anspruch 1 genannten Bedeutungen haben, reagieren:

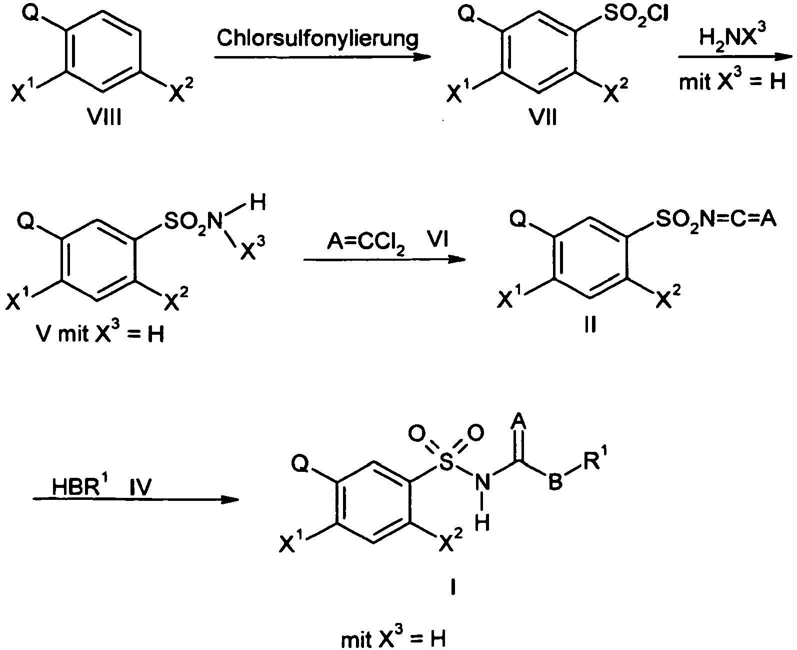

Q in Formel VIII steht für die oben genannten Reste Q¹ bis Q³⁹ oder für einen Substituenten, der eine für die Synthese von Q¹ bis Q³⁹ geeignete Vorstufe darstellt, z.B. eine Nitro- oder Carboxygruppe.
Die Chlorsufonylierung der Aromaten der Formel VIII zu den entsprechenden Benzolsulfonylchloriden der Formel VII erfolgt üblicherweise bei Temperaturen von 0°C bis 150°C, vorzugsweise 20°C bis 130°C, besonders bevorzugt 30°C bis 110°C, beispielsweise mit Chlorsulfonsäure, Sulforylchlorid (SO₂Cl₂)oder mit Sulforylchlorid in Gegenwart von Chlorsulfonsäure in einem inerten organischen Lösungsmittel [vgl. Houben-Weyl, Methoden der organischen Chemie, Bd. 9, 1955, S. 572 - 579].
Geeignete Lösungsmittel sind halogenierte Kohlenwasserstoffe wie Methylenchlorid, Chloroform und Chlorbenzol, Nitrile wie Acetonitril und Propionitril, sowie Chlorsulfonsäure, besonders bevorzugt Chlorsulfonsäure.
Es können auch Gemische der genannten Lösungsmittel verwendet werden. Gegebenenfalls kann diese Reaktion auch in Gegenwart eines Metallkatalysators, beispielsweise Aluminiumchlorid, nach Art einer Friedel-Crafts-Reaktion durchgeführt werden [vgl. Houben-Weyl, Methoden der organischen Chemie, Bd. 9, 1955, S. 578 - 579].
Als Säuren und saure Katalysatoren finden auch anorganische Säuren wie Fluorwasserstoffsäure, Salzsäure, Bromwasserstoffsäure, Schwefelsäure und Perchlorsäure, Lewis-Säuren wie Bortrifluorid, Aluminiumtrichlorid, Eisen-III-chlorid, Zinn-IV-chlorid, Titan-IV-chlorid und Zink-II-chlorid, Verwendung.
Die sauren Katalysatoren werden im allgemeinen in katalytischen Mengen eingesetzt, sie können aber auch äquimolar, im Überschuß oder gegebenenfalls als Lösungsmittel verwendet werden.
Die Edukte werden im allgemeinen in äquimolaren Mengen miteinander umgesetzt. Es kann vorteilhaft sein, Chlorsulfonsäue oder Sulforylchlorid in einem Überschuß bezogen auf VII einzusetzen, oder direkt in Chlorsulfonsäure als Lösungsmittel zu arbeiten.
Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.
Des weiteren können auch Dialkylsulfide mit Chlor in Gegenwart von Wasser gespalten und zu den entsprechenden Benzolsulfonylchloriden der Formel VII umgesetzt werden [vgl. Houben-Weyl, Methoden der organischen Chemie, Bd. 9, 1955, S. 580 - 582].
Analog lassen sich auch Thiophenole in die entsprechenden Benzolsulfonylchloriden der Formel VII umwandeln [vgl. Houben-Weyl, Methoden der organischen Chemie, Bd. 9, 1955, S. 582].
Benzolsulfonylchloride der Formel VII sind auch durch die Umsetzung von Benzolsulfonsäuren mit Chlorierungsmitteln wie Thionylchlorid, Phosgen, Phosphortrichlorid oder Phosphorpentachlorid darstellbar [vgl. Houben-Weyl, Methoden der organischen Chemie, Bd. 9, 1955, S. 564 - 568].
Des weiteren lassen sich auch Anilide über ihre Diazoniumsalze mittels Schwefeldioxid in Gegenwart von Kupfer(II)chlorid (Meerwein-Reaktion) in die entsprechenden Benzolsulfonsulfonylchloride der Formel VII überführen [vgl. Houben-Weyl, Methoden der organischen Chemie, Bd. 9, 1955, S. 579-580].
Die für die Herstellung der Verbindungen I benötigten Ausgangsstoffe sind in der Literatur bekannt [z.B. CAS 112, 157842; JP 01/168662] oder können gemäß der zitierten Literatur hergestellt werden.
Benzolsulfonylchloride der Formel VII, in denen Q für Q⁷ steht, sind z.B. aus WO 02/38562 bekannt.
Die Herstellung von Benzolsulfonylchloriden der Formel VII, in dem Q für Q²¹ steht, ist z.B. in US 5,169,430 beschrieben.
Benzolsulfonylchloride der Formel VII, in denen Q für Q³² steht, sind z.B. aus WO 96/15115 bekannt.
Die Herstellung von Benzolsulfonylchloriden der Formel VII, in dem Q für Q³⁸ steht, ist z.B. in WO 95/02580 beschrieben.
Die Herstellung von Benzolsulfonylchloriden der Formel VII mit weiteren Resten Q kann in Analogie zu den oben genannten Methoden durchgeführt werden (vgl. z.B.
JP 05/164386). Weitere Vorprodukte sind in Böger, Wakabayashi, Peroxidizing Herbicides, Springer Verlag 1999 beschrieben.

Die Folgereaktion der Benzolsulfonylchloride der Formel VII mit gasförmigem oder wässrigem Ammoniak zu den entsprechenden Sulfonamiden der Formel V mit X³ = Wasserstoff erfolgt üblicherweise bei Temperaturen von -10°C bis 50°C, vorzugsweise 0°C bis 30°C, besonders bevorzugt 5°C bis 15°C, in einem inerten organischen Lösungsmittel, gegebenenfalls in Gegenwart einer Base [vgl. US 5,169,430; WO 95/02580; Houben-Weyl, Methoden der organischen Chemie, Bd. 9, 1955, S. 398-400 und 605].
Vorzugsweise verwendet man Ammoniak in einem Überschuß von 200 bis 230%, man kann jedoch auch eine Hilfsbase einsetzen.
Als Hilfsbasen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetallamide wie Lithiumamid, Natriumamid und Kaliumamid, Alkalimetall- und Erdalkalimetallcarbonate wie Lithiumcarbonat, Kaliumcarbonat und Calciumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat, Kalium-tert.-Pentanolat und Dimethoxymagnesium, außerdem organische Basen, z.B. tertiäre Amine wie Trimethylamin, Triethylamin, Diisopropylethylamin und N-Methylpiperidin, Pyridin, substituierte Pyridine wie Collidin, Lutidin und 4-Dimethylaminopyridin sowie bicyclische Amine in Betracht.
Die Basen werden im allgemeinen in katalytischen Mengen eingesetzt, sie können aber auch äquimolar, im Überschuß oder gegebenenfalls als Lösungsmittel verwendet werden.
Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Toluol, o-, m- und p-Xylol, halogenierte Kohlenwasserstoffe wie Methylenchlorid, 1,2-Dichlorethan, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylformamid und Dimethylacetamid, besonders bevorzugt Dioxan, Tetrahydrofuran, 1,2-Dichlorethan, Toluol oder Cyclohexan.
Es können auch Gemische der genannten Lösungsmittel verwendet werden.
Die Aufarbeitung und Isolierung der Produkte kann in an sich bekannter Weise erfolgen.
Weitere Sulfonamide der Formel V lassen sich durch die analoge Umsetzung von Benzolsulfonylchloriden der Formel VII mit einem Amin H₂NX³ herstellen.
Sulfonamide der Formel V, in denen Q für Q⁷ steht, werden z.B. in WO 02/38562 genannt.
In US 5,169,430 und WO 95/02580 werden Sulfonamide der Formel V, in denen Q für Q²¹ bzw. Q²⁸ steht, beschrieben.

Die Umsetzung der Sulfonamide der Formel V mit X³ = Wasserstoff mit (Thio)phosgen der Formel VI zu Benzolsulfonyliso(thio)cyanaten der Formel II erfolgt üblicherweise bei Temperaturen von 50°C bis 110°C, vorzugsweise 60°C bis 90°C, in einem inerten organischen Lösungsmittel gegebenenfalls in Gegenwart eines Katalysators [vgl. Houben-Weyl, Methoden der organischen Chemie, Bd. 11,2, 1985, S. 1106; US 4,379,769; DD 238 522].
Als Katalysatoren eignen sich z.B. aliphatische Isocyanante wie z.B. n-Propylisocyanat, Isoropylisocyanat oder n-Butylisocyanat.
Der Katalysator wird im allgemeinen in einem Unterschuß von 5% bis 15% pro Mol Sulfonamid der Formel V eingesetzt.
Geeignete Lösungsmittel sind aliphatische Kohlenwasserstoffe wie Pentan, Hexan, Cyclohexan und Gemische von C₅-C₆-Alkanen, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, besonders bevorzugt Toluol, 1,2-Dichlorethan oder Chlorbenzol.
Es können auch Gemische der genannten Lösungsmittel verwendet werden.
Die Edukte werden im allgemeinen in äquimolaren Mengen miteinander umgesetzt. Es kann vorteilhaft sein, die VI in einem Überschuß bezogen auf V einzusetzen.
Die Aufarbeitung und Isolierung der Produkte kann in an sich bekannter Weise erfolgen.

Die Umsetzung der Sulfonamide der Formel V mit X³ = Wasserstoff zu Benzolsulfonyliso(thio)cyanaten der Formel II kann auch mit Diphosgen [ClC(O)OCCl₃] oder mit Schwefelkohlenstoff in Phosgen erfolgen.
Zweckmäßiger Weise können die Sulfonamide der Formel V mit X³ = Wasserstoff auch zunächst mit Thionylchlorid unter Rückfluß vorbehandelt und anschließend mit Phosgen zu Benzolsulfonyliso(thio)cyanaten der Formel II umgesetzt werden (vgl. DE 43 22 726).
Benzolsulfonyliso(thio)cyanate der Formel II können auch durch die Reaktion von Sulfonamiden der Formel V mit X³ = Wasserstoff mit Chlorsulfonylisocyanat hergestellt werden (vgl. DE 31 32 944).
Benzolsulfonyliso(thio)cyanate der Formel II lassen sich ferner in an sich bekannter Weise durch Umsetzung von Benzolsulfonylchloriden der Formel VII mit Alkylimetallisocyanaten herstellen (vgl. US 4,546,179).

Die Umsetzung von Benzolsulfonyliso(thio)cyanaten der Formel II mit einem primären Amin der Formel III oder einem Alkohol bzw. Thiol der Formel IV zu den gewünschten Benzolsulfonamid-Derivaten der Formel I mit X³ = Wasserstoff erfolgt üblicherweise bei Temperaturen von 0°C bis 120°C, vorzugsweise 10°C bis 100°C, besonders bevorzugt 20°C bis 70°C, in einem inerten organischen Lösungsmittel [vgl. EP 162 723].
Die Reaktion kann unter Normaldruck oder unter erhöhtem Druck (bis 50 bar), vorzugsweise bei 1 bis 5 bar, kontinuierlich oder diskontinuierlich durchgeführt werden. Geeignete Lösungsmittel sind aliphatische Kohlenwasserstoffe wie Pentan, Hexan, Cyclohexan und Gemische von C₅-C₈-Alkanen; Nitrokohlenwasserstoffe wie Nitromethan, Nitroethan, Nitrobenzol, o-, m-, p-Chlornitrobenzol und o-Nitrotoluol; aromatische Kohlenwasserstoffe wie Toluol, o-, m- und p-Xylol, halogenierte Kohlenwasserstoffe wie Methylenchlorid, 1,2-Dichlorethan, Chloroform und Chlorbenzol, Ether wie Diethylether, Diisopropylether, tert.-Butylmethylether, Dioxan, Anisol und Tetrahydrofuran sowie Nitrile wie Acetonitril und Propionitril, besonders bevorzugt Tetrahydrofuran, Dioxan sowie 1,2-Dichlorethan.
Es können auch Gemische der genannten Lösungsmittel verwendet werden.
Als Katalysator kann vor oder während der Reaktion eine Base zugesetzt werden, wodurch die Reaktion beschleunigt und die Produktqualität verbessert wird.
Als Basen kommen allgemein organische Basen, z.B. tertiäre Amine wie Trimethylamin, Triethylamin, Diisopropylethylamin, Tri(n-propyl)amin, N-Methylpiperidin, Pyridin, substituierte Pyridine wie Collidin, Lutidin und 4-Dimethylaminopyridin sowie bicyclische Amine in Betracht. Besonders bevorzugt werden Triethylamin oder 1,4-Diazabicyclo[2,2,2]octan.
Die Basen werden im allgemeinen in katalytischen Mengen eingesetzt, sie können aber auch äquimolar verwendet werden.
Die Benzolysulfonyliso(thio)cyanate der Formel II werden im allgemeinen in äquimolaren Mengen mit dem primären Amin der Formel III bzw. dem Alkohol bzw. Thiol der Formel IV umgesetzt. Es kann vorteilhaft sein, III oder IV in einem Überschuß bezogen auf II einzusetzen.
Die Aufarbeitung und Isolierung der Produkte kann in an sich bekannter Weise erfolgen.

### Verfahren B

Sulfonamide der Formel V können mit Halogeniden der Formel XI zu den gewünschten Benzolsulfonamid-Derivaten der Formel I umgesetzt werden:

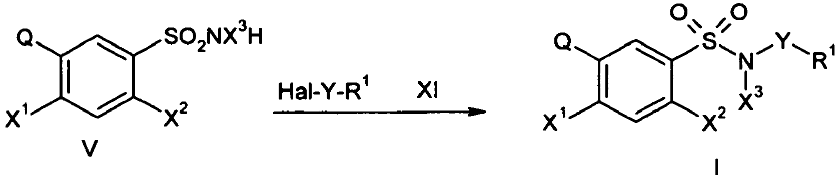

Hal in Formel XI steht für Halogen wie Fluor, Chlor, Brom, besonders bevorzugt Chlor. Diese Umsetzung erfolgt üblicherweise bei Temperaturen von 0°C bis 150°C, vorzugsweise 10°C bis 100°C, in einem inerten organischen Lösungsmittel [vgl. JP 05/194386, CAS 120, 134277].
Die Reaktion kann unter Normaldruck oder unter erhöhtem Druck (bis 50 bar), vorzugsweise bei 1 bis 5 bar, kontinuierlich oder diskontinuierlich, durchgeführt werden. Geeignete Lösungsmittel sind aliphatische oder cycloaliphatische Kohlenwasserstoffe wie Pentan, 1,2,4-Trimethylpentan, 2,2,3-Trimethylpentan, 2,3,3-Trimethylpentan, Hexan, Heptan, Octan, Nonan, Gemische von C₅-C₈-Alkanen, Pinan, Cyclohexan, Methylcyclohexan, o-, m-, p-Cymol, Benzinfraktionen innerhalb eines Siedepunktintervalls von 70°C bis 190°C, Dekalin, Petrolether, Ligroin; Nitrokohlenwasserstoffe wie Nitromethan, Nitroethan, Nitrobenzol, o-, m-, p-Chlornitrobenzol und o-Nitrotoluol; aromatische Kohlenwasserstoffe wie Toluol, o-, m- und p-Xylol, halogenierte Kohlenwasserstoffe wie 1,2-Dichlorethan, 1,1-Dichlorethan, 1,2-cis-Dichlorethylen, 1,1,1- oder 1,1,2-Trichlorethan, Trichlorethylen, Tetrachlorethylen, 1,1,2,2- oder 1,1,1,2-Tetrachlorethan, Pentachlorethan, Dichlorpropan, Methylenchlorid, Dichlorbutan, Chloroform, Tetrachlorkohlenstoff, Fluorbenzol, Chlorbenzol, Brombenzol, Jodbenzol, o-, m-, p-Difluorbenzol, o-, m-, p-Dichlorbenzol, o-, m-, p-Dibrombenzol, o-, m-, p-Chlortoluol, 1,2,4-Trichlorbenzol, Chlornaphthalin, Dichlornaphthalin; Ether wie Diethylether, E-thylpropylether, Diisopropylether, tert.-Butylmethylether, n-Butylethylether, Di-n-butylether, Diisobutylether, Diisoamylether, Dioxan, Cyclohexylmethylether, Ethylenglykoldimethylether, β,β'-Dichlordiethylether, Tetrahydrofuran, Anisol, Thioanisol, Phenetol; Nitrile wie Acetonitril, Propionitril, Butyronitril, Isobutyronitril, Benzonitril, m-Chlorbenzonitril; Ketone wie Aceton, Methylethylketon, Diethylketon und tert.-Butylmethylketon, sowie Dimethylformamid; Ester wie Ethylacetat, Acetessigester, Isobutylacetat; Amide wie Formamid, Methylformamid, Dimethylformamid; besonders bevorzugt 1,2-Dichlorethan, Tetrahydrofuran, Ethylacetat, Acetonitril sowie Toluol.
Es können auch Gemische der genannten Lösungsmittel verwendet werden.
Als Katalysator kann vor oder während der Reaktion eine Base zugesetzt werden, wodurch die Reaktion beschleunigt und die Produktqualität verbessert wird.
Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetallcarbonate wie Lithiumcarbonat, Kaliumcarbonat und Calciumcarbonat sowie Alkalimetallhydrogencarbonate wie Natriumhydrogencarbonat, außerdem organische Basen, z.B. tertiäre Amine wie Trimethylamin, Triethylamin, Diisopropylethylamin, Tri(n-propyl)amin , N-Methylpiperidin, Pyridin, substituierte Pyridine wie Collidin, Lutidin und 4-Dimethylaminopyridin sowie bicyclische Amine in Betracht. Besonders bevorzugt werden Triethylamin oder 2,4,6-Collidin. Die Basen werden im allgemeinen in katalytischen Mengen eingesetzt, sie können aber auch äquimolar verwendet werden.
Die Sulfonamide der Formel V werden im allgemeinen in äquimolaren Mengen mit dem Isocyanat bzw. Isothiocyanat der Formel X umgesetzt. Es kann vorteilhaft sein, XI in einem Überschuß bezogen auf V einzusetzen.
Zur Beendigung der Umsetzung kann nach Zugabe der Komponenten die Reaktionsmischung noch 20 min bis 24 h bei 0 bis 120 °C, vorzugsweise 10 bis 100°C, insbesondere 20 bis 80 °C nachgerührt werden.
Die Aufarbeitung und Isolierung der Produkte kann in an sich bekannter Weise erfolgen.

### Verfahren C

Verbindungen der Formel I, in denen die Reste Q an ihren Stickstoffatomen die Substituenten R³, R⁴, R⁷, R¹¹, R¹⁸, R¹⁹, R²⁴, R²⁷, R²⁹, R³², R³⁹, R⁴⁴-R⁴⁷ tragen, [wobei diese Reste unter anderem für C₁-C₆-Alkyl oder Amino, C₁-C₆-Alkylamino oder Di(C₁-C₆-alkyl)amino stehen], lassen sich herstellen, indem entweder vor dem Aufbau der Sulfonamidseitenkette (d.h., auf der Stufe der Aromaten der Formel VIII) oder nach Aufbau der Sufonamidseitenkette mit einem Alkylhalogenid, -sulfat, -tosylat oder einem elektrophilen Aminierungsreagenz der Formel XVII analog zu den in der Literatur beschriebenen Methoden umgesetzt wird.
Beispiele für elektrophile Aminierungsreagenzien der Formel XVII sind 2,4-Dinitrophenylhydroxylamin und o-Mesitylensulfonylhydroxylamin.
Beispielsweise lassen sich die oben genannten Benzolsulfonsäurechloride der Formel VII durch Einwirkung von Alkoholen, zweckmäßigerweise in Gegenwart einer Base, in die entsprechenden Benzolsulfonsäureester umwandeln [vgl. Houben-Weyl, Methoden der organischen Synthese, Bd. 9, 1955, S. 663]. Anschließend lassen sich die Benzolsulfonsäureester an den freien Stickstoffatomen der entsprechenden Reste Q alkylieren oder aminieren. Im Anschluß daran können die Benzolsulfonsäureester wieder verseift werden [vgl. Kocienski, Protecting groups, Thieme-Verlag 1994; Greene, Wuts, Protecting groups in organic sythesis, Wiley 1999; Houben-Weyl, Methoden der organischen Chemie, Bd. E5 Teil I, 1985, S. 223f.).
Beispielhaft sei hier eine Aminierung am Rest Q = Q²¹ dargestellt. Die Aminierungen für die anderen Reste Q sowie Alkylierungen an den Resten Q sind analog durchführbar. Man erhält auf diesem Weg z.B. Sulfonsäuren der Formel XVI. Diese können anschließend nach literaturbekannten Methoden zu den gewünschten Benzolsulfonamid-Derivaten der Formel I umgesetzt werden.

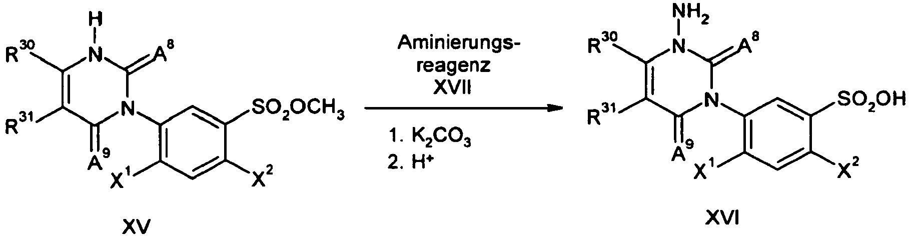

Diese Umsetzung erfolgt üblicherweise bei Temperaturen von 10°C bis 80°C, vorzugsweise 20°C bis 40°C, in einem inerten organischen Lösungsmittel in Gegenwart einer Base [vgl. DE 19 652 431; WO 01/83459].
Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid, Dimethylformamid und Dimethylacetamid, besonders bevorzugt Tetrahydrofuran, Dioxan, Acetonitril sowie Dimethylformamid.
Es können auch Gemische der genannten Lösungsmittel verwendet werden.
Als Basen kommen allgemein anorganische Verbindungen, z.B. Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetall- und Erdalkalimetallcarbonate wie Lithiumcarbonat, Kaliumcarbonat und Calciumcarbonat sowie Alkalimetallhydrogencarbonate wie Natriumhydrogencarbonat, Alkalimetall- und Erdalkalimetallalkoholate wie Natriummethanolat, Natriumethanolat, Kaliumethanolat, Kalium- tert.-Butanolat, Kalium-tert.-pentanolat und Dimethoxymagnesium, außerdem organische Basen, z.B. tertiäre Amine wie Trimethylamin, Triethylamin, Diisopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Kaliumcarbonat sowie Calciumcarbonat.
Die Basen werden im allgemeinen in katalytischen Mengen eingesetzt, sie können aber auch äquimolar, im Überschuß oder gegebenenfalls als Lösungsmittel verwendet werden.
Die Edukte werden im allgemeinen in äquimolaren Mengen miteinander umgesetzt. Es kann aber auch vorteilhaft sein, XVII in einem Überschuß bezogen auf XV einzusetzen. Die Aufarbeitung und Isolierung der Produkte kann in an sich bekannter Weise erfolgen.
Die für die Herstellung der Verbindungen I benötigten Ausgangsstoffe sind in der Literatur bekannt [z. B. CAS 112, 157842; JP 01168662] oder können gemäß der zitierten Literatur hergestellt werden.

Benzolsulfonyliso(thio)cyanate der Formel II 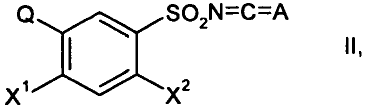 wobei X¹, X², A und Q die unter Anspruch 1 genannten Bedeutungen haben, sind ebenfalls ein Gegenstand der vorliegenden Erfindung.

Die besonders bevorzugten Ausführungsformen der Zwischenprodukte in Bezug auf die Variablen entsprechen denen der Reste X¹, X², A und Q der Formel I.

Besonders bevorzugt werden Zwischenprodukte der Formel IV, in denen
- X¹: Wasserstoff, Fluor oder Chlor;
besonders bevorzugt Wasserstoff oder Fluor;
insbesondere bevorzugt Fluor; und
- Q: Q¹, Q², Q⁵, Q⁷, Q⁸, Q¹⁰, Q¹², Q¹³, Q¹⁷, Q²⁰ Q²¹, Q²², Q²³, Q²⁴, Q²⁷, Q³¹, Q³², Q³⁴, Q³⁸ oder Q³⁹,
besonders bevorzugt Q¹, Q², Q⁵, Q⁷, Q⁸, Q¹⁰, Q¹², Q¹³, Q¹⁷, Q²⁰, Q²¹, Q²², Q²⁴, Q²⁷, Q³¹, Q³², Q³⁸ oder Q³⁹,
insbesondere bevorzugt Q⁵, Q⁷, Q²¹, Q²², Q²⁷, Q³², Q³⁸ oder Q³⁹,
außerordentlich bevorzugt Q⁷, Q²¹, Q²², Q²⁷, Q³², Q³⁸ oder Q³⁹,
sehr außerordentlich bevorzugt Q²¹, Q³² oder Q³⁸
bedeuten.

### Herstellungsbeispiele

### Beispiel 1

### 2-Ch lor-5-[3,6-dihydro-3-methyl-2,6-dioxo-4-trifluormethyl-(2H)-pyrim idin-1-yl]-benzolsulfonamid

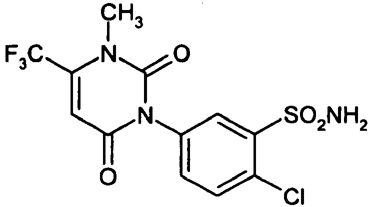

1.6 g (93.8 mmol) Ammoniakgas wurden unter Rühren bei 0°C in eine Mischung aus 18 g (44.6 mmol) 2-Chlor-5-[3,6-dihydro-3-methyl-2,6-dioxo-4-trifluormethyl-(2H)-pyrimidin-1-yl]-benzolsulfonylchlorid in Tetrahydrofuran (THF) geleitet. Anschließend wurde bei 10 °C Essigsäureethylester zugegeben und mit 1 N Salzsäure angesäuert. Nach Trennung der Phasen und Extraktion der wässrigen Phase wurden die vereinigten organischen Phasen gewaschen, getrocknet und vom Lösungsmittel befreit. Nach üblichen Reinigungsmethoden erhielt man 14.4 g (82.4% der Theorie) der Titelverbindung (Schmp.: 257-258 °C).

### Beispiel 2

### 2-Chlor-4-fluor-5-[3,6-dihydro-3-methyl-2,6-dioxo-4-trifluormethyl-(2H)-pyrimidin-1-yl] benzolsulfonylisocyanat

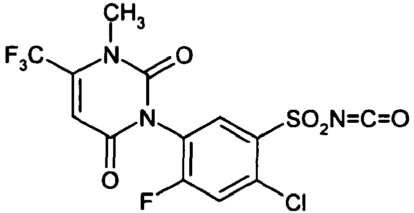

7.4 g (62.3 mmol) Thionylchlorid wurden bei 60°C unter Rühren zu einer Suspension von 10.0 g (24.9 mmol) 2-Chlor-4-fluor-5-[3,6-dihydro-3-methyl-2,6-dioxo-4-trifluormethyl-(2H)-pyrimidin-1-yl]-benzolsulfonylamid in 1,2-Dichlorethan getropft. Anschließend wurde 4 h am Rückfluß gekocht. Anschließend wurde auf 60°C abgekühlt, katalytische Mengen Pyridin zugegeben und 12 h unter Rückfluß Phosgen eingeleitet, bis eine klare Lösung entstand. Nach Abkühlen auf 30 °C wurde das Produkt vom Lösungsmittel befreit. Man erhielt 11.6 g (98% der Theorie) der Titelverbindung. ¹H-NMR (400 MHz, CDCl₃) δ [ppm] = 8.12 (d, 1H), 7.55 (d, 1H), 6.38 (s, 1H), 3.57 (s, 3H).

### Beispiel 3

### 2-Chlor-5-[3,6-dihydro-3-methyl-2, 6-dioxo-4-trifluormethyl-(2H)-pyrimidin-1-yl]-benzolsulfonylisocyanat

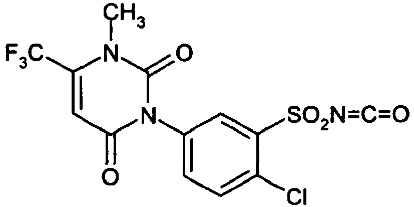

10.0 g (26.1 mmol) 2-Chlor-5-[3,6-dihydro-3-methyl-2,6-dioxo-4-trifluormethyl-(2H)-pyrimidin-1-yl]-benzolsulfonamid wurde analog der in Beispiel 2 beschriebenen Methode umgesetzt. Man erhielt 13.4 g (99% der Theorie) der Titelverbindung.
¹H-NMR (400 MHz, CDCl₃) δ [ppm] = 8.02 (s, 1H), 7.76 (d, 2H), 7.5 (d, 1H), 6.38 (s, 1H), 3.70 (s, 3H).

### Beispiel 4 (Nr. 2.32)

### Benzyl{2-chlor-4-fluor-5-[3,6-dihydro-3-methyl-2,6-dioxo-4-trifluormethyl-(2H)-pyrimidin-1-yl]-phenyl}sulfonylcarbamat

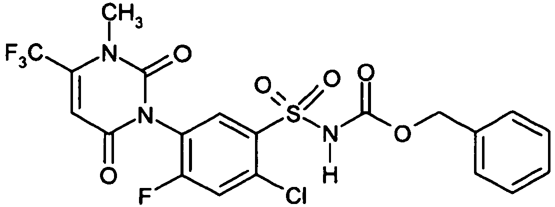

0.6 g (1.4 mmol) 2-Chlor-4-fluor-5-[3,6-dihydro-3-methyl-2,6-dioxo-4-trifluormethyl-(2H)-pyrimidin-1-yl]-benzolsulfonylisocyanat in 1,2-Dichlorethan wurden unter Rühren zu einer Lösung von 0.15 g (1.4 mmol) Benzylalkohol in Methylenchlorid gegeben und die Reaktionsmischung über Nacht gerührt. Nach Entfernen des Lösungsmittels und üblichen Reinigungsmethoden erhielt man 0.4 g (52% der Theorie) der Titelverbindung als farblosen Feststoff (Schmp: 231-232 °C).

In Tabelle 2 sind neben den voranstehenden Verbindungen noch weitere Benzolsulfonamid-Derivate der Formel I aufgeführt, die in analoger Weise nach den voranstehend beschriebenen Verfahren hergestellt wurden oder herstellbar sind.

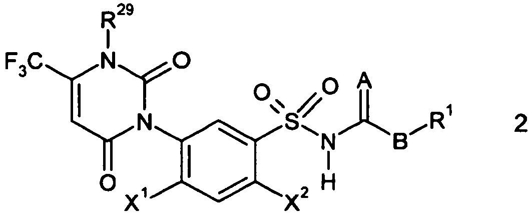

**Tabelle 2**

| Nr. | X¹ | X² | A | B | R¹ | R²⁹ | Schmp. [°C] |
|---|---|---|---|---|---|---|---|
| 2.1 | H | Cl | O | O | CH₃ | CH₃ | 120-148 |
| 2.2 | H | Cl | O | O | C₂H₅ | CH₃ | 189-190 |
| 2.3 | H | Cl | O | O | CH₂CH₂CH₃ | CH₃ | |
| 2.4 | H | Cl | O | O | CH(CH₃)₂ | CH₃ | |
| 2.5 | H | Cl | O | O | (CH₂)₃CH₃ | CH₃ | 194-195 |
| 2.6 | H | Cl | O | O | CH(CH₃)CH₂CH₃ | CH₃ | |
| 2.7 | H | Cl | O | O | CH₂CH(CH₃)₂ | CH₃ | |
| 2.8 | H | Cl | O | O | C(CH₃)₃ | CH₃ | |
| 2.9 | H | Cl | O | O | (CH₂)₄CH₃ | CH₃ | |
| 2.10 | H | Cl | O | O | Cyclopentyl | CH₃ | 114-116 |
| 2.11 | H | Cl | O | O | CH₂CH₂Cl | CH₃ | |
| 2.12 | H | Cl | O | O | (CH₂)OCH₃ | CH₃ | |
| 2.13 | H | Cl | O | O | (CH₂)SCH₃ | CH₃ | |
| 2.14 | H | Cl | O | O | CH₂CH₂CN | CH₃ | |
| 2.15 | H | Cl | O | S | CH₃ | CH₃ | |
| 2.16 | H | Cl | O | S | C₂H₅ | CH₃ | |
| 2.17 | H | Cl | O | S | CH₂CH₂CH₃ | CH₃ | |
| 2.18 | F | Cl | O | O | CH₃ | CH₃ | 120-135 |
| 2.19 | F | Cl | O | O | C₂H₅ | CH₃ | 228-231 |
| 2.20 | F | Cl | O | O | CH₂CH₂CH₃ | CH₃ | 203 |
| 2.21 | F | Cl | O | O | CH(CH₃)₂ | CH₃ | 228-230 |
| 2.22 | F | Cl | O | O | (CH₂)₃CH₃ | CH₃ | 238 |
| 2.23 | F | Cl | O | O | CH(CH₃)CH₂CH₃ | CH₃ | 195-198 |
| 2.24 | F | Cl | O | O | CH₂CH(CH₃)₂ | CH₃ | 233-235 |
| 2.25 | F | Cl | O | O | C(CH₃)₃ | CH₃ | 185 |
| 2.26 | F | Cl | O | O | (CH₂)₄CH₃ | CH₃ | 235 |
| 2.27 | F | Cl | O | O | Cyclopentyl | CH₃ | 214 |
| 2.28 | F | Cl | O | O | CH₂CH₂Cl | CH₃ | |
| 2.29 | F | Cl | O | O | (CH₂)OCH₃ | CH₃ | |
| 2.30 | F | Cl | O | O | (CH₂)SCH₃ | CH₃ | |
| 2.31 | F | Cl | O | O | CH₂CH₂CN | CH₃ | |
| 2.32 | F | Cl | O | O | CH₂C₆H₅ | CH₃ | 231-232 |
| 2.33 | F | Cl | O | S | CH₃ | CH₃ | |
| 2.34 | F | Cl | O | S | C₂H₅ | CH₃ | |
| 2.35 | F | Cl | O | S | CH₂CH₂CH₃ | CH₃ | |
| 2.36 | Cl | Cl | O | S | CH₃ | CH₃ | |
| 2.37 | Cl | Cl | O | S | C₂H₅ | CH₃ | |
| 2.38 | Cl | Cl | O | S | CH₂CH₂CH₃ | CH₃ | |
| 2.39 | Cl | Cl | O | O | CH₃ | CH₃ | 218-220 |
| 2.40 | Cl | Cl | O | O | C₂H₅ | CH₃ | 235-237 |
| 2.41 | F | Cl | O | O | CH₂COOCH₃ | CH₃ | 142-160 |
| 2.42 | F | Cl | O | O | C(CH₃)₂CH₂OCH₃ | CH₃ | 178 |

### Biologische Wirksamkeit

Die Benzolsulfonamid-Derivate der Formel I und deren landwirtschaftlich brauchbaren Salze eignen sich - sowohl als Isomerengemische als auch in Form der reinen Isomeren - als Herbizide. Die Verbindungen der Formel I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen der Formel I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen der Formel I auch in Kulturen, die durch Züchtung einschließlich gentechnischer Methoden gegen die Wirkung von Herbiziden tolerant sind, verwandt werden.

Des Weiteren eignen sich die Benzolsulfonamid-Derivate der Formel I und deren landwirtschaftlich brauchbaren Salze auch zur Desikkation und / oder Defoliation von Pflanzen.

Als Desikkantien eignen sie sich insbesondere zur Austrocknung der oberirdischen Teile von Kulturpflanzen wie Kartoffel, Raps, Sonnenblume und Sojabohnen. Damit wird ein vollständig mechanisches Beernten dieser wichtigen Kulturpflanzen ermöglicht.

Von wirtschaftlichem Interesse ist auch
- das zeitlich konzentrierte Abfallen von Früchten oder das Vermindern ihrer Haftfestigkeit an der Pflanze, beispielsweise bei Zitrusfrüchten, Oliven oder anderen Arten und Sorten von Kern-, Stein- und Schalenobst, da hierdurch die Ernte dieser Früchte erleichtert wird, sowie
- das kontrollierte Entblättern von Nutzpflanzen, insbesondere Baumwolle (Defoliation).

Das dadurch die Anwendung von erfindungsgemäßen Verbindungen der Formel I und deren landwirtschaftlich brauchbaren Salzen geförderte Abfallen beruht auf der Ausbildung von Trenngewebe zwischen Frucht- oder Blatt- und Sprossteil der Pflanzen.

Die Baumwolldefoliation ist von ganz besonderem wirtschaftlichem Interesse, da sie die Ernte erleichtert. Gleichzeitig führt die Verkürzung des Zeitintervalls, in dem die einzelnen Pflanzen reif werden, zu einer erhöhten Qualität des geernteten Fasermaterials.

Die Verbindungen der Formel I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel enthalten eine herbizid wirksame Menge mindestens einer Verbindung der Formel I oder eines landwirtschaftlich brauchbaren Salzes von I und für die Formulierung von Pflanzenschutzmitteln übliche Hilfsmittel.
Als inerte Hilfsstoffe kommen im Wesentlichen in Betracht:
Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser. Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.
Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.
Pulver-, Streu- und Stäubemittel können durch Mischen oder gemeinsames Vermahlen der wirksamen Substanzen mit einem festen Trägerstoff hergestellt werden. Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Verbindungen der Formel I in den anwendungsfertigen Zubereitungen können in weiten Bereichen variiert werden. Im allgemeinen enthalten die Formulierungen etwa von 0.001 bis 98 Gew.%, vorzugsweise 0.01 bis 95 Gew.-%, mindestens eines Wirkstoffs. Die Wirkstoffe werden dabei in einer Reinheit von 90% bis 100%, vorzugsweise 95% bis 100% (nach NMR-Spektrum) eingesetzt.

Die folgenden Formulierungsbeispiele verdeutlichen die Herstellung solcher Zubereitungen:
I. 20 Gewichtsteile eines Wirkstoffs der Formel I werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Rizinusöl besteht. Durch Ausgießen und feines Verteilen der Lösung in 100000 Gewichtsteilen Wasser erhält man eine wäßrige Dispersion, die 0,02 Gew.-% des Wirkstoffs der Formel I enthält.
II. 20 Gewichtsteile eines Wirkstoffs der Formel I werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Rizinusöl besteht. Durch Eingießen und feines Verteilen der Lösung in 100000 Gewichtsteilen Wasser erhält man eine wäßrige Dispersion, die 0,02 Gew.-% des Wirkstoffs der Formel I enthält.
III. 20 Gewichtsteile eines Wirkstoffs der Formel I werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs der Formel I enthält.
IV. 20 Gewichtsteile eines Wirkstoffs der Formel I werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser erhält man eine Spritzbrühe, die 0,1 Gew.-% des Wirkstoffs der Formel I enthält.
V. 3 Gewichtsteile eines Wirkstoffs der Formel I werden mit 97 Gewichtsteilen feinteiligem Kaolin vermischt. Man erhält auf diese Weise ein Stäubemittel, das 3 Gew.-% des Wirkstoffs der Formel I enthält.
VI. 20 Gewichtsteile eines Wirkstoffs der Formel I werden mit 2 Gewichtsteilen Calciumsalz der Dodecylbenzolsulfonsäure, 8 Gewichtsteilen Fettalkoholpolyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil eines Wirkstoffs der Formel I wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. 1 Gewichtsteil eines Wirkstoffs der Formel I wird in einer Mischung gelöst, die aus 80 Gewichtsteilen Cyclohexanon und 20 Gewichtsteilen Wettol^{R} EM 31 (= nichtionischer Emulgator auf der Basis von ethoxyliertem Rizinusöl) besteht. Man erhält ein stabiles Emulsionskonzentrat.

Die Applikation der Verbindungen der Formel I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Verbindung der Formel I betragen je nach Bekämpfungsziel, Jahreszeit, Zielpflanzen und Wachstumsstadium 0.001 bis 3.0, vorzugsweise 0.01 bis 1.0 kg/ha aktive Substanz (a.S.).

Zur Verbreiterung des Wirkungsspektrums und zur Erzielung synergistischer Effekte können die Benzolsulfonamid-Derivate der Formel I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexenonoximetherderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen der Formel I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Anwendungsbeispiele (herbizide Wirksamkeit)

Die herbizide Wirkung der Benzolsulfonamid-Derivate der Formel I ließ sich durch die folgenden Gewächshausversuche zeigen:
Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt.

Die Aufwandmenge für die Vor- und Nachauflaufbehandlung betrug zwischen 62.5 und 3.1 g a.S./ha.

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Abutilon theophrasti | Chinesischer Hanf | Velvetleaf |
| Amaranthus retroflexus | Amarant | Pigweed |
| Chenopodium album | Weißer Gänsefuß | Common lambsquaters |
| Commelina benghalensis | Bengalische Commeline | Bengal commelina |
| Galium aparine | Klettenlabkraut | Cleavers harrif |
| Ipomoea hederacea | Trichterwinde | Morningglory |
| Pharbitis purpurea | Purpurne Trichterwinde | Common morningglory |
| Polygonum convolvulus | Windenknöterich | wild Buckwheat |
| Polygonum persicaria | Flohknöterich | Ladysthump |
| Solanum nigrum | Nachtschatten | Common nightshade |

Bei Aufwandmengen von 12.5 bzw. 6.2 g/ha zeigten die Verbindungen 2.1 und 2.18 (Tabelle 2) im Vorauflauf eine sehr gute Wirkung gegen die unerwünschten Pflanzen Amarant, Weißer Gänsefuß, Purpurne Trichterwinde und Windenknöterich.

Weiterhin bekämpfte Verbindung 2.24 (Tabelle 2) im Vorauflauf bei Aufwandmengen von 6.2 bzw. 3.1 g/ha die unerwünschten Pflanzen Chinesischer Hanf, Amarant, Weißer Gänsefuß und Trichterwinde sehr gut.

Die Wirkung von Verbindung 2.32 (Tabelle 2) im Vorauflauf bei Aufwandmengen von 62.5 bzw. 31.2 g/ha auf die unerwünschten Pflanzen Chinesischer Hanf, Amarant, Weißer Gänsefuß und Nachtschatten war sehr gut.

Bei Aufwandmengen von 15.6 bzw. 7.8 g/ha zeigten die Verbindungen 2.27, 2.19, 2.20 und 2.22 (Tabelle 2) im Nachauflauf eine sehr gute Wirkung gegen die unerwünschten Pflanzen Amarant, Weißer Gänsefuß, Purpurne Trichterwinde und Flohknöterich.

Weiterhin bekämpfte Verbindung 2.26 (Tabelle 2) im Nachauflauf bei Aufwandmengen von 15.6 bzw. 7.8 g/ha die unerwünschten Pflanzen Amarant, Weißer Gänsefuß und Purpurne Trichterwinde sehr gut.

Die Wirkung von Verbindung 2.42 (Tabelle 2) im Nachauflauf bei Aufwandmengen von 15.63 g/ha auf die unerwünschten Pflanzen Chinesischer Hanf, Purpurne Trichterwinde und Flohknöterich war sehr gut.

Bei Aufwandmengen von 15,63 g/ha zeigt die Verbindung 2.41 (Tabelle 2) im Nachauflauf eine sehr gute Wirkung gegen die unerwünschten Pflanzen Amarant, Klettenlabkraut und Flohknöterich.

### Anwendungsbeispiele (desikkative/defoliante Wirksamkeit)

Als Testpflanzen dienten junge, 4-blättrige (ohne Keimblätter) Baumwollpflanzen, die unter Gewächshausbedingungen angezogen wurden (rel. Luftfeuchtigkeit 50 bis 70 %; Tag-/Nachttemperatur 27/20°C).

Die jungen Baumwollpflanzen wurden tropfnass mit wässrigen Aufbereitungen der Wirkstoffe (unter Zusatz von 0,15 Gew.-% des Fettalkoholalkoxylats Plurafac^{®} LF 700¹), bezogen auf die Spritzbrühe) blattbehandelt. Die ausgebrachte Wassermenge betrug umgerechnet 1000 l/ha. Nach 13 Tagen wurde die Anzahl der abgeworfenen Blätter und der Grad der Entblätterung in % bestimmt.
¹⁾ ein schaumarmes, nichtionisches Tensid der BASF AG

Bei den unbehandelten Kontrollpflanzen trat kein Blattfall auf.

## Patentansprüche

1. Benzolsulfonamid-Derivate der Formel I 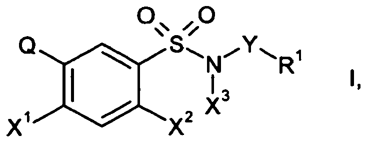 in der die Variablen die folgenden Bedeutungen haben:
X¹ Wasserstoff oder Halogen;
X² Chlor;
X³ Wasserstoff, Cyano, C₁-C₆-Alkyl, C₁-C₆-Alkoxy-C₁-C₄-alkyl, C₃-C₇-Cycloalkyl, C₃-C₆-Alkenyl, C₃-C₆-Alkinyl oder Phenyl-C₁-C₄-alkyl,
wobei der Phenylrest seinerseits partiell oder vollständig halogeniert und/oder durch ein bis drei Reste aus der Gruppe C₁-C₆-Alkyl und C₁-C₆-Alkoxy substituiert sein kann;
Y eine Gruppe -C(A)B;
A Sauerstoff;
B Sauerstoff oder Schwefel;
R¹ Wasserstoff, Hydroxy, C₁-C₈-Alkyl, C₃-C₇-Cycloalkyl, C₃-C₇-Cycloalkyl-C₁-C₄-alkyl, C₂-C₈-Alkenyl, C₁-C₇-Cycloalkenyl, C₃-C₈-Alkinyl, C₁-C₈-Alkoxy, C₃-C₇-Cycloalkyloxy, C₂-C₈-Alkenyloxy, C₃-C₈-Alkinyloxy, Aryl, Aryloxy, Aryl-C₁-C₄-alkyl;
wobei die 13 letztgenannten Reste ihrerseits partiell oder vollständig halogeniert sein können und/oder ein bis drei Substituenten aus der Gruppe Cyano, NO₂, Hydroxy, C₁-C₆-Alkyl, C₁-C₆-Halogenalkyl, C₃-C₇-Cycloalkyl, C₁-C₆-Alkoxy, C₁-C₆-Halogenalkoxy, C₃-C₇-Cycloalkyloxy, C₂-C₆-Alkenyloxy, C₃-C₆-Alkinyloxy, C₁-C₆-Alkylthio, C₁-C₆-Halogenalkylthio, Amino, C₁-C₆-Alkylamino, Di(C₁-C₆-alkyl)amino, C₁-C₆-Alkylsulfinyl, C₁-C₆-Halogen-alkylsulfinyl, C₁-C₆-Alkylsulfonyl, C₁-C₆-Halogenalkylsulfonyl, C₁-C₆-Alkoxysulfonyl, Formyl, C₁-C₆-Alkylcarbonyl, C₁-C₆-Halogenalkylcarbonyl, C₂-C₆-Alkenylcarbonyl, C₃-C₆-Alkinylcarbonyl, Carboxy, C₁-C₆-Alkoxycarbonyl, C₁-C₆-Halogenalkoxycarbonyl, C₂-C₆-Alkenyloxycarbonyl, C₃-C₆-Alkinyloxycarbonyl, Mercaptocarbonyl, C₁-C₆-Alkylthiocarbonyl, C₁-C₆-Halogenalkylthiocarbonyl, C₂-C₆-Alkenylthiocarbonyl, C₃-C₆-Alkinylthiocarbonyl, Aminocarbonyl, C₁-C₆-Alkylaminocarbonyl, Di(C₁-C₆-alkylamino)carbonyl, C₁-C₆-Halogenalkylaminocarbonyl, Di(C₁-C₆-halogenalkylamino)carbonyl, C₂-C₆-Alkenylaminocarbonyl, Di(C₂-C₆-alkenylamino)carbonyl, C₃-C₆-Alkinylaminocarbonyl, Di(C₃-C₆-alkinylamino)carbonyl, Phenyl, Phenoxy, Phenyl-C₁-C₄-Alkyl und Phenyl-C₁-C₄-alkoxy, tragen können;
vier- bis sechsgliedriges Heterocyclyl, das partiell oder vollständig halogeniert und/oder durch ein bis drei Reste aus der Gruppe C₁-C₆-Alkyl und C₁-C₆-Alkoxy substituiert sein kann; oder
vier- bis sechsgliedriges Heterocyclyl-C₁-C₄-alkyl, das partiell oder vollständig halogeniert und/oder durch ein bis drei Reste aus der Gruppe C₁-C₆-Alkyl und C₁-C₆-Alkoxy substituiert sein kann; oder
fünf- bis sechsgliedriges Heteroaryl mit ein bis vier Stickstoffatomen, oder mit ein bis drei Stickstoffatomen und einem Sauerstoff- oder einem Schwefelatom, oder mit einem Sauerstoff oder Schwefelatom; das partiell oder vollständig halogeniert und/oder durch ein bis drei Reste aus der Gruppe C₁-C₆-Alkyl, C₁-C₆-Halogenalkyl, C₁-C₆-Alkoxy, C₁-C₆-Halogenalkoxy, Amino, C₁-C₆-Alkylamino und Di(C₁-C₆alkyl)amino substituiert sein kann, oder
fünf- bis sechsgliedriges Heteroaryl-C₁-C₄-alkyl mit ein bis vier Stickstoffatomen, oder mit ein bis drei Stickstoffatomen und einem Sauerstoff- oder einem Schwefelatom, oder mit einem Sauerstoff oder Schwefelatom; das partiell oder vollständig halogeniert und/oder durch ein bis drei Reste aus der Gruppe C₁-C₆-Alkyl, C₁-C₆-Halogenalkyl, C₁-C₆-Alkoxy, C₁-C₆-Halogen-alkoxy, Amino, C₁-C₆-Alkylamino und Di(C₁-C₆alkyl)amino substituiert sein kann;
R² Wasserstoff, C₁-C₈-Alkyl, C₂-C₈-Alkenyl, C₃-C₈-Alkinyl, C₃-C₇-Cycloalkyl, wobei die vier letztgenannten Reste teilweise oder vollständig halogeniert sein können; oder
R¹ und R² bilden zusammen mit dem N-Atom, an das sie gebunden sind, einen drei- bis siebengliedrigen Heterocyclus, welcher seinerseits partiell oder vollständig halogeniert und/oder durch ein bis drei Reste aus der Gruppe C₁-C₆-Alkyl, C₁-C₆-Halogenalkyl und C₁-C₆-Alkoxy substituiert sein kann;
Q ein Rest aus der Gruppe Q¹ bis Q³⁹ 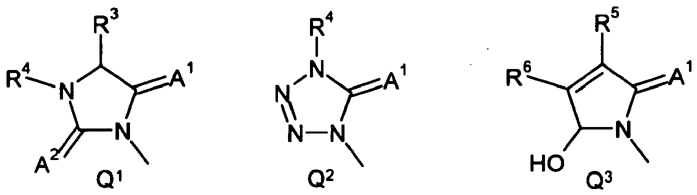 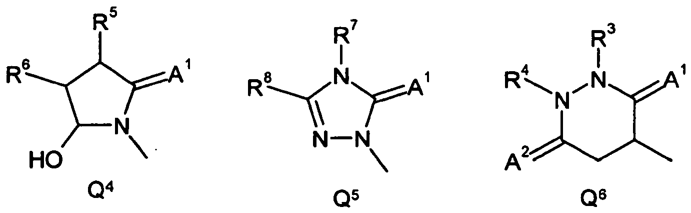 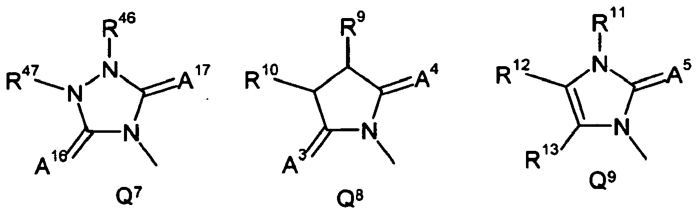 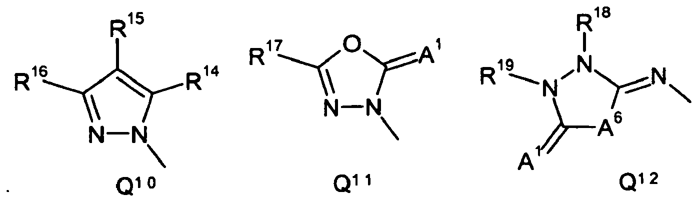 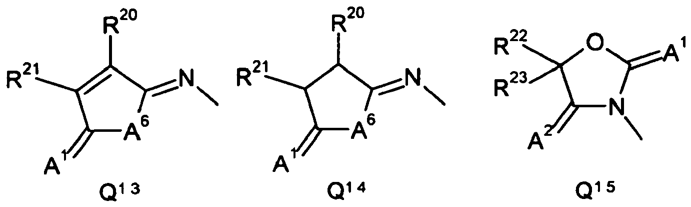 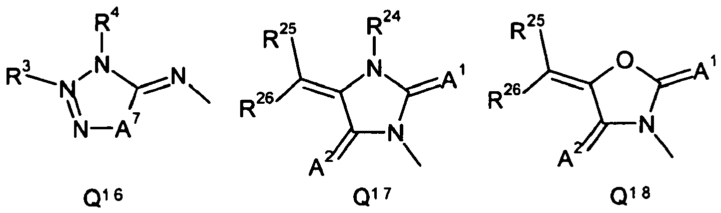 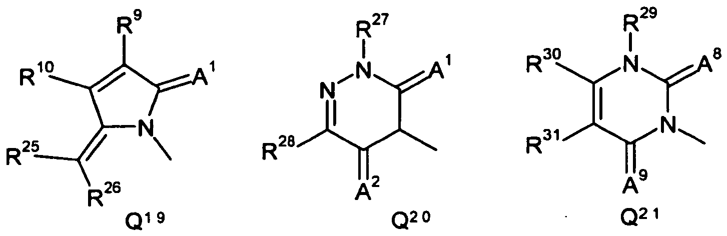 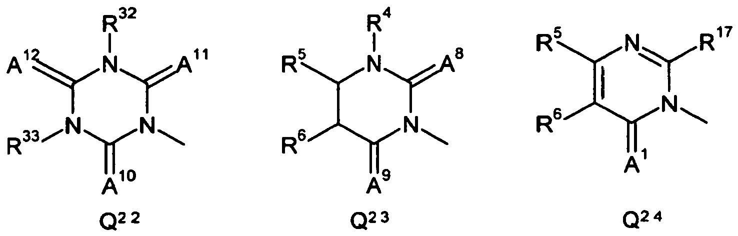 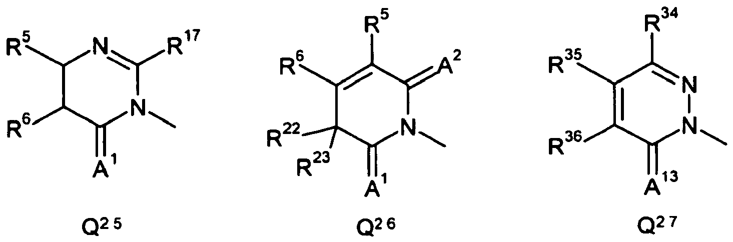 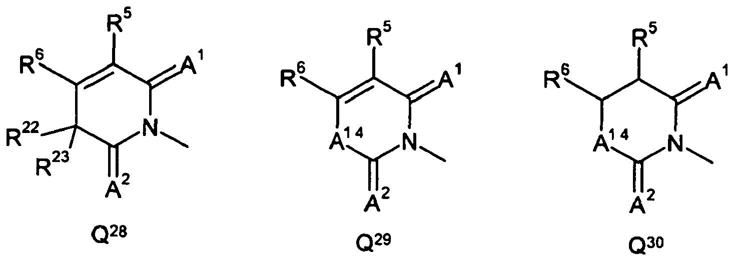 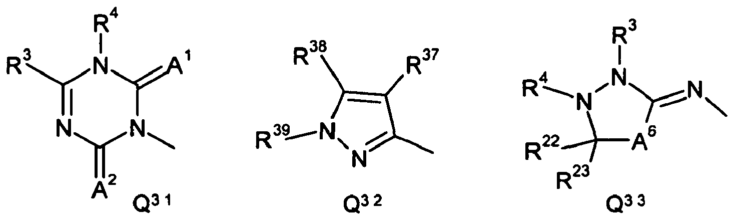 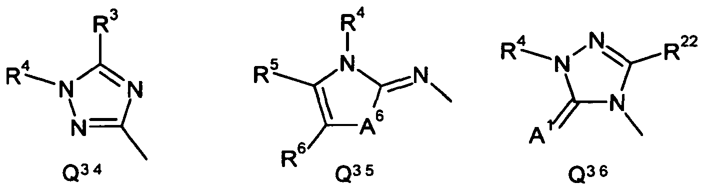 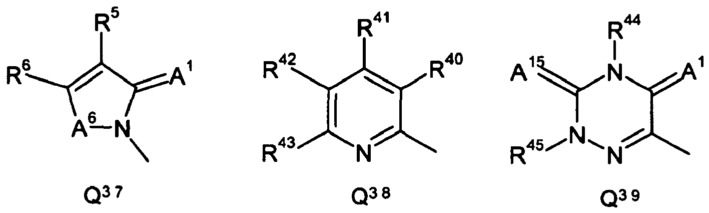
A¹ bis A¹⁷ Sauerstoff oder Schwefel;
R³, R⁴, R⁷, R⁸, R¹¹, R¹², R¹⁸, R¹⁹, R²⁷, R²⁹, R³², R³³, R³⁶, R³⁹, R⁴⁴, R⁴⁵, R⁴⁶ und R⁴⁷ Wasserstoff, Cyano, Hydroxy, C₁-C₆-Alkyl, C₁-C₆-Cyanoalkyl, C₁-C₆-Halogenalkyl, C₃-C₇-Cycloalkyl, C₃-C₇-Cycloalkyloxy, C₁-C₆-Alkoxy, C₁-C₆-Halogenalkoxy, C₂-C₆-Alkenyl, C₂-C₆-Halogenalkenyl, C₂-C₆-Alkenyloxy, C₃-C₆-Alkinyl, C₃-C₆-Alkinyloxy, C₁-C₆-Alkylsulfinyl, C₁-C₆-Alkylsulfonyl, Phenyl-C₁-C₆-alkyl, Amino, C₁-C₆-Alkylamino oder Di(C₁-C₆-alkyl)amino; oder
R³ und R⁴, R¹¹ und R¹²,
R¹⁸ und R¹⁹, oder R⁴⁶ und R⁴⁷ bilden zusammen mit den Atomen, an die sie gebunden sind, einen drei- bis siebengliedrigen Heterocyclus, welcher seinerseits partiell oder vollständig halogeniert und/oder durch ein bis drei Reste aus der Gruppe C₁-C₆-Alkyl und C₁-C₆-Alkoxy substituiert sein kann;
R⁵, R⁶, R⁹, R¹⁰,
R¹⁵, R¹⁶, R²⁰, R²¹,
R³⁰, R³¹, R³⁵, R³⁶,
R⁴¹, R⁴² und R⁴³ Wasserstoff, Hydroxy, C₁-C₆-Alkyl, C₁-C₆-Halogenalkyl, C₃-C₇-Cycloalkyl, C₃-C₇-Cycloalkyloxy, C₁-C₆-Alkoxy, C₁-C₆-Halogenalkoxy, C₂-C₆-Alkenyl, C₂-C₆-Halogenalkenyl, C₂-C₆-Alkenyloxy, C₃-C₆-Alkinyl, C₃-C₆-Alkinyloxy, C₁-C₆-Alkylthio, C₁-C₆-Alkylsulfinyl, C₁-C₆-Alkylsulfonyl, C₁-C₆-Alkoxysulfonyl, C₁-C₆-Alkylsulfonyloxy, Amino, C₁-C₆-Alkylamino oder Di(C₁-C₆-alkyl)amino; oder
R⁵ und R⁶, R⁹ und
R¹⁰, R¹⁵ und R¹⁶,
R²⁰ und R²¹, oder R³⁰ und R³¹ bilden zusammen mit den Atomen, an die sie gebunden sind, einen drei- bis siebengliedrigen Heterocyclus, welcher seinerseits partiell oder vollständig halogeniert und/oder durch ein bis drei Reste aus der Gruppe C₁-C₆-Alkyl und C₁-C₆-Alkoxy substituiert sein kann;
R¹³, R¹⁴, R²²,
R²³, R²⁵ und R²⁶ Wasserstoff, Halogen oder C₁-C₆-Alkyl;
R¹⁷, R²⁸, R³⁴,
R³⁷ oder R⁴⁰ Wasserstoff, Halogen, Hydroxy, C₁-C₆-Alkyl, C₁-C₆-Halogenalkyl, C₃-C₇-Cycloalkyl, C₃-C₇-Cycloalkyloxy, C₁-C₆-Alkoxy, C₁-C₆-Halogenalkoxy, C₁-C₆-Alkylthio, C₁-C₆-Halogenalkylthio, C₂-C₆-Alkenyl, C₂-C₆-Halogenalkenyl, C₂-C₆-Alkenyloxy, C₃-C₆-Alkinyl oder C₃-C₆-Alkinyloxy;
R²⁴ Wasserstoff, C₁-C₆-Alkyl, C₁-C₆-Halogenalkyl, C₂-C₆-Alkenyl, C₃-C₆-Alkinyl, C₁-C₆-Halogenalkoxy, Amino, C₁-C₆-Alkylamino oder Di(C₁-C₆-alkyl)amino;
sowie deren landwirtschaftlich brauchbaren Salze.

2. Benzolsulfonamide der Formel I gemäß Anspruch 1, in der X¹ Wasserstoff, Fluor oder Chlor bedeutet.

3. Benzolsulfonamide der Formel I gemäß Anspruch 1, in der Q
Q¹, Q², Q⁵, Q⁷, Q⁸, Q¹⁰, Q¹², Q¹³, Q¹⁷, Q²⁰, Q²¹, Q²², Q²³, Q²⁴, Q²⁷, Q³¹, Q³², Q³⁴, Q³⁸ oder Q³⁹ bedeutet.

4. Benzolsulfonamide der Formel I gemäß Anspruch 1, in der Q
Q⁷, Q²¹, Q²², Q²⁷, Q³², Q³⁸ oder Q³⁹ bedeutet.

5. Verfahren zur Herstellung von Benzolsulfonamid-Derivaten der Formel I gemäß Anspruch 1, wobei X³ für Wasserstoff steht, **dadurch gekennzeichnet daß** Benzolsulfonyliso(thio)cyanate der Formel II 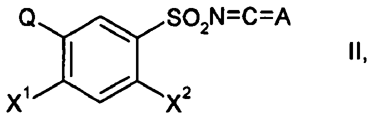 wobei X¹, X², Y, A, B und Q die unter Anspruch 1 genannten Bedeutungen haben,
mit Alkoholen bzw. Thiolen der Formel IV
HBR¹ IV
mit B = O, S
wobei R¹ und R² die unter Anspruch 1 genannten Bedeutungen haben, umgesetzt werden.

6. Benzolsulfonyliso(thio)cyanate der Formel II 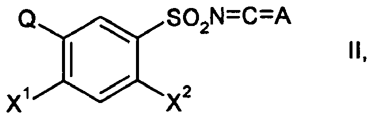 wobei X¹, X², A und Q die unter Anspruch 1 genannten Bedeutungen haben.

7. Mittel, enthaltend eine herbizid wirksame Menge mindestens eines Benzolsulfonamid-Derivates der Formel I oder eines landwirtschaftlich brauchbaren Salzes von I gemäß den Ansprüchen 1 bis 4 und für die Formulierung von Pflanzenschutzmitteln übliche Hilfsmittel.

8. Mittel zur Desikkation und/oder Defoliation von Pflanzen, enthaltend eine desikkant und/oder defoliant wirksame Menge mindestens eines Benzolsulfonamid-Derivates der Formel I oder eines landwirtschaftlich brauchbaren Salzes von I gemäß den Ansprüchen 1 bis 4 und für die Formulierung von Pflanzenschutzmitteln übliche Hilfsmittel.

9. Verfahren zur Herstellung von herbizid wirksamen Mitteln, **dadurch gekennzeichnet, dass** man eine herbizid wirksame Menge mindestens eines Benzolsulfonamid-Derivates der Formel I oder eines landwirtschaftlich brauchbaren Salzes von I gemäß den Ansprüchen 1 bis 4 und für die Formulierung von Pflanzenschutzmitteln übliche Hilfsmittel mischt.

10. Verfahren zur Herstellung von desikkant und/oder defoliant wirksamen Mitteln, **dadurch gekennzeichnet, dass** man eine desikkant und/oder defoliant wirksame Menge mindestens eines Benzolsulfonamid-Derivates der Formel I oder eines landwirtschaftlich brauchbaren Salzes von I gemäß den Ansprüchen 1 bis 4 und für die Formulierung von Pflanzenschutzmitteln übliche Hilfsmittel mischt.

11. Verfahren zur Bekämpfung von unerwünschtem Pflanzenwuchs, **dadurch gekennzeichnet, dass** man eine herbizid wirksame Menge mindestens eines Benzolsulfonamid-Derivates der Formel I oder eines landwirtschaftlich brauchbaren Salzes von I gemäß den Ansprüchen 1 bis 4 auf Pflanzen, deren Lebensraum und/oder auf Samen einwirken lässt.

12. Verfahren zur Desikkation und/oder Defoliation von Pflanzen, **dadurch gekennzeichnet, dass** man eine desikkant und/oder defoliant wirksame Menge mindestens eines Benzolsulfonamid-Derivates der Formel I oder eines landwirtschaftlich brauchbaren Salzes von I gemäß den Ansprüchen 1 bis 4 auf Pflanzen einwirken lässt.

13. Verwendung der Benzolsulfonamid-Derivate der Formel I und deren landwirtschaftlich brauchbaren Salze gemäß den Ansprüchen 1 bis 4 als Herbizide oder zur Desikkation und/oder Defoliation von Pflanzen.

## Claims

1. A benzenesulfonamide derivative of the formula I 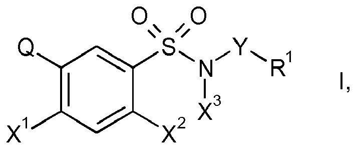 in which the variables are as defined below:
X¹ is hydrogen or halogen;
X² is chlorine;
X³ is hydrogen, cyano, C₁-C₆-alkyl, C₁-C₆-alkoxy-C₁-C₄-alkyl, C₃-C₇-cycloalkyl, C₃-C₆-alkenyl, C₃-C₆-alkynyl or phenyl-C₁-C₄-alkyl, where the phenyl radical for its part may be partially or fully halogenated and/or substituted by one to three radicals from the group consisting of C₁-C₆-alkyl and C₁-C₆-alkoxy;
Y is a group -C(A)B;
A is oxygen;
B is oxygen or sulfur;
R¹ is hydrogen, hydroxyl, C₁-C₈-alkyl, C₃-C₇-cycloalkyl, C₃-C₇-cycloalkyl-C₁-C₄-alkyl, C₂-C₈-alkenyl, C₅-C₇-cycloalkenyl, C₃-C₈-alkynyl, C₁-C₈-alkoxy, C₃-C₇-cycloalkyloxy, C₂-C₈-alkenyloxy, C₃-C₈-alkynyloxy, aryl, aryloxy, aryl-C₁-C₄-alkyl;
where the 13 last mentioned radicals for their part may be partially or fully halogenated and/or may carry one to three substituents from the group consisting of cyano, NO₂, hydroxyl, C₁-C₆-alkyl, C₁-C₆-haloalkyl, C₃-C₇-cycloalkyl, C₁-C₆-alkoxy, C₁-C₆-haloalkoxy, C₃-C₇-cycloalkyloxy, C₂-C₆-alkenyloxy, C₃-C₆-alkynyloxy, C₁-C₆-alkylthio, C₁-C₆-haloalkylthio, amino, C₁-C₆-alkylamino, di(C₁-C₆-alkyl)amino, C₁-C₆-alkylsulfinyl, C₁-C₆-haloalkylsulfinyl, C₁-C₆-alkylsulfonyl, C₁-C₆-haloalkylsulfonyl, C₁-C₆-alkoxysulfonyl, formyl, C₁-C₆-alkylcarbonyl, C₁-C₆-haloalkylcarbonyl, C₂-C₆-alkenylcarbonyl, C₃-C₆-alkynylcarbonyl, carboxy, C₁-C₆-alkoxycarbonyl, C₁-C₆-haloalkoxycarbonyl, C₂-C₆-alkenyloxycarbonyl, C₃-C₆-alkynyloxycarbonyl, mercaptocarbonyl, C₁-C₆-alkylthiocarbonyl, C₁-C₆-haloalkylthiocarbonyl, C₂-C₆-alkenylthiocarbonyl, C₃-C₆-alkynylthiocarbonyl, aminocarbonyl, C₁-C₆-alkylaminocarbonyl, di(C₁-C₆-alkylamino)carbonyl, C₁-C₆-haloalkylaminocarbonyl, di(C₁-C₆-haloalkylamino)carbonyl, C₂-C₆-alkenylaminocarbonyl, di(C₂-C₆-alkenylamino)carbonyl, C₃-C₆-alkynylaminocarbonyl, di(C₃-C₆-alkynylamino)carbonyl, phenyl, phenoxy, phenyl-C₁-C₄-alkyl and phenyl-C₁-C₄-alkoxy;
four- to six-membered heterocyclyl which may be partially or fully halogenated and/or substituted by one to three radicals from the group consisting of C₁-C₆-alkyl and C₁-C₆-alkoxy; or
four- to six-membered heterocyclyl-C₁-C₄-alkyl which may be partially or fully halogenated and/or substituted by one to three radicals from the group consisting of C₁-C₆-alkyl and C₁-C₆-alkoxy; or
five- or six-membered heteroaryl having one to four nitrogen atoms or having one to three nitrogen atoms and one oxygen or one sulfur atom or having one oxygen or sulfur atom, which radical may be partially or fully halogenated and/or substituted by one to three radicals from the group consisting of C₁-C₆-alkyl, C₁-C₆-haloalkyl, C₁-C₆-alkoxy, C₁-C₆-haloalkoxy, amino, C₁-C₆-alkylamino and di(C₁-C₆-alkyl)amino; or
five- or six-membered heteroaryl-C₁-C₄-alkyl having one to four nitrogen atoms or having one to three nitrogen atoms and one oxygen or one sulfur atom or having one oxygen or sulfur atom, which radical may be partially or fully halogenated and/or substituted by one to three radicals from the group consisting of C₁-C₆-alkyl, C₁-C₆-haloalkyl, C₁-C₆-alkoxy, C₁-C₆-haloalkoxy, amino, C₁-C₆-alkylamino and di(C₁-C₆-alkyl)amino;
R² is hydrogen, C₁-C₈-alkyl, C₂-C₈-alkenyl, C₃-C₈-alkynyl, C₃-C₇-cycloalkyl, where the four last mentioned radicals may be partially or fully halogenated; or
R¹ and R² together with the nitrogen atom to which they are attached form a three- to seven-membered heterocycle which for its part may be partially or fully halogenated and/or substituted by one to three radicals from the group consisting of C₁-C₆-alkyl, C₁-C₆-haloalkyl and C₁-C₆-alkoxy;
Q is a radical from the group consisting of Q¹ to Q³⁹ 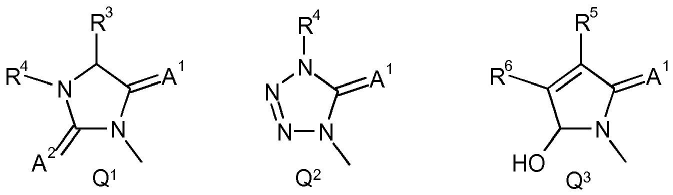 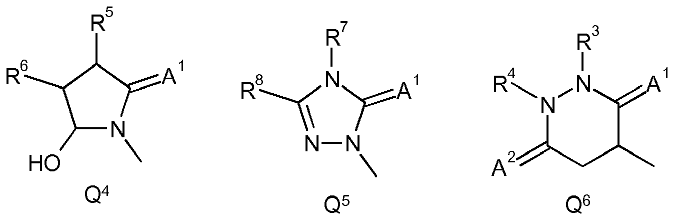 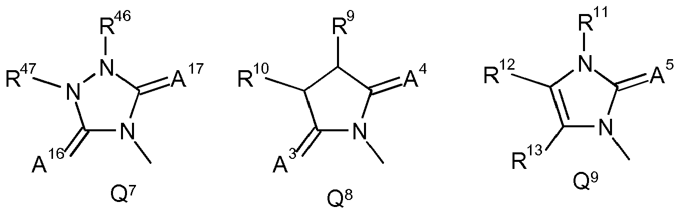 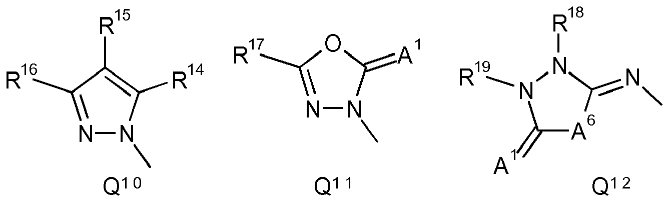 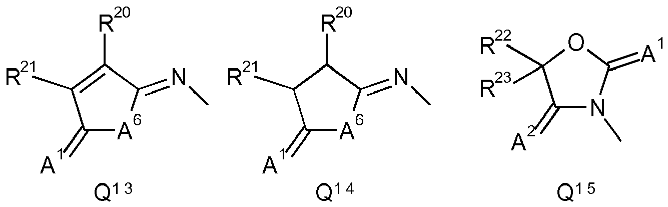 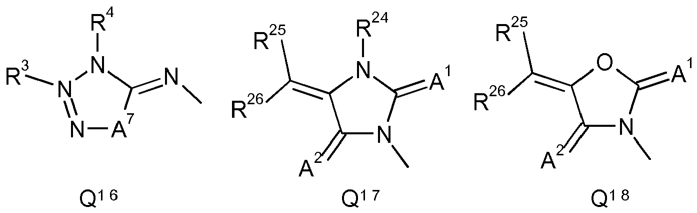 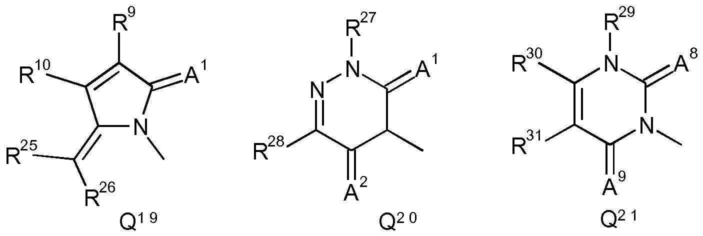 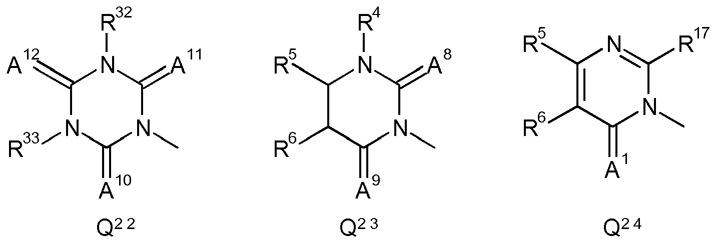 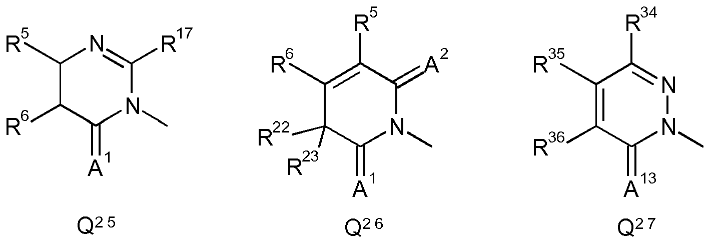 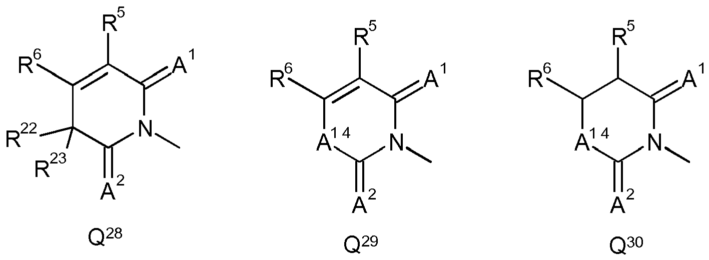 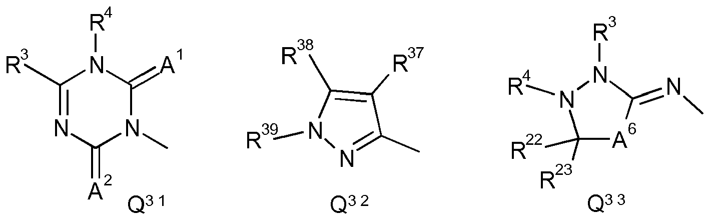 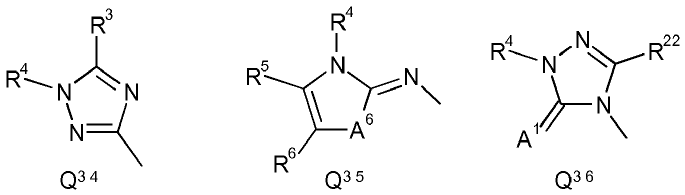 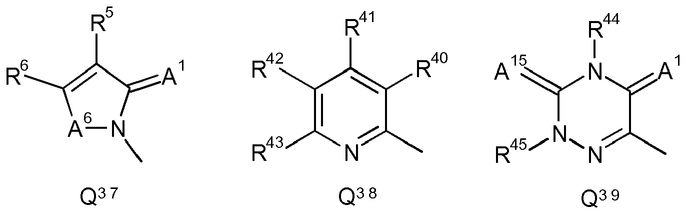
A¹ to A¹⁷ are oxygen or sulfur;
R³, R⁴, R⁷, R⁸,
R¹¹, R¹², R¹⁸, R¹⁹,
R²⁷, R²⁹, R³², R³³,
R³⁸, R³⁹, R⁴⁴, R⁴⁵,
R⁴⁶ and R⁴⁷ are hydrogen, cyano, hydroxyl, C₁-C₆-alkyl, C₁-C₆-cyanoalkyl, C₁-C₆-haloalkyl, C₃-C₇-cycloalkyl, C₃-C₇-cycloalkyloxy, C₁-C₆-alkoxy, C₁-C₆-haloalkoxy, C₂-C₆-alkenyl, C₂-C₆-haloalkenyl, C₂-C₆-alkenyloxy, C₃-C₆-alkynyl, C₃-C₆-alkynyloxy, C₁-C₆-alkylsulfinyl, C₁-C₆-alkylsulfonyl, phenyl-C₁-C₆-alkyl, amino, C₁-C₆-alkylamino or di(C₁-C₆-alkyl)amino; or
R³ and R⁴, R¹¹ and
R¹², R¹⁸ and R¹⁹, or
R⁴⁶ and R⁴⁷ together with the atoms to which they are attached form a three- to seven-membered heterocycle which for its part may be partially or fully halogenated and/or substituted by one to three radicals from the group consisting of C₁-C₆-alkyl and C₁-C₆-alkoxy;
R⁵, R⁶, R⁹,
R¹⁰, R¹⁵, R¹⁶, R²⁰,
R²¹, R³⁰, R³¹, R³⁵,
R³⁶, R⁴¹, R⁴² and R⁴³ are hydrogen, hydroxyl, C₁-C₆-alkyl, C₁-C₆-haloalkyl, C₃-C₇-cycloalkyl, C₃-C₇-cycloalkyloxy, C₁-C₆-alkoxy, C₁-C₆-haloalkoxy, C₂-C₆-alkenyl, C₂-C₆-haloalkenyl, C₂-C₆-alkenyloxy, C₃-C₆-alkynyl, C₃-C₆-alkynyloxy, C₁-C₆-alkylthio, C₁-C₆-alkylsulfinyl, C₁-C₆-alkylsulfonyl, C₁-C₆-alkoxysulfonyl, C₁-C₆-alkylsulfonyloxy, amino, C₁-C₆-alkylamino or di(C₁-C₆-alkyl)amino; or
R⁵ and R⁶, R⁹ and
R¹⁰, R¹⁵ and R¹⁶,
R²⁰ and R²¹, or R³⁰ and R³¹ together with the atoms to which they are attached form a three- to seven-membered heterocycle which for its part may be partially or fully halogenated and/or substituted by one to three radicals from the group consisting of C₁-C₆-alkyl and C₁-C₆-alkoxy;
R¹³, R¹⁴, R²²,
R²³, R²⁵ and R²⁶ are hydrogen, halogen or C₁-C₆-alkyl;
R¹⁷, R²⁸, R³⁴,
R³⁷ and R⁴⁰ are hydrogen, halogen, hydroxyl, C₁-C₆-alkyl, C₁-C₆-haloalkyl, C₃-C₇-cycloalkyl, C₃-C₇-cycloalkyloxy, C₁-C₆-alkoxy, C₁-C₆-haloalkoxy, C₁-C₆-alkylthio, C₁-C₆-haloalkylthio, C₂-C₆-alkenyl, C₂-C₆-haloalkenyl, C₂-C₆-alkenyloxy, C₃-C₆-alkynyl or C₃-C₆-alkynyloxy;
R²⁴ is hydrogen, C₁-C₆-alkyl, C₁-C₆-haloalkyl, C₂-C₆-alkenyl, C₃-C₆-alkynyl, C₁-C₆-haloalkoxy, amino, C₁-C₆-alkylamino or di(C₁-C₆-alkyl)amino;
or an agriculturally useful salt thereof.

2. The benzenesulfonamide of the formula I according to claim 1, in which X¹ is hydrogen, fluorine or chlorine.

3. The benzenesulfonamide of the formula I according to claim 1, in which Q is Q¹, Q², Q⁵, Q⁷, Q⁸, Q¹⁰, Q¹², Q¹³, Q¹⁷, Q²⁰, Q²¹, Q²², Q²³, Q²⁴, Q²⁷, Q³¹, Q³², Q³⁴, Q³⁸ or Q³⁹.

4. The benzenesulfonamide of the formula I according to claim 1, in which Q is Q⁷, Q²¹, Q²², Q²⁷, Q³², Q³⁸ or Q³⁹.

5. A process for preparing benzenesulfonamide derivatives of the formula I according to claim 1, where X³ is hydrogen, which comprises reacting benzenesulfonyl iso(thio)cyanates of the formula II 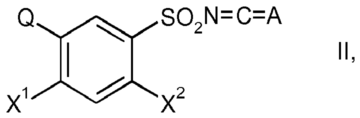 where X¹, X², Y, A, B and Q are as defined under claim 1,
with alcohols or thiols of the formula IV
HBR¹ IV
where B = O, S
where R¹ and R² are as defined in claim 1.

6. A benzenesulfonyl iso(thio)cyanate of the formula II 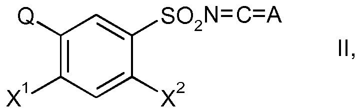 where X¹, X², A and Q are as defined under claim 1.

7. A composition, comprising a herbicidally effective amount of at least one benzenesulfonamide derivative of the formula I or an agriculturally useful salt of I according to any of claims 1 to 4 and auxiliaries customary for formulating crop protection agents.

8. A composition for the desiccation and/or defoliation of plants, comprising such an amount of at least one benzenesulfonamide derivative of the formula I or an agriculturally useful salt of I according to any of claims 1 to 4 that it acts as a desiccant and/or defoliant, and auxiliaries customary for formulating crop protection agents.

9. A process for preparing herbicidally effective compositions, which comprises mixing a herbicidally effective amount of at least one benzenesulfonamide derivative of the formula I or an agriculturally useful salt of I according to any of claims 1 to 4 and auxiliaries customary for formulating crop protection agents.

10. A process for preparing compositions having desiccant and/or defoliant action, which comprises mixing such an amount of at least one benzenesulfonamide derivative of the formula I or an agriculturally useful salt of I according to any of claims 1 to 4 that it acts as a desiccant and/or defoliant and auxiliaries customary for formulating crop protection agents.

11. A method for controlling unwanted vegetation, wherein a herbicidally effective amount of at least one benzenesulfonamide derivative of the formula I or an agriculturally useful salt of I according to any of claims 1 to 4 is allowed to act on plants, their habitat and/or on seeds.

12. A method for the desiccation and/or defoliation of plants, which comprises allowing such an amount of at least one benzenesulfonamide derivative of the formula I or an agriculturally useful salt of I according to any of claims 1 to 4 that it acts as a desiccant and/or defoliant to act on plants.

13. The use of the benzenesulfonamide derivatives of the formula I and their agriculturally useful salts according to any of claims 1 to 4 as herbicides or for the desiccation and/or defoliation of plants.

## Revendications

1. Dérivés de benzènesulfonamide de formule I 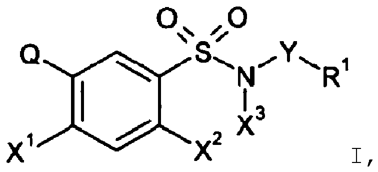 dans laquelle les variables ont les significations suivantes :
X¹ un atome d'hydrogène ou d'halogène ;
X² un atome de chlore ;
X³ un atome d'hydrogène, un groupe cyano, alkyle en C₁-C₆, alcoxy(C₁-C₆)-alkyle(C₁-C₄), cycloalkyle en C₃-C₇, alcényle en C₃-C₆, alcynyle en C₃-C₆ ou phényl-alkyle(C₁-C₄), le radical phényle pouvant pour sa part être halogéné partiellement ou totalement et/ou substitué par un à trois radicaux choisis dans l'ensemble constitué par des groupes alkyle en C₁-C₆ et alcoxy en C₁-C₆ ;
Y un groupe -C(A)B ;
A un atome d'oxygène ;
B un atome d'oxygène ou de soufre ;
R¹ un atome d'hydrogène, un groupe hydroxy, alkyle en C₁-C₈, cycloalkyle en C₃-C₇, cycloalkyle(C₃-C₇)-alkyle(C₁-C₄), alcényle en C₂-C₈, cycloalcényle en C₅-C₇, alcynyle en C₃-C₈, alcoxy en C₁-C₈, cycloalkyloxy en C₃-C₇, alcényloxy en C₂-C₈, alcynyloxy en C₃-C₈, aryle, aryloxy, aryl-alkyle(C₁-C₄) ;
les 13 radicaux nommés en dernier pouvant pour leur part être partiellement ou totalement halogénés et/ou pouvant porter un à trois substituants choisis dans l'ensemble constitué par les groupes cyano, NO₂, hydroxy, alkyle en C₁-C₆, halogénoalkyle en C₁-C₆, cycloalkyle en C₃-C₇, alcoxy en C₁-C₆, halogénoalcoxy en C₁-C₆, cycloalkyloxy en C₃-C₇, alcényloxy en C₂-C₆, alcynyloxy en C₃-C₆, alkyl(C₁-C₆) thio, halogénoalkyl(C₁-C₆)-thio, amino, alkyl(C₁-C₆)amino, di[alkyl(C₁-C₆)]amino, alkyl(C₁-C₆)sulfinyle, halogénoalkyl(C₁-C₆)sulfinyle, alkyl(C₁-C₆)-sulfonyle, halogénoalkyl(C₁-C₆)sulfonyle, alcoxy(C₁-C₆)sulfonyle, formyle, alkyl(C₁-C₆)-carbonyle, halogénoalkyl(C₁-C₆)carbonyle, alcényl (C₂-C₆) carbonyle, alcynyl(C₃-C₆) - carbonyle, carboxy, alcoxy(C₁-C₆)carbonyle, halogénoalcoxy(C₁-C₆)carbonyle, alcényloxy(C₂-C₆) carbonyle, alcynyloxy(C₃-C₆)-carbonyle, mercaptocarbonyle, alkyl(C₁-C₆)-thiocarbonyle, halogénoalkyl(C₁-C₆)thiocarbonyle, alcényl(C₂-C₆)thiocarbonyle, alcynyl(C₃-C₆)thiocarbonyle, aminocarbonyle, alkyl(C₁-C₆)aminocarbonyle, di[alkyl(C₁-C₆)-amino] carbonyle, halogénoalkyl(C₁-C₆)aminocarbonyle, di[halogénoalkyl (C₁-C₆)amino]-carbonyle, alcényl(C₂-C₆)aminocarbonyle, di [alcényl (C₂-C₆) amino] carbonyle, alcynyl(C₃-C₆)aminocarbonyle, di[alcynyl(C₃-C₆)amino]carbonyle, phényle, phénoxy, phényl-alkyle(C₁-C₄) et phénylalcoxy(C₁-C₄) ;
hétérocyclyle à quatre à six chaînons, qui peut être halogéné partiellement ou totalement et/ou substitué par un à trois radicaux choisis dans l'ensemble constitué par des groupes alkyle en C₁-C₆ et alcoxy en C₁-C₆ ; ou
hétérocyclyl(à quatre à six chaînons)-alkyle(C₁-C₄), qui peut être halogéné partiellement ou totalement et/ou substitué par un à trois radicaux choisis dans l'ensemble constitué par des groupes alkyle en C₁-C₆ et alcoxy en C₁-C₆ ; ou
hétéroaryle à cinq à six chaînons, comportant un à quatre atomes d'azote, ou comportant un à trois atomes d'azote et un atome d'oxygène ou un atome de soufre, ou comportant un atome d'oxygène ou atome de soufre ; qui peut être halogéné partiellement ou totalement et/ou substitué par un à trois radicaux choisis dans l'ensemble constitué par des groupes alkyle en C₁-C₆, halogénoalkyle en C₁-C₆, alcoxy en C₁-C₆, halogénoalcoxy en C₁-C₆, amino, alkyl(C₁-C₆)amino et di[alkyl(C₁-C₆)]-amino ; ou
hétéroaryl (à cinq à six chaînons)-alkyle(C₁-C₄), comportant un à quatre atomes d'azote, ou comportant un à trois atomes d'azote et un atome d'oxygène ou un atome de soufre, ou comportant un atome d'oxygène ou atome de soufre ; qui peut être halogéné partiellement ou totalement et/ou substitué par un à trois radicaux choisis dans l'ensemble constitué par des groupes alkyle en C₁-C₆, halogénoalkyle en C₁-C₆, alcoxy en C₁-C₆, halogénoalcoxy en C₁-C₆, amino, alkyl(C₁-C₆)amino et di[alkyl(C₁-C₆)]amino ;
R² un atome d'hydrogène, un groupe alkyle en C₁-C₈, alcényle en C₂-C₈, alcynyle en C₃-C₈, cycloalkyle en C₃-C₇, les quatre radicaux nommés en dernier pouvant être partiellement ou totalement halogénés ; ou
R¹ et R² forment ensemble avec l'atome d'azote, auquel ils sont liés, un hétérocycle à trois à sept chaînons, qui pour sa part peut être halogéné partiellement ou totalement et/ou substitué par un à trois radicaux choisis dans l'ensemble constitué par des groupes alkyle en C₁-C₆, halogénoalkyle en C₁-C₆ et alcoxy en C₁-C₆ ;
Q un radical choisi dans l'ensemble constitué par Q¹ à Q³⁹ 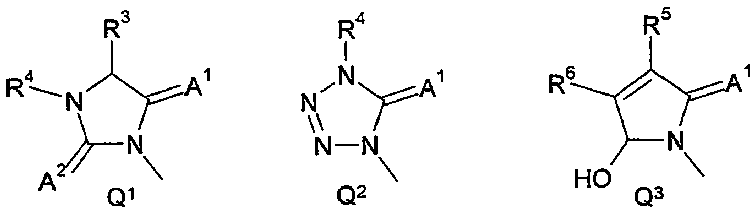 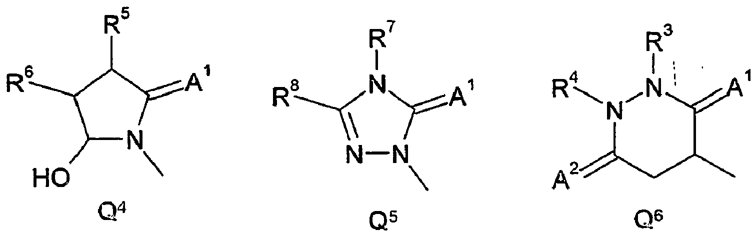 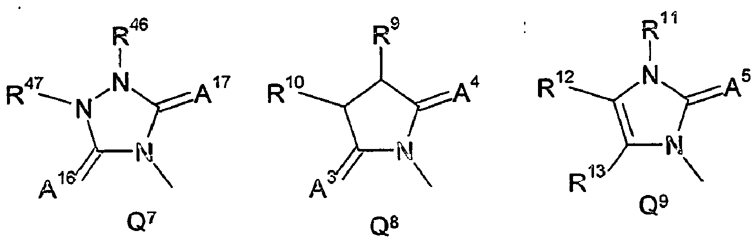 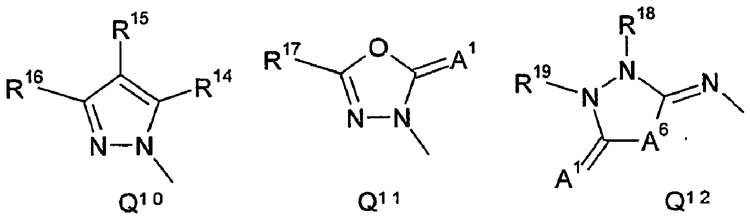 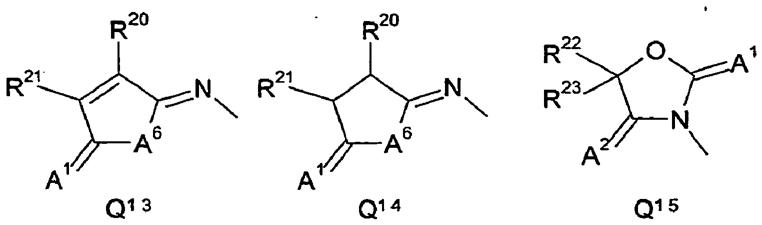 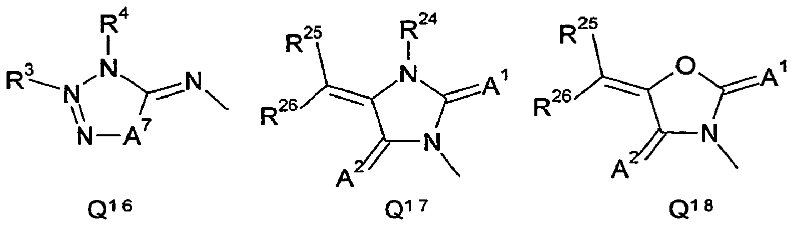 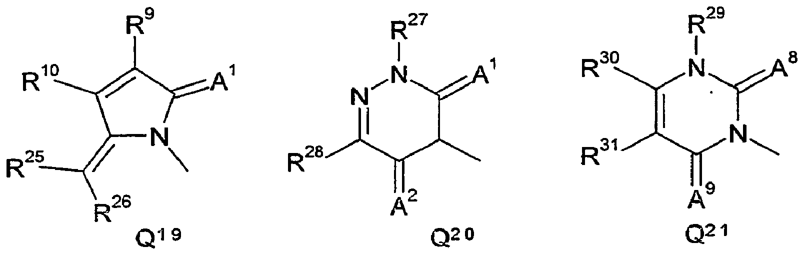 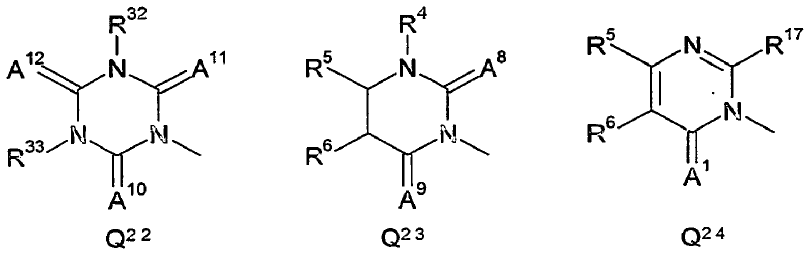 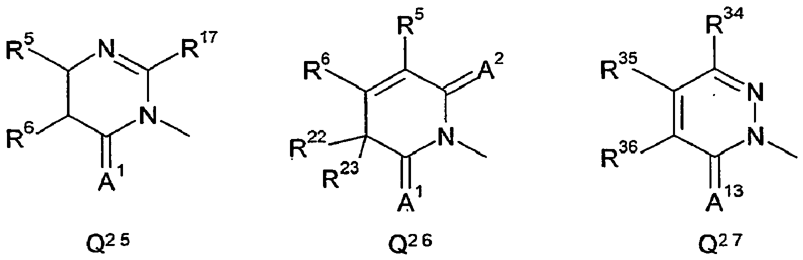 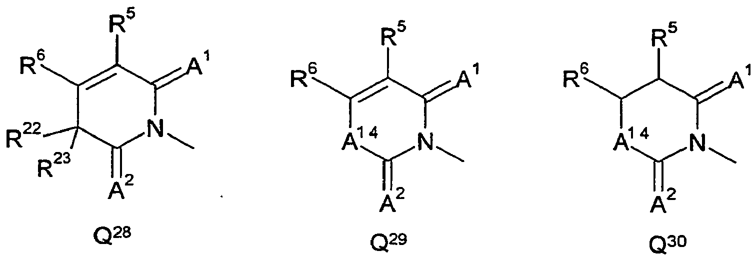 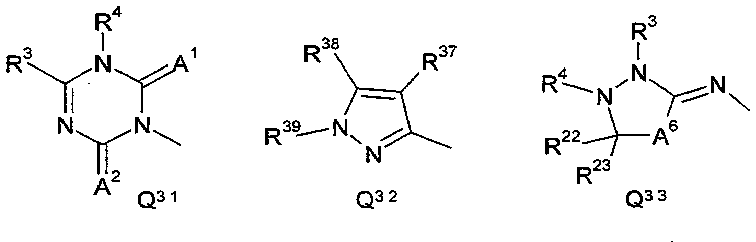 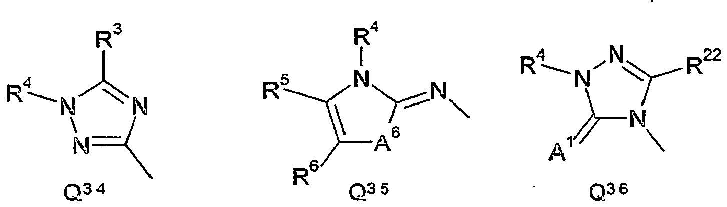 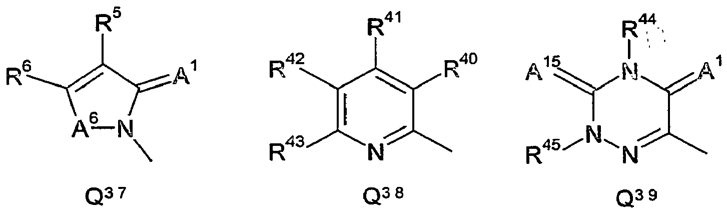
A¹ à A¹⁷ un atome d'oxygène ou de soufre ;
R³⁹, R⁴⁴, R³, R⁴,
R⁷, R⁸, R¹¹, R¹²,
R¹⁸, R¹⁹, R²⁷, R²⁹,
R³², R³³, R³⁸,R⁴⁵,
R⁴⁶ et R⁴⁷ un atome d'hydrogène, un groupe cyano, hydroxy, alkyle en C₁-C₆, cyanoalkyle en C₁-C₆, halogénoalkyle en C₁-C₆, cycloalkyle en C₃-C₇, cycloalkyloxy en C₃-C₇, alcoxy en C₁-C₆, halogénoalcoxy en C₁-C₆, alcényle en C₂-C₆, halogénoalcényle en C₂-C₆, alcényloxy en C₂-C₆, alcynyle en C₃-C₆, alcynyloxy en C₃-C₆, alkyl(C₁-C₆)sulfinyle, alkyl(C₁-C₆)sulfonyle, phényl-alkyle(C₁-C₆), amino, alkyl(C₁-C₆)amino ou di[alkyl(C₁-C₆)]amino ; ou
R³ et R⁴, R¹¹ et R¹²,
R¹⁸ et R¹⁹, ou R⁴⁶ et R⁴⁷ forment ensemble avec les atomes, auxquels ils sont liés, un hétérocycle à trois à sept chaînons, qui pour sa part peut être halogéné partiellement ou totalement et/ou substitué par un à trois radicaux choisis dans l'ensemble constitué par des groupes alkyle en C₁-C₆ et alcoxy en C₁-C₆ ;
R⁵, R⁶, R⁹, R¹⁰,
R¹⁵, R¹⁶, R²⁰,
R²¹, R³⁰, R³¹,
R³⁵, R³⁶, R⁴¹,
R⁴² et R⁴³ un atome d'hydrogène, un groupe hydroxy, alkyle en C₁-C₆, halogénoalkyle en C₁-C₆, cycloalkyle en C₃-C₇, cycloalkyloxy en C₃-C₇, alcoxy en C₁-C₆, halogénoalcoxy en C₁-C₆, alcényle en C₂-C₆, halogénoalcényle en C₂-C₆, alcényloxy en C₂-C₆, alcynyle en C₃-C₆, alcynyloxy en C₃-C₆, alkyl(C₁-C₆)thio, alkyl(C₁-C₆)sulfinyle, alkyl(C₁-C₆)sulfonyle, alcoxy(C₁-C₆) sulfonyle, alkyl(C₁-C₆)-sulfonyloxy, amino, alkyl(C₁-C₆) amino ou di[alkyl(C₁-C₆)]amino ; ou
R⁵ et R⁶, R⁹ et R¹⁰,
R¹⁵ et R¹⁶, R²⁰ et R²¹,
ou R³⁰ et R³¹ forment ensemble avec les atomes, auxquels ils sont liés, un hétérocycle à trois à sept chaînons, qui pour sa part peut être halogéné partiellement ou totalement et/ou substitué par un à trois radicaux choisis dans l'ensemble constitué par des groupes alkyle en C₁-C₆ et alcoxy en C₁-C₆ ;
R¹³, R¹⁴, R²², R²³,
R²⁵ et R²⁶ un atome d'hydrogène ou d'halogène ou un groupe alkyle en C₁-C₆ ;
R¹⁷, R²⁸, R³⁴, R³⁷ ou R⁴⁰ un atome d'hydrogène ou d'halogène, un groupe hydroxy, alkyle en C₁-C₆, halogénoalkyle en C₁-C₆, cycloalkyle en C₃-C₇, cycloalkyloxy en C₃-C₇, alcoxy en C₁-C₆, halogénoalcoxy en C₁-C₆, alkyl(C₁-C₆)thio, halogénoalkyl(C₁-C₆) - thio, alcényle en C₂-C₆, halogénoalcényle en C₂-C₆, alcényloxy en C₂-C₆, alcynyle en C₃-C₆ ou alcynyloxy en C₃-C₆ ;
R²⁴ un atome d'hydrogène, un groupe alkyle en C₁-C₆, halogénoalkyle en C₁-C₆, alcényle en C₂-C₆, alcynyle en C₃-C₆, halogénoalcoxy en C₁-C₆, amino, alkyl(C₁-C₆)amino ou di[alkyl(C₁-C₆)]amino ;
ainsi que leurs sels utilisables en agriculture.

2. Benzènesulfonamides de formule I selon la revendication 1, dans lesquels X¹ représente un atome d'hydrogène, de fluor ou de chlore.

3. Benzènesulfonamides de formule I selon la revendication 1, dans lesquels Q représente Q¹, Q², Q⁵, Q⁷, Q⁸, Q¹⁰, Q¹², Q¹³, Q¹⁷, Q²⁰, Q²¹, Q²², Q²³, Q²⁴, Q²⁷, Q³¹, Q³², Q³⁴, Q³⁸ ou Q³⁹.

4. Benzènesulfonamides de formule I selon la revendication 1, dans lesquels Q représente Q⁷, Q²¹, Q²², Q²⁷, Q³², Q³⁸ ou Q³⁹.

5. Procédé pour la préparation de dérivés de benzènesulfonamide de formule I selon la revendication 1, dans laquelle X³ représente un atome d'hydrogène, **caractérisé en ce qu'**on fait réagir des benzènesulfonyliso(thio)cyanates de formule II 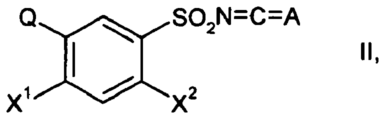 dans laquelle X¹, X², Y, A, B et Q ont les significations données dans la revendication 1, avec des alcools ou thiols de formule IV
HBR¹ IV
où B = 0, S
R¹ et R² ayant les significations données dans la revendication 1.

6. Benzènesulfonyliso(thio)cyanates de formule II 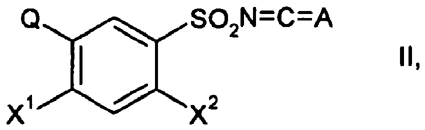 dans laquelle X¹, X², A et Q ont les significations données dans la revendication 1.

7. Compositions, contenant une quantité efficace en tant qu'herbicide d'au moins un dérivé de benzènesulfonamide de formule I ou un sel utilisable en agriculture de I selon les revendications 1 à 4 et des adjuvants usuels pour la formulation de produits phytosanitaires.

8. Composition destinée à la dessiccation et/ou la défoliation de plantes, contenant une quantité efficace en tant que dessiccant et/ou défoliant d'au moins un dérivé de benzènesulfonamide de formule I ou un sel utilisable en agriculture de I selon les revendications 1 à 4 et des adjuvants usuels pour la formulation de produits phytosanitaires.

9. Procédé pour la préparation de compositions à activité herbicide, **caractérisé en ce qu'**on mélange une quantité efficace en tant qu'herbicide d'au moins un dérivé de benzènesulfonamide de formule I ou un sel utilisable en agriculture de I selon les revendications 1 à 4 et des adjuvants usuels pour la formulation de produits phytosanitaires.

10. Procédé pour la préparation de compositions à activité dessiccante et/ou défoliante, **caractérisé en ce qu'**on mélange une quantité efficace en tant que dessiccant et/ou défoliant d'au moins un dérivé de benzènesulfonamide de formule I ou un sel utilisable en agriculture de I selon les revendications 1 à 4 et des adjuvants usuels pour la formulation de produits phytosanitaires.

11. Procédé pour la lutte contre la croissance indésirable de plantes, **caractérisé en ce qu'**on fait agir sur des plantes, leur habitat et/ou sur des semences une quantité efficace en tant qu'herbicide d'au moins un dérivé de benzènesulfonamide de formule I ou un sel utilisable en agriculture de I selon les revendications 1 à 4.

12. Procédé pour la dessiccation et/ou la défoliation de plantes, **caractérisé en ce qu'**on fait agir sur des plantes une quantité efficace en tant que dessiccant et/ou défoliant d'au moins un dérivé de benzènesulfonamide de formule I ou un sel utilisable en agriculture de I selon les revendications 1 à 4.

13. Utilisation des dérivés de benzènesulfonamide de formule I et de leurs sels utilisables en agriculture selon les revendications 1 à 4, en tant qu'herbicides ou pour la dessiccation et/ou la défoliation de plantes.
